# EUROPEAN PATENT APPLICATION

(11) **EP 2 784 085 A1**
(43) Date of publication of application: **01.10.2014**
(21) Application number: 12857862.2
(22) Date of filing: 22.11.2012
(51) Int. Cl.: C07K 14/62, A61K 38/28, A61P 5/50

(54) **HUMAN INSULIN ANALOGUE AND ACYLATED DERIVATIVE THEREOF**

(30) Priority: 15.12.2011 CN 201110422095
(71) Applicant: Shanghai Hengrui Pharmaceutical Co. Ltd., Minhang District Shanghai 200-245 (CN); Jiangsu Hengrui Medicine Co. Ltd., Jiangsu 222047 (CN)
(72) Inventor: SUN, Piaoyang, Lianyungang Jiangsu 222047 (CN); ZHANG, Lianshan, Lianyungang Jiangsu 222047 (CN); LIU, Jiajian, Shanghai 200245 (CN); YUAN, Jijun, Shanghai 200245 (CN); FANG, Chunqian, Shanghai 200245 (CN); SUN, Changan, Shanghai 200245 (CN); YUAN, Hengli, Shanghai 200245 (CN); WANG, Yali, Shanghai 200245 (CN)
(74) Representative: Regimbeau
(86) International application number: PCT/CN2012/085054
(87) International publication number: WO 2013/086927

(57) **Abstract**

The present invention provides a human insulin analogue, an acylated derivative thereof and a physiologically tolerable salt, and the present invention further provides a preparation method for the insulin analogue and an application of the insulin analogue as a therapeutic agent, and particularly as a diabetes mellitus therapeutic agent.

## Description

### FIELD OF THE INVENTION

The present invention relates to a novel human insulin analogue and acylated derivatives thereof, the preparation methods thereof, the pharmaceutical composition comprising the said analogue and acylated derivatives thereof, and the use of the same as a therapeutic agent, especially as diabetes therapeutic agent.

### BACKGROUND OF THE INVENTION

Diabetes (Diabetes Mellitus, DM) is a common metabolism endocrine disease. It's a clinically chronic and systemically metabolic syndrome, which is characterized in chronic hyperglycemia, disturbance of carbohydrate, fat and protein metabolism resulting from absolute or relative insulin deficiency, or insensitivity of the target tissues to insulin. It is caused by the interaction of genetic and environmental factors, involving various body systems, including cardio-cerebrovascular, kidney, eye, nerve and other organ complications. It's a lifelong disease, and is seriously harmful to human health.

Diabetes is a common, frequently-occuring disease, and it has become the third largest common disease threatening human lives following cancer and cardiovascular disease. It's a challenge to the world. DM hazards to people's health regardless of race and nation. According to the statistics from the International Diabetes Federation (IDF), during the ten years from the mid-1980s to the mid-1990s of the last century, the total number of diabetics increased 4-fold, up to 120 million. In 2007, the global number of diabetics is 246 million, of which 46% were 40-59 years old labor force population. It is anticipated that in 2025. the global number of diabetics will be increased to 380 million, accounting for 7.1 % of the global adult population.

According to the data announced by ADA (American Diabetes Association) in January 2011, there are 25.6 million diabetics in the United States, accounting for 8.3% of the U.S. population. Among the huge Medical cost in the United States, one of every 10 dolar is spent for diabetes. In china, the number of diabetics increases significantly for the recent 20 years. Epidemiological investigations revealed the prevalence of diabetes is less than 4% before 2002. Recently, a national epidemiological survey of diabetes shows that the prevalence of diabetes among Chinese adults (20 years of age or older) is more than 10%. The overall prevalence of diabetes was 9.7%. Currently, there are more than 92 million people suffering from diabetes, another 100 million and 5000 people will become diabetics. China is becoming a country having the largest number of diabetic patients, instead of India.

Diabetes is mainly divided into insulin-dependent diabetes mellitus (type I diabetes), insulin-independent diabetes mellitus (type II diabetes), as well as other special types of diabetes. Type I diabetes mainly occurs in children and adolescents, the peak age is 12 years old, accounting for less than 10% of diabetics. Type II diabetes mainly occurs in adults, accounting for more than 90% of diabetics. The exact pathological mechanisms underlying diabetes is still unknown, there is no cure for diabetes. Currently therapy for diabetes focuses on drug treatment and control. For a small portion of patients suffering from type □ diabetes, diet and exercise therapy can be used to control the condition, whereas for the vast majority of patients, drug therapy is necessary. Drug therapy includes Chinese medicine and chemical medicine. Chemical medicine is predominant, and is divided into two categories: protein and polypeptide drugs represented by insulin and analogues thereof, and small molecule anti-diabetic drugs for oral administration.

With the dramatic increase in the number of diabetic patients worldwide, the global diabetes drug market is also rapidly growing. According to statistics, in 2005 the diabetes drug market reached $ 18.6 billion, an increase of 11.5% compared to the previous year. In 2006, it was $ 21.2 billion, an increase of 14%. In 2007, it was $ 24.1 billion, an increase of 13.7%, ranking fifth in the global pharmaceutical market. 15 to 20 percent annual growth rate is predicted. Research and Markets reports that insulin and analogues thereof account for 40.1% of the total diabetes drug market, oral hypoglycemic drugs account for 58% and other drugs share the remaining 1.9%.

A series of patent applications have disclosed some insulin analogues, which comprise amino acid substitutions at various sites of natural human insulin sequences. EP0425482B1 discloses an insulin analogue having His or Tyr substitutions on position B25. EP0419504B1 discloses an insulin analogue having a substitution on position B3, simultaneously having Gln substitution on A5 or A15, alternatively, Asn substitution on A21 or A18. US5008241A discloses an insulin analogue having a specific amino acid substitution on A21, as well as a specific amino acid substitution on A4, A17, B13 or B21. US5164366A discloses an insulin analogue having amino acid deletion on one of B24, B25. B26 and B27 positions. CN1195777C discloses an insulin analogue having substitutions on A8, A9, A10, B30 positions. US7193035B2 discloses a crystalline insulin analogue having a substitution on B3 position and at least one substitution on one of B27, B28 and B29 positions. CN1780854 discloses an insulin analogue of A0 (Arg) A21 (Gly) B31 (Arg) B32 (Arg).

Nevertheless, in order to achieve better modification effect, to get a better efficacy, to decrease the binding activity between drugs and insulin-like growth factor -1 (IGF-1) receptor, and to provide more selective drugs, more modified insulin analogues are still required for the treatment of diabetes.

### SUMMARY OF THE INVENTION

Accordingly, the present invention is to provide novel drugs having excellent properties for diabetes treatment. More specifically, the present invention provides novel human insulin analogues and acylated derivatives thereof, and preparation methods for the analogues and derivatives, pharmaceutical compositions comprising the same, and use of the human insulin derivatives for the treatment of diabetes.

In the first respect, the present invention is directed to a human insulin analogue and a physiologically tolerable salt thereof, having A chain and B chain as follows: 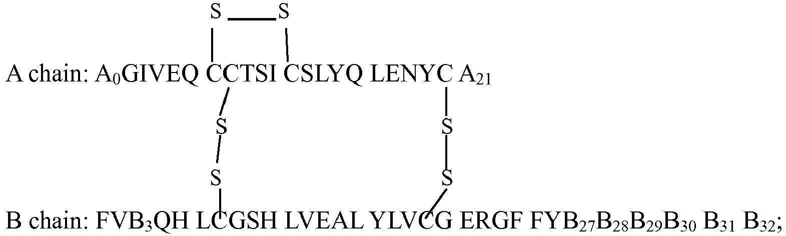 wherein:
A₀ can be R or missing;
A₂₁ can be N or G;
B₃ can be K, D or N;
B₂₇ can be E, T, D or K;
B₂₈ can be E, D, P or K;
B₂₉ can be E, K or P;
B₃₀ can be K, R, E, T or missing;
B₃₁,B₃₂ can be R, optionally missing or both missing;
wherein,
when the said A₀ is missing, A₂₁ is N, B₃ is N:
B₃₁B₃₂ are missing, B₂₇B₂₈B₂₉B₃₀ are not TPKT, TKPT or TDKT;
or B₃₀ B₃₁B₃₂ are missing, B₂₇B₂₈B₂₉ is not TPK;
alternatively, when A₀ is missing, A₂₁ is N, B₃ is K, and B₃₁B₃₂ are missing:
B₂₇B₂₈B₂₉B₃₀ is not TEKT;
alternatively, when A₀ is missing, A₂₁ is G, and B₃ is N,
B₂₇B₂₈B₂₉B₃₀ B₃₁B₃₂ is not TPKTRR.

Preferably, only one amino acid residue on positions B₃, B₂₇-B₃₀ is lysine (K) residue.

When A₀ on A chain is missing, and B₃ on B chain is N:
B₂₈ is preferably selected from E or D, B₂₉ is selected from E, K or P, B₃₀ is selected from R, E or missing, and B₃₁, B32 are missing; more preferably, when B₃₀ B₃₁B₃₂ is missing, B₂₈B₂₉ is KE.

When B₃ on B chain is D, B₂₈ and B₂₉ are preferably P or K, B₃₀ is T or missing, B₃₁ and B₃₂ are R or missing, most preferably B₂₈B₂₉ is PK.

When A₀ on A chain is missing and B₃ on B chain is K:
B₂₈ and B₂₉ are preferably P or E, B₃₀ is E or R, B₃₁ is R or missing, B₃₂ is missing, most preferably, B₂₈B₂₉ is PE.

The present invention provides a human insulin analogue or a physiologically tolerable salt thereof, wherein the A chain and the B chain of human insulin analogue are connected by disulfide bond (A7-B7, A20-B19), A6 and A11 on A chain are connected by disulfide bond; the sequences are selected from but not limited to the following sequences:

| NO | Sequences of Insulin Analogue | | Abbreviation of Insulin Analogue |
|---|---|---|---|
| | A chain: | RGIVEQCCTSICSLYQLENYCN | B(1-29),R-A(1-21) |
| 1 | B chain: | FVNQHLCGSHLVEALYLVCGERGFFYTPK | |
| | A chain: | GIVEQCCTSICSLYQLENYCN | B(1-27)-K-E-R, A(1-21) |
| 2 | B chain: | FVNQHLCGSHLVEALYLVCGERGFFYTKER | |
| | A chain: | GIVEQCCTSICSLYQLENYCN | B(1-27)-K-E, A(1-21) |
| 3 | B chain: | FVNQHLCGSHLVEALYLVCGERGFFYTKE | |
| | A chain: | GIVEQCCTSICSLYQLENYCN | B(1-27)-K-P-E, A(1-21) |
| 4 | B chain: | FVNQHLCGSHLVEALYLVCGERGFFYTKPE | |
| | A chain: | GIVEQCCTSICSLYQLENYCN | B(1-2)-K-B(4-28)-E-E, A(1-21) |
| 5 | B chain: | FVKQHLCGSHLVEALYLVCGERGFFYTPEE | |
| | A chain: | GIVEQCCTSICSLYQLENYCN | B(1-27)-D-K-E,A(1 -21) |
| 6 | B chain: | FVNQHLCGSHLVEALYLVCGERGFFYTDKE | |
| | A chain: | GIVEQCCTSICSLYQLENYCG | B(1-2)-D-B(4-30)-R-R, A(1-20)-G |
| 7 | B chain : | FVDQHLCGSHLVEALYLVCGERGFFYTPKTRR | |
| | A chain: | RGIVEQCCTSICSLYQLENYCG | B(1-2)-D-B(4-30)-R, R-A(1-20)-G |
| 8 | B chain: | FVDQHLCGSHLVEALYLVCGERGFFYTPKTR | |
| | A chain: | GIVEQCCTSICSLYQLENYCN | B(1-2)-K-B(4-28)-E-R-R, A(1-21) |
| 9 | B chain: | FVKQHLCGSHLVEALYLVCGERGFFYTPERR | |
| | A chain: | GIVEQCCTSICSLYQLENYCN | B(1-2)-D-B(4-29), A(1-21) |
| 10 | B chain: | FVDQHLCGSHLVEALYLVCGERGFFYTPK | |
| | A chain: | RGIVEQCCTSICSLYQLENYCN | B(1-2)-D-B(4-29), R-A(1-21) |
| 11 | B chain: | FVDQHLCGSHLVEALYLVCGERGFFYTPK | |
| | A chain: | GIVEQCCTSICSLYQLENYCG | B(1-27)-K-E, A(1-20)-G |
| 12 | B chain: | FVNQHLCGSHLVEALYLVCGERGFFYTKE | |
| | A chain: | GIVEQCCTSICSLYQLENYCG | B(1-2)-K-B(4-28)-E-E, A(1-20)-G |
| 13 | B chain: | FVKQHLCGSHLVEALYLVCGERGFFYTPEE | |
| | A chain: | GIVEQCCTSICSLYQLENYCG | B(1-2)-D-B(4-29)-R,A(1-20)-G |
| 14 | B chain: | FVDQHLCGSHLVEALYLVCGERGFFYTPKR | |
| | A chain: | GIVEQCCTSICSLYQLENYCG | B(1-2)-D-B(4-29), A(1-20)-G |
| 15 | B chain: | FVDQHLCGSHLVEALYLVCGERGFFYTPK | |
| 16 | A chain: | RGIVEQCCTSICSLYQLENYCG | B(1-2)-D-B(4-29), |
| | B chain: | FVDQHLCGSHLVEALYLVCGERGFFYTPK | R-A(1-20)-G |
| | A chain: | GIVEQCCTSICSLYQLENYCG | B(1-27)-D-K-E, A(1-20)-G |
| 17 | B chain: | FVNQHLCGSHLVEALYLVCGERGFFYTDKE | |

In another aspect, the present invention also provides a preferable example of the above human insulin analogue or a physiologically tolerable salt thereof, wherein the said human insulin analogue is PEGylated so as to obtain a PEGylated insulin according to the present invention. The molecular weight of the said PEG molecule is 5-100 KD, preferably 10-80 KD, more preferably 15-45KD, most preferably 20-40KD; the said PEG molecule is a branched-chain or straight-chain type.

The present invention also provides a preparation method for human insulin analogue or physiologically tolerable salt thereof, comprising constructing an expression vector, expressing the vector transformed into the host cells, isolating and purifying the expressed precursor, releasing the said insulin analogue from the expressed precursor via chemical and/or enzymatic methods; optionally the human insulin analogue is further PEGylated, the said PEgylation is preferably a chemically acylating modification method, which comprises synthesizing an acylating agent, acylating the amino group located in human insulin analogues, and removing the protecting group from the acylated group.

Wherein the said expressed precursor has the following formula (I):

A-R1-B (I)

wherein:
R1 is a peptide fragment having 0 to 5 amino acid residues, the said peptide fragment consists of alanine (A), lysine (K) and arginine (R);
A and B correspond to A chain and B chain of human insulin analogue, respectively.

Preferably, the said precursor is selected from the group consisting of:
FVNQHLCGSHLVEALYLVCGERGFFYTDKEKRGIVEQCCTSICSLYQLENYCN SEQ ID NO. 19,
FVKQHLCGSHLVEALYLVCGERGFFYTPEEKRGIVEQCCTSICSLYQLENYCG SEQ ID NO. 20,
FVKQHLCGSHLVEALYLVCGERGFFYTPEEKRGIVEQCCTSICSLYQLENYCN SEQ ID NO. 21,
FVNQHLCGSHLVEALYLVCGERGFFYTDKEKRGIVEQCCTSICSLYQLENYCG SEQ ID NO. 77.

The present invention also provides a DNA encoding the said expressed precursor.

The present invention also provides an expression vector comprising the said DNA.

The present invention also provides a host cell transformed with the said expression vector.

The said host cell is preferably bacterium, more preferably Escherichia coli.

Preferably the said host cell is yeast, more preferably Pichia pastoris or Saccharomyces cerevisiae.

The present invention also provides an insulin derivative, wherein acylated insulin is formed by connecting an acylated group to the α-amino group at the N-terminal of A chain and B chain of the said insulin, or to the ε-amino group at lysine (K) residue of B3, B27-B30, the acylated insulin has the following formula:

S-W-X-Y-Z

wherein S is insulin or insulin analogue according to the first aspect;
-W-X-Y-Z is an acylated group of insulin analogue, wherein, W is selected from
   - a group having a two-acyl structure of -OC(CH2)mCO-, wherein m is an integer between 2 and 10, an amide bond is formed between one of carboxylic groups on the said structure and α-amino group at the N-terminal amino acid residue of A-chain or B-chain of the parent insulin or analogue thereof or ε-amino group at a Lys residue existing in B-chain;
   - an α-amino acid residue with a carboxyl group on the side chain or a peptide having 2 to 4 α-amino acids with a carboxyl group on the side chain, wherein an amide bond is formed between the said residue or the said peptide and α-amino group at the N-terminal of A-chain or B-chain of the parent insulin or analogue thereof or ε-amino group at a Lys residue existing in B-chain;
X is selected from
   - -CO-;
   - a diamino compound comprising a carboxylic group, wherein an amide bond is formed between one of α-amino groups of the said diamino compound and the carboxylic group of W;
      a) when W is an α-amino acid residue or a peptide having 2 to 4 α-amino acids, an amide bond is formed between the amino group of W and the -CO- of X;
      b) when W is a group having a two-acyl structure, the said X group is linked to the two-acyl structure via one of its amino groups;
Y is selected from
   - -A(CH₂)ₘ-, wherein m is an integer between 6 and 32, and A is absent or is CO-;
   - bivalent hydrocarbon chain comprising an acyl group, which contains 1, 2 or 3 -CH = CH- groups and several -CH2- groups sufficient to obtain totally 10-32 carbon atoms on the chain;
   - bivalent hydrocarbon chain having the formula of -B(CH₂)ᵥC₆H₄(CH₂)_{w}-, wherein B is absent or CO-, v and w are integers or one of them is 0, making v and w in the range of 6 to 30;
      a) when X is CO-, A or B is absent; or
      b) when X is a diamino compound, A or B is CO-;

Z is selected from the group consisting of -OH, -NH₂, -COOH, -SO₃H, -PO₃H.

Preferably, the present invention provides an insulin derivative, wherein the acylated group -W-X-Y-Z is connected to the ε-amino group at lysine (K) residue of B3, B27 to B30 of parent insulin.

Preferably, the insulin derivative as above, wherein the acylated group -W-X-Y-Z is connected to the α-amino group at the N-terminal selected from chain A and chain B of insulin.

Preferably, the present invention provides an insulin derivative, wherein S is human insulin analogue selected from the group consisting of:

| | | |
|---|---|---|
| A chain: | GIVEQCCTSICSLYQLENYCN | SEQ ID NO.1 |
| B chain: | FVNQHLCGSHLVEALYLVCGERGFFYTDKE | SEQ ID NO.10 |
| | | |
| A chain: | GIVEQCCTSICSLYQLENYCN | SEQ ID NO.1 |
| B chain: | FVKQHLCGSHLVEALYLVCGERGFFYTPEE | SEQ ID NO.9 |
| | | |
| A chain: | GIVEQCCTSICSLYQLENYCG | SEQ ID NO.2 |
| B chain: | FVKQHLCGSHLVEALYLVCGERGFFYTPEE | SEQ ID NO.16 |

Preferably, the present invention provides an insulin derivative, wherein the acylated group -W-X-Y-Z is selected from the group consisting of:
N^{α}-(HOOC(CH₂)₁₄CO)-γ-Glu;
N^{α}-(HO(CH₂)₁₅CO)-γ-Glu;
N^{α}-(HOOC(CH₂)₁₄CO)-N^{ε}-(3-acyl acid *)-Lys,
in which * is the connection point for the insulin.

Preferably, the present invention provides an insulin derivative selected from the group consisting of:
B28D-N^{εB29}-(N^{α}-(HOOC(CH₂)₁₄CO)-γ-Glu)-B30E human insulin; (code: HS061)
B28D-N^{εB29}-(N^{α}-(HO(CH₂)₁₅CO)-γ-Glu)-B30E human insulin; (code: HS062)
N^{α}-(HOOC(CH₂)₁₄CO)-N^{ε}-(OCCH₂CH₂CO-(B28D-N^{εB29}-B30E human insulin;)
-Lys-OH; (code: HS067)
N^{εB3}-(N^{α}-(HOOC(CH₂)₁₄CO)-γ-Glu)-B29E-B30E human insulin; (code: HS605)
N^{α}-(HOOC(CH₂)₁₄CO)-N^{ε}-(OCCH₂CH₂CO-(N^{εB3}-B29E-B30E human insulin))-Lys-OH; (code: HS606)
N^{εB3}-(N^{α}-(HOC(CH₂)₁₅CO)-γ-Glu)-B29E-B30E human insulin;)) (code: HS607) N^{εB3}-(N^{α}-(HOOC(CH₂)₁₄CO)-γ-Glu)-B29E-B30E, A21G human insulin;(code: HS608)
N^{α}-(HOOC(CH₂)₁₄CO)-N^{ε}-(OCCH₂CH₂CO-(N^{εB3}-B29E-B30E, A21G human insulin;))-Lys-OH. (code: HS609)
N^{εB3}-(N^{α}-(HOC(CH₂)₁₅CO)-γ-Glu)-B29E-B30E, A21G human insulin; (code: HS610).

The present invention also provides a pharmaceutical formulation comprising insulin analogue and/or pharmaceutically acceptable salts thereof according to the invention, or insulin derivative as described above.

Preferably, the said pharmaceutical formulation comprises human insulin analogue or a physiologically tolerable salt thereof present in dissolved, amorphous and/or crystalline forms.

Preferably, the pharmaceutical formulation comprises long-term adjuvant, the said long-term adjuvant is present along with the medicament in a manner of co-crystallization.

The present invention also provides an injectable solution having insulin activity, wherein the solution comprises the said pharmaceutical formulation present in dissolved form.

The present invention also provides use of the human insulin analogue and/or a physiologically tolerable salt thereof, or insulin derivative in the preparation of a medicament for the treatment of type II diabetes, hyperglycemia, obesity or insulin resistance syndrome.

The present invention also provides use of the said human insulin analogue and/or a physiologically tolerable salt thereof, in the preparation of a quick-acting and/or long-acting pharmaceutical formulation having insulin activity.

The present invention provides novel human insulin analogues with good modification effects and better pharmacological activity, and provides more drugs for clinical option for treatment of diabetes.

**Terms** As used herein, the single-letter code and the three-letter code for amino acids were described in J.biol.chem, 243, the (1968) p3558.

In the DNA sequences, A refers to adenine, C refers to cytosine, G refers to guanine, and T refers to thymine.

"Human insulin analogue" refers to human insulin, wherein one or more amino acid residues has been deleted and/or replaced with other amino acid residues, alternatively, additional amino acid residues are introduced, i.e. the number of amino acid residues is more than 51.

"Insulin derivative" herein refers to acylated insulin formed by connecting an acylated group to the α-amino group at the N-terminal of A chain and B chain of insulin, or to the ε-amino group at lysine residue of B3, B27-B30. The said acylated insulin has the following formula: S-W-X-Y-Z; wherein each code is defined in the specification.

"PEGylated insulin" refers to pegylated human insulin analogue; wherein the PEG molecule has molecular weight of 5-100 KD, preferably 10-80 KD, more preferably 15-45KD, most preferably 20-40KD; wherein the PEG molecule is branched- or straight-chain type. In the present invention, PEGylated (20KD) insulin means that each subunit of the human insulin analogue is conjugated with a 20KD PEG molecule; PEGylated (30KD) insulin means that each subunit of the human insulin analogue is conjugated with a 30KD PEG molecule; PEGylated (branched type 40KD) insulin means that each subunit of the human insulin analogue is conjugated with a 40KD branched PEG molecule.

"Long-acting adjuvant" refers to protamine sulfate.

"A quick-acting pharmaceutical formulation having insulin activity" refers to the formulation comprising human insulin analogue or a physiologically tolerable salt thereof having quick-acting insulin activity.

"A long-acting pharmaceutical formulation having insulin activity" refers to the formulation comprising human insulin analogue or a physiologically tolerable salt thereof having long-acting insulin activity.

Sequences in the present invention are designed on the basis of amino acid sequences of natural human insulin. Full length of human insulin comprises two chains of A and B. A Chain and B chain are linked via disulfide bond, and there is another disulfide bond in the A chain. The human insulin has three disulfide bonds, marked with lines. Serial numbers of amino acids on each chain are indicated as the following rules: for example, amino acid residues on positions 1-21 of A chain are labeled as A1, A2, A3 ....... ; amino acid residues on positions 1-30 of B chain are labeled as B1, B2, B3 ........ In addition, if amino acid residues are added to the N-terminal of chain A and chain B, such amino acid residues are labeled as A0, A (-1), A (-2) ......, and B (0), B (-1), B (-2) ......,respectively. If amino acid residues are added to the C-terminal of chain A and chain B, such amino acid residues are labeled as A22, A23, ......, and B31, B32 ......, respectively. To represent a specific formula of human insulin analogue, the determined amino acid is presented by amino acid code, while the unsure substitution or deletion of amino acid is presented by the serial number of the position, as shown in the formula of the present invention.

In the embodiments of the present invention, B (1-29) used herein refers to shortened B chain consisting of B1 to B29 of human insulin, B (1-30) refers to B chain consisting of B1 to B30 of human insulin, B (1-31) refers to B chain consisting of B1 to B31 of human insulin, B (1-32) refers to shortened B chain consisting of B1 to B32 of human insulin; A (1-21) refers to A chain of human insulin, A (0-21) refers to A chain of human insulin having an additional amino acid residue on position A0. According to a particular embodiment of the present invention, a substituted residue in the human insulin is represented with the serial number of human insulin. For example, B (1-2)-D-B-(4-30), A(1-21) human insulin refers to a human insulin analogue wherein N is replaced with D at position 3 of B-chain. Unless indicated otherwise, the abbreviation of B (1-30), A (1-21) refers to natural human insulin; B (1-29), A (1-21) refers to human insulin having the deletion at position B30; B(1-2)-K-B(4-28)-E-E, A(1-21) means that substitution K is at position B3, and substitution EE is at position B29B30 (Example 6). Furthermore, as in human insulin, B chain and A chain of human insulin analogue is connected via interchain disulfide bonds formed between A (7) Cys and B (7) Cys, and between A (20) Cys and B (19) Cys. Simultaneously, A chain contains an intrachain disulfide bond formed between A (6) Cys and A (11) Cys.

According to the present invention, transformation of the recombinant DNA to a host cell is performed by conventional techniques well known to the skilled person in the art. The obtained transformant can be cultured by a conventional method to express polypeptide encoded by the gene according to the present invention. Dependent on the host cell used, the culture medium used may be selected from various conventional culture media. The host cells were cultured under conditions suitable for growing. Chemical and/or enzymatic methods used for releasing insulin analogue from the expressed precursor are conventional techniques well known to the skilled person in the art, such as use of trypsin, carboxypeptidase B, lysine endopeptidase C, etc.

### BRIEF DESCRIPTION

Figure 1 displays a schematic diagram for cloning recombinant human insulin B (1-30), A (1-21) precursor.
Figure 2 displays electrophoretic results after PCR amplification of the inserted recombinant human insulin B (1-30), A (1-21) precursor fragment.

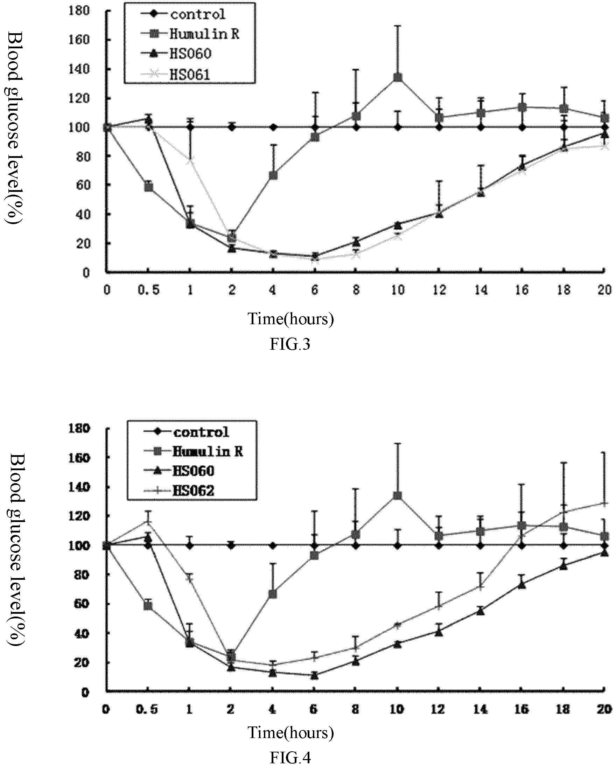

Figure 3 displays the comparative results of *in vivo* long-lasting activity assay of HS061 and HS060.
Figure 4 displays comparative results of *in vivo* long-lasting activity assay of HS062 and HS060.
Figure 5 displays comparative results of *in vivo* long-lasting activity assay of HS067 and HS060.
Figure 6 displays *in vivo* long-lasting activity assay of PEGylated (20kD) B (1-27)-D-K-E, A (1-21) (PEG (20kD)-156), and PEGylated (20kD) B(1-2)-K-B(4-28) -E-E, A(1-21) (PEG (20kD) -107).
Figure 7 displays *in vivo* long-lasting activity assay of PEG(30kD)-107, PEG (40kD)-107, PEG (30kD)-156, PEG (40kD)-156.

### DETAILED DESCRIPTION OF THE INVENTION

The present invention is further described by the following Examples which are not intended to limit the scope of the invention.

The reagent formulations used in the present invention are as follows:
YPD broth (1L) (1% yeast extract, 2% peptone, 2% glucose):
   1. 10g yeast extract, 20g peptone was dissolved in 900mL water;
   2. Autoclaved for 20min;
   3. 100mL of 20% sterile glucose was added.

### Basic glucose medium (1L) (1.34% YNB; 4 × 10⁻⁵% biotin; 2% glucose):

1. 800 mL water was autoclaved for 20min, (in order to prepare an Agar Plate, 15 ∼ 20g agar can be added prior to autoclave);
2. Cooled to 60°C, followed by adding 100mL sterile 10× YNB, 2mL sterile 0.02% biotin and 100mL of 20% sterile glucose.

### BMMY liquid medium (1L):

1. 10g of yeast extract, 20g peptone was dissolved in 700mL of water;
2. Autoclaved for 20min;
3.Cooled to room temperature, followed by adding and mixing the following substances:
   100mL sterile 1M potassium phosphate buffer, pH6.0; 100mL sterile 10× YNB, 2mL sterile 0.02% biotin, 100mL sterile 5% methanol. 1.

### Medium to induce the expression (1L) (BMGY):

1. 10g of yeast extract, 20g peptone was dissolved in 700mL of water;
2. Autoclaved for 20min;
3. Cooled to room temperature, followed by adding and mixing the following substances:
   100mL sterile 1M potassium phosphate buffer, pH6.0; 100mL sterile 10× YNB, 2mL sterile 0.02% biotin, 100mL sterile 10% glycerol.

The Examples described herein were generally performed in accordance with conventional conditions, e.g., Sambrook et al, Molecular Cloning: A Laboratory Manual (New York: cold spring harbor laboratory press, 1989); Pichia yeast Expression Laboratory Manual (version: 10 April 2009), or performed following the recommendation by the manufacturer of the product, if the specific conditions were not indicated. The reagents used herein were routinely purchased from the manufacturer. The Unit of molecular weight mentioned in the following examples of the present invention is daltons (Dalton).

### Examples

### Example 1. Preparation of recombinant human insulin B(1-30), A(1-21) and B(1-29), R-A(1-21)

### Step 1: Cloning and expression ofrecombinant human insulin B(1-30), A(1-21) precursors

### 1. Construction of expression vector having recombinant human insulin B(1-30), A(1-21) precursors:

The genes encoding for the recombinant human insulin B(1-30), A(1-21) precursors were synthesized by using three rounds of polymerase chain reaction (PCR). five single-stranded DNA fragments were synthesized by Invitrogen and were used as primers, the sequences of which are as follows:
Primer 1: 
5’ GTGCGGGGAACGAGGCTTCTTCTACACACCCAAGACCAAGCGTGGCATTG
3’ SEQ ID NO. 45
Primer 2: 
5’ GTAGAGGGAGCAGATGCTGGTACAGCATTGTTCCACAATGCCACGCTTGG
3’ SEQ ID NO. 46
Primer 3: 
5’ TGCGGCTCACACCTGGTGGAAGCTCTCTACCTAGTGTGCGGGGAACGAGG
3’ SEQ ID NO. 47
Primer 4: 
5’ CTGACTGAATTCTAGTTGCAGTAGTTCTCCAGCTGGTAGAGGGAGCAGAT
3’ SEQ ID NO. 48
Primer 5:

5’ ACTTGCTCGAGAAAAGATTTGTGAACCAACACCTGTGCGGCTCACACCTG
3’ SEQ ID NO. 49.

The first round of PCR was preformed by using KOD Synthesis Kit (TOYOBO, Cat KOD-201) 50µL system: 5µL 10 × KOD buffer, 2µL 2mM dNTPs, 1.5µL primer 1 (10µM), 1.5µL primer 2 (10µM), 0.5 µL KOD plus, 2µL 25mM MgSO₄, 38µL ddH₂O. The amplification program was 94°C 2min, 94°C 30sec, 55°C 30sec, 68°C 30sec, for 25 amplification cycles, followed by incubation at 68°C for 2 min. The PCR product having 85 nucleotides was synthesized, then identified with a 1.2% agarose gel and recovered.

The second round of PCR was performed in 50µL PCR System: 5µL 10 × KOD buffer, 2µL 2mM dNTPs, 1µL product 1, 1.5 µL primer 3 (10µM), 1.5µL Primer 4 (10µM), 0.5µL KOD plus, 2µL 25mM MgSO₄, 37µL ddH₂O. The amplification program was 94°C 2min, 94°C 30sec, 55°C 30sec, 68°C 30sec, for 25 amplification cycles, followed by incubation at 68°C for 2 min. The obtained PCR product 2 having 155 nucleotides was identified by 1.2% agarose gel and recovered.

The third round of PCR was performed in 50µL PCR System: 5µL 10 × KOD buffer, 2µL 2mM dNTPs, 1µL product 2, 1.5 µL primer 4 (10µM), 1.5µL Primer 5 (10µM), 0.5µL KOD plus, 2µL 25mM MgSO₄, 37µL ddH₂O. Program of the synthesis was 94°C 2min, 94°C 30sec, 55°C 30sec, 68°C 30sec, for 25 amplification cycles, followed by incubation at 68°C, for 2min. Product 3 was identified by 1.2% agarose gel and recovered.

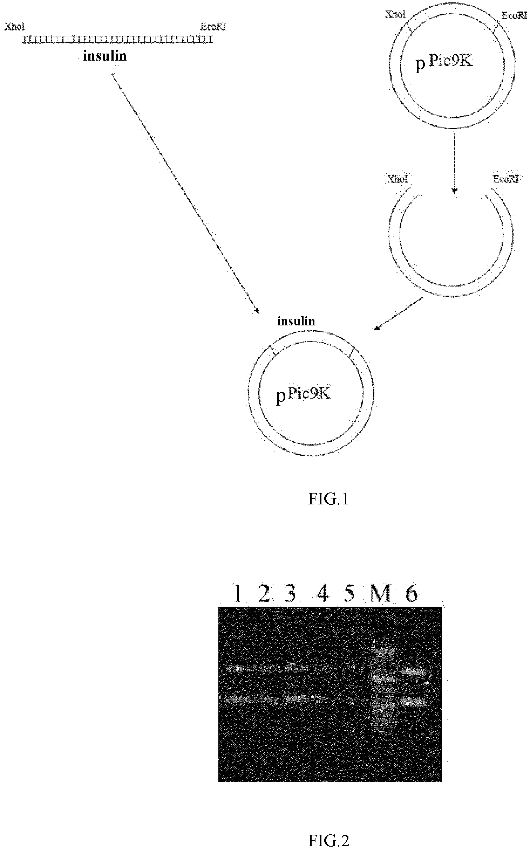

Product 3 was linked into T vector by T vector kit (Takara, Cat. D103A), and then was double digested by EcoR I/Xho I (New England Biolabs, Cat. R0101S/R0146V). The obtained fragment was recovered by 1.2% agarose gel, then was linked to pPIC9K expression vector (Invitrogen, Cat. K1750-01) using T4 ligase (New England Biolabs, Cat.M0202S). The structure was shown in Figure 1.

Sequence analysis of the resulted recombinant expression vector was conducted by Invitrogen. The product 3 was verified as a DNA fragment encoding the human insulin B (1-30), A (1-21) precursor, the sequence is as follows:

TTTGTGAACCAACACCTGTGCGGCTCACACCTGGTGGAAGCTCTCTACCTAG
TGTGCGGGGAACGAGGCTTCTTCTACACACCCAAGACCAAGCGTGGCATTG
TGGAACAATGCTGTACCAGCATCTGCTCCCTCTACCAGCTGGAGAACTACTG
CAACTAG SEQ ID NO. 32.

### 2. Transformation of the Expression vector comprising the recombinant human insulin B (1-30), A (1-21) precursor:

The 5-10µg expression vectors comprising recombinant human insulin precursor obtained from the above steps were linearized with Sail (Takata, Cat. D1080a), then added 1/10 volume of 3M sodium acetate and 2 volumes of anhydrous ethanol. After thoroughly mixed, the mixture was placed at -20°C, for 2hrs. The mixture was centrifuged at high speed (13,000 rpm) for 5min, removed the supernatant, and washed the pellet with 75% ethanol twice. The pellet was dried via upside down, and dissolved with 10µL ddH₂O. 80µL linearized plasmid and competent cells (Pichia pastoris GS115. Invitrogen, Cat. K1750-01) were added into the Electroporation Cuvette (Bio Rad, Cat. 1652086) on ice for 5min. The parameters of the Electroporator (Bio Rad Micropulser) were set to 2 kV, 25Ω, 200 uF. Electroporation was conducted. Then 1 mL cooled D-Sorbitol (Biological Engineering Co., Ltd.) was added quickly and mixed up, 100-300µL mixture was plated on a MD culture plate, and cultured at 30°C for 3 days, until colonies were formed.

### 3. Screening clones of recombinant human insulin precursor by using G418:

Colonies on MD culture plate were eluted with 3mL YPD broth, resuspended, and the concentration of resuspended cells (1 OD₆₀₀ = 5 × 10⁷ cells / mL) was measured with a spectrophotometer (Beckman, DU800). 1×10⁵ cells were plated on YPD culture plate with various concentrations of G418 (GIBCO, Cat. 11811-031) (0.25. 0.5. 1.0, 2.0, 4.0 mg/mL), cultured at 30°C for 5 days, until colonies were formed. 30 single-clones were selected from the five plates with different G418 concentrations, and verified by PCR of recombinant fragments (Polymerase chain reaction).

### 4. Identification of clones with inserted recombinant human insulin B(1-30), A(1-21) precursor by PCR :

Fragments inserted into G418-resistant colonies were verified by PCR. The 18 µL broth was placed in 1.5mL tubes and 2 µL cytase (5 U / µL) (Sigma, Cat. L2524) was added, incubated at 30°C for 10min. The sample was then placed at -80°C, 10min for complete lysis. The identification was performed by using KOD kit (TOYOBO, Cat KOD-201.) 25 µL PCR System: 2.5 µL 10×reaction buffer, 1.5 µL 25 mM MgCl₂, 2.5 µL 2mM dNTPs, 1 µL 5 'AOX1 primer (10 pmol/µL), 1 µL 3 'AOX1 primer (10 pmol / µL), 0.5µL KOD polymerase, 15 µL ddH₂O, 1 µL lysate buffer. The mixture was placed in a PCR instrument system (Eppendorf, 22331 type), the amplification program was, 94°C 30sec, 55°C 30sec, 68°C 4min, for 25 amplification cycles, followed by incubation at 68°C for 10min. 10 µL PCR products were identified by 1.0% agarose gel (Sigma, A9539). The results were shown in Figure 2, two distinct amplified bands were obviously observed. The larger band of 2.2 Kb corresponds to AOX gene carried by GS1115 per se, and the smaller one of 635 bp corresponds to inserted foreign gene. Lane 6 of Figure 2 corresponds to clone No. 1001-17. Clone no 1001-17 was chosen for further use.

### 5. Expression and characterization of the recombinant human insulin B (1-30), A (1-21) precursor:

The single colony of Clone 1001-17 was cultured in 50mL BMGY medium, at 30°C, 250 rpm, overnight. On the next day, OD600 value detected should be between 2-6. At room temperature, centrifuged (Beckman Coulter) at low speed(1,500 g) for 5min, the cells were collected and resuspended with BMMY medium to an OD600 of 1.0. 1/200 of the total volume of 100% methanol was added to the medium with a final concentration of 0.5%. The medium was then cultured at 28°C, 250 rpm for 72hr, during the period of culture, 1/200 of the total volume of 100% methanol was added every 24hr. After induction, the medium was centrifuged with low speed (1,500 g) and the supernatant was collected. The expression of Clone 1001-17 precursor protein was verified by SDS-PAGE (Invitrogen, Cat. No. 456-1083). The clone No.1001-17 was selected for the next fermentation.

### Step 2: Fermentation of recombinant human insulin B (1-30), A (1-21) precursor

### 1. Strain: expression strain No.1001-17.

### 2. Operation process for 5 L fermentor:

### 2.1 Activation and culture of the strain:

1mL Glycerol Stock of the said strain was inoculated in 100mL BMGY medium, at 30°C, 220r/min (rpm) for 20hrs.

### 2.2 Inoculation:

Inoculum size was 5%, and 0.4% sterilized PTM1 solution was added. Fermentation was started.

### 2.3 The initial fermentation stage:

After a period of adaptive phase (10-12hr), strain fermentation enters into the exponential growth phase. Agitation speed and aeration were continuously increased to meet the requirement of DO>30% for cell growth. Agitation speed was increased by 50-100rpm every time. 25% industrial ammonia was added via automatically feeding to fix pH to setting value.

### 2.4 growth phase in glycerol feeding:

When substrate in initial medium was consumed (18-24hrs), 50% glycerol was added via fed-batch, in a restrictive rate.

### 2.5 methanol induction phase:

After 4∼6hrs of glycerol transient cell growth phase, glycerol feeding was stopped. Starvation was maintained for 30min to consume the rest glycerol completely. Then methanol was added to start induction. After 96hrs, the fermentation was stopped.

### Step 3: Isolation and purification of fermentation product

The obtained fermentation broth was ultrafiltrated conventionally, and purified by SP Sepharose FF (GE, cast.17-0729-10) and Q Sepharose FF (GE, cast.17-0510-10) column. The purified product was analyzed by HPLC (Waters e2695-2489 liquid meter, Column: phenomenex, Jupiter, C18, 250 x 4.6 mm, 5µm, 300Å), the purity is greater than 90% purity. The sequence of resulted expression precursor is as follows:
FVNQHLCGSHLVEALYLVCGERGFFYTPKTKRGIVEQCCTSICSLYQLENYCN SEQ ID NO. 22

The molecular weight of the obtained product detected by LC-MS (liquid MS) is 6074, which is consistent with the theoretically predicted molecular weight of 6074.

### Step 4: Digestion and purification of fermentation product

The above purified product was adjusted to pH9.0 with 50mM Tris, digested by trypsin (sigma, Cat.T1426) and CPB (Sigma, C9584). Reaction process was analyzed with HPLC. After complete digestion, the pH value was adjusted to 2 with 1M HCL and the reaction was terminated. Two insulin products were prepared by reverse phase (Jupiter C4, 10 µm, 300Å, 50 × 250 mm) as follws:
Product 1: recombinant human insulin B (1-30), A (1-21) (referred as hI):
   FVNQHLCGSHLVEALYLVCGERGFFYTPKT, SEQ ID NO. 5
   GIVEQCCTSICSLYQLENYCN SEQ ID NO. 1

The molecular weight of the obtained product detected by LC-MS is 5807, which is consistent with the theoretically predicted molecular weight of 5807.7.
Product 2: recombinant human insulin B (1-29), R-A (1-21):
   FVNQHLCGSHLVEALYLVCGERGFFYTPK SEQ ID NO. 78
   RGIVEQCCTSICSLYQLENYCN SEQ ID NO. 4

The molecular weight of the obtained product detected by LC-MS is 5863, which is consistent with the theoretically predicted molecular weight of 5862.7.

### Step 5: Analysis of disulfide bond structure in the cleaved products

The above-prepared product 1 and product 2 was digested with GluC (Sigma, P6181) at 37°C for 4hrs. 2µl of 1M HCl was added to terminate the reaction. Product was analyzed by LC-MS. The results were as follows:

### Analysis of disulfide bond structure in Human insulin B (1-30), A (1-21) :

| Fragment NO | Peptide sequence | LC-MS Mol. wt. | Theoretical Mol. wt. |
|---|---|---|---|
| I | GIVE | 416 | 416.47 |
| II | QCCTSICSLYQLE | 2969 | 2969.39 |
| | FVNQHLCGSHLVE | | |
| III | NYCN ALYLVCGE | 1377 | 1377.55 |
| IV | RGFFYTPKT | 1116 | 1116.28 |

The above results confirmed that in recombinant human insulin B (1-30), A (1-21), the configurations of disulfide bonds are as follows: one formed between A20 and B19; the other two are formed between any two selected fromA6 Cys, A7 Cys, A11 Cys and B7 Cys.

### Analysis disulfide bond structure in Human insulin B (1-29), R-A (1-21)

| Fragment NO | Peptide sequence | LC-MS Mol. wt. | Theoretical Mol. wt. |
|---|---|---|---|
| I | RGIVE | 573 | 572.66 |
| II | QCCTSICSLYQLE | 2969 | 2969.39 |
| | FVNQHLCGSHLVE | | |
| III | NYCN ALYLVCGE | 1377 | 1377.55 |
| IV | RGFFYTPK | 1015 | 1015.18 |

These results confirmed that the configurations of disulfide bonds in recombinant human insulin B (1-29), R-A (1-21) are as follows: one is formed between A20 and B19; the other twos are formed between any two selected from A6 Cys, A7 Cys, and A11 Cys and B7 Cys.

### Example 2 Preparation of recombinant human insulin B (1-29), A (1-21)

### Step 1: Cloning and expression of recombinant human insulin B (1-29), A (1-21) precursor

### 1. Construction of expression vector comprising recombinant human insulin B (1-29), A (1-21) precursor:

B (1-29), A (1-21) was obtained via site-directed mutagenesis by using polymerase chain reaction (PCR). Two single-stranded DNA fragments were synthesized by Invitrogen and used as primers for site-directed mutagenesis, sequences of the primers are as follows:
Primer 1: 5' CTTCTACACACCCAAGCGTGGCATTGTGGAAC 3' SEQ ID NO. 50
Primer 2: 5' GTTCCACAATGCCACGCTTGGGTGTGTAGAAG 3' SEQ ID NO. 51

The recombinant vector finally obtained from Example 1 was used as a template in the procedure of site-directed mutagenesis. KOD kit (TOYOBO, Cat KOD-201.) 25µL system was applied: 2.5µL 10×KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by directly adding 1µL of DpnI (NEB, Cat. R0176L), and was transformed into TOP10 competent cells to obtain an interest plasmid. The resulted recombinant expression vector was delivered to Invitrogen for sequence analysis.

### 2. Result of sequence analysis:

The linked recombinant plasmid was analyzed by Invitrogen. The sequences of obtained DNA encoding the human insulin B (1-29), A (1-21) precursor were as follows:

TTTGTGAACCAACACCTGTGCGGCTCACACCTGGTGGAAGCTCTCTACCTAG
TGTGCGGGGAACGAGGCTTCTTCTACACACCCAAGCGTGGCATTGTGGAACA
ATGCTGTACCAGCATCTGCTCCCTCTACCAGCTGGAGAACTACTGCAACTAG
SEQ ID NO. 33.

### 3. Transformation of the expression vector comprising recombinant human insulin B(1-29), A(1-21) precursor via electroporation:

The Expression vector comprising recombinant human insulin precursor was transformed as described in Example 1.

### 4. Screening the clone of recombinant human insulin B(1-29), A(1-21) precursor by using G418:

The clone of recombinant human insulin precursor was screened as described in Example 1.

### 5. Identification of clones with inserted fragments of recombinant human insulin precursor by using PCR:

Inserted fragments of recombinant human insulin precursor were verified as described in Example 1. Clone 006-15 was selected for further use.

### 6. Expression and characterization of recombinant human insulin B(1-29), A(1-21) precursor:

Recombinant human insulin precursor was expressed and identified as described in Example 1. The results showed that clone No. 006-15 expressed the interest protein, and was selected for following fermentation.

### Step 2: Fermentation of recombinant human insulin B (1-29), A (1-21) precursor

No. 006-15 clone was selected for fermentation. The fermentation method was the same as that described in Example 1.

### Step 3: Isolation and purification of fermentation product

The fermentation product was isolated and purificated as described in Example 1. The resulted precursor was as follows:
FVNQHLCGSHLVEALYLVCGERGFFYTPKRGIVEQCCTSICSLYQLENYCN SEQ ID NO. 23.

The molecular weight of the obtained product detected by LC-MS is 5844.7, and the theoretically predicted molecular weight is 5846.2.

### Step 4: Enzymatic digestion and purification of fermentation product

Method of enzymatic digestion and purification are as described in Example 1. After optimization of the enzymatic reaction system, enzymatically digested products prepared by reverse-phase were as follows:

Recombinant human insulin B (1-29), A (1-21) (referred as Des (B30)-hI):
FVNQHLCGSHLVEALYLVCGERGFFYTPK, SEQ ID NO. 78
GIVEQCCTSICSLYQLENYCN SEQ ID NO. 1.

The molecular weight of the obtained product detected by LC-MS is 5578, which is consistent with the theoretically predicted molecular weight of 5578.

### Step 5: Analysis of disulfide bond structure in the cleaved products

Structure of disulfide bond in cleaved products was analyzed as described in Example 1.

Results from analysis of Disulfide bond structure in Human insulin B(1-29), A(1-21) were as follows:

| Fragment NO | Peptide sequence | LC-MS Mol. wt. | Theoretical Mol. wt. |
|---|---|---|---|
| I | GIVE | 417 | 416.47 |
| II | QCCTSICSLYQLE | 2971 | 2969.39 |
| | FVNQHLCGSHLVE | | |
| III | NYCN ALYLVCGE | 1378 | 1377.55 |
| IV | RGFFYTPK | 1015 | 1015 |

These results confirmed that the configurations of disulfide bonds in the recombinant human insulin B (1-29), A (1-21) are as follows: one is formed between A20 and B19; the other two are formed between any two selected from A6 Cys, A7 Cys, and A11 Cys and B7 Cys.

### Example 3. Preparation of recombinant human insulin (1-27)-K-E-R, A(1-21)

### Step 1: Cloning and expression of recombinant human insulin (1-27)-K-E-R, A(1-21) precursor

### 1. Construction of expression vector comprising recombinant human insulin (1-27)-K-E-R, A(1-21) precursor:

B(1-27)-K-E-R, A(1-21) was mutated by using polymerase chain reaction (PCR). Two single-stranded DNA fragments were synthesized by Invitrogen and were used as primers for site-directed mutagenesis, the sequences of the primers are as follows:
Primer 1:
   5' GGTTTCTTTTACACCAAGGAAAGAGCTGCTAAAGGTATCGTTGAGC 3' SEQ ID NO. 52
Primer 2:
   5' GCTCAACGATACCTTTAGCAGCTCTTTCCTTGGTGTAAAAGAAACC 3' SEQ ID NO. 53

The recombinant vector finally obtained in Example 1 was used as template in procedure of site-directed mutagenesis. KOD kit (TOYOBO, Cat KOD-201.) 25µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows:94°C 2min, 94°C 30sec, 55°C 30sec, 68°C 11min, for 25 amplification cycles, followed by incubation for 11 min at 68°C. PCR product was digested for 1hr by directly adding 1µL of DpnI (NEB, Cat. R0176L), and then was transformed into TOP10 competent cells to obtain interest plasmid. The resulted recombinant expression vector was delivered to Invitrogen for sequence analysis.

### 2. Result of sequence analysis:

The linked recombinant plasmid was analyzed by Invitrogen. Sequence of DNA fragment encoding the human insulin B(1-27)-K-E-R, A(1-21) precursor was as follows:

TTCGTCAACCAGCACTTGTGTGGTTCCCATTTGGTTGAGGCTCTGTACTTGGT
CTGTGGAGAAAGAGGTTTCTTTTACACCAAGGAAAGAGCTGCTAAAGGTATC
GTTGAGCAATGTTGCACCTCTATTTGTTCCCTGTATCAGTTGGAAAACTACTG
CAACTAA SEQ ID NO. 34.

### 3 Transformation of expression vector comprising recombinant human insulin B(1-27)-K-E-R, A(1-21) precursor via Electroporation:

Expression vector comprising recombinant human insulin precursor was transformed as described in Example 1.

### 4. Screening clone of recombinant human insulin B(1-27)-K-E-R, A(1-21) precursor by using G418:

Clone of recombinant human insulin precursor was screened as described in Example 1.

### 5. Identification of clones with inserted recombinant human insulin precursor fragments by PCR:

Inserted fragments of recombinant human insulin precursor clone were verified as described in Example 1. Clone 064-6 was selected for further use.

### 6. Expression and characterization of recombinant human insulin B(1-27)-K-E-R, A(1-21) precursor:

The method was the same as that described in Example 1. The results showed that clone No. 064-6 expressed the interest protein, and was selected for the following fermentation.

### Step 2: Fermentation of recombinant human insulin precursor

No. 064-6 clone was selected for fermentation. The fermentation method was as described in Example 1.

### Step 3:Isolation and purification of fermentation product

The fermentation product was isolated and purificated as described in Example 1. The resulted precursor was as follows:

FVNQHLCGSHLVEALYLVCGERGFFYTKERAAKGIVEQCCTSICSLYQLEN
YCN SEQ ID NO. 24.

The molecular weight of the obtained product detected by LC-MS is 6147, which is consistent with the theoretically predicted molecular weight of 6147.

### Step 4: Enzymatic digestion and purification of fermentation product

The above purified product was adjusted to pH9.0 with 50mM Tris, digested by trypsin (sigma, Cat.T1426). Reaction process was analyzed with HPLC. After complete digestion, pH value was adjusted to 2 with 1M HCL to terminate the reaction. The digested products prepared by reverse phase (Jupiter C4, 10 µm, 300Å, 50 × 250 mm) were as follows:
Human insulin B (1-27)-K-E-R, A (1-21):
   FVNQHLCGSHLVEALYLVCGERGFFYTKER, SEQ ID NO. 6
   GIVEQCCTSICSLYQLENYCN SEQ ID NO. 1.

The molecular weight of the obtained product detected by LC-MS is 5894, which is consistent with the theoretically predicted molecular weight of 5894.7.

### Step 5: Analysis of disulfide bond structure in cleaved products

Structure of disulfide bond in cleaved products was analyzed as described in Example 1.

Results from analysis of disulfide bond structure in Human insulin B(1-27)-K-E-R, A(1-21) were as follows:

| Fragment NO | Peptide sequence | LC-MS Mol. wt. | Theoretical Mol. wt. |
|---|---|---|---|
| I | GIVE | 417 | 416.47 |
| II | QCCTSICSLYQLE | 2971 | 2969.39 |
| | FVNQHLCGSHLVE | | |
| III | NYCN ALYLVCGE | 1377 | 1377.55 |
| IV | RGFFYTKER | 1203 | 1203.37 |

These results confirmed that the configurations of disulfide bonds in recombinant human insulin B(1-27)-K-E-R, A(1-21) are as follows: one is formed between A20 and B19; the other two are formed by any two selected from A6 Cys, A7 Cys, A11 Cys and B7 Cys.

### Example 4. Preparation of recombinant human insulin B(1-27)-K-E, A(1-21)

### Step 1: Cloning and expression of recombinant human insulin B(1-27)-K-E, A(1-21) precursor

### 1. Construction of expression vector comprising recombinant human insulin precursor:

B(1-27)-K-E, A(1-21) was obtained by site-directed mutation via polymerase chain reaction (PCR). Two single-stranded DNA fragments were synthesized by Invitrogen and used as primers for site-directed mutagenesis, the sequences are as follows:
Primer 1: 5' GGTTTCTTTTACACCAAGGAAAAAAGAGGTATCGTTG 3' SEQ ID NO. 54
Primer 2: 5' CAACGATACCTCTTTTTTCCTTGGTGTAAAAGAAACC 3' SEQ ID NO. 55.

The recombinant vector finally obtained in Example 1 was used as template in site-directed mutagenesis procedure. KOD kit (TOYOBO, Cat KOD-201.) 25 µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows, 94°C 2min, 94°C 30sec, 55°C 30sec, 68°C 11min, for 25 amplification cycles, followed by incubation for 11 min at 68°C. PCR product was digested for 1hr by directly adding 1µL of DpnI (NEB, Cat. R0176L), and then was transformed into TOP10 competent cells to obtain interest plasmid. The resulted recombinant expression vector was delivered to Invitrogen for sequence analysis.

### 2. Result of sequence analysis:

The linked recombinant plasmid was analyzed by Invitrogen. The sequence of DNA encoding the human insulin B(1-27)-K-E, A(1-21) precursor was as follows:

TTCGTCAACCAGCACTTGTGTGGTTCCCATTTGGTTGAGGCTCTGTACTTGGT
CTGTGGAGAAAGAGGTTTCTTTTACACCAAGGAAAAAGAGGTATCGTTGA
GCAATGTTGCACCTCTATTTGTTCCCTGTATCAGTTGGAAAACTACTGCAACT
AA SEQ ID NO.35.

### 3. Transformation of expression vector comprising recombinant human insulin B(1-27)-K-E, A(1-21) precursor via Electroporation:

Expression vector comprising recombinant human insulin precursor was transformed as Example 1.

### 4. Screening clones of recombinant human insulin B(1-27)-K-E, A(1-21) precursor by using G418:

Recombinant human insulin precursor clone was screened as Example 1.

### 5. Identification of clones with inserted recombinant human insulin precursor fragments by PCR:

Inserted fragments of recombinant human insulin precursor were verified as described in Example 1. Clone 062-6 was selected for further use.

### 6. Expression and characterization of recombinant human insulin B(1-27)-K-E, A(1-21) precursor:

Recombinant human insulin precursor was expressed and identified as in Example 1. The results showed that clone No. 062-6 expressed the interest protein, and was selected for following fermentation.

### Step 2: Fermentation of recombinant human insulin precursor

No. 062-6 clone was selected for fermentation. The fermentation method was as described in Example 1.

### Step 3: Isolation and purification of fermentation product

The fermentation product was isolated and purificated as described in Example 1. The resulted precursor was as follows:
FVNQHLCGSHLVEALYLVCGERGFFYTKEKRGIVEQCCTSICSLYQLENYC N SEQ ID NO. 25.

The molecular weight of the obtained product detected by LC-MS is 6004.91, which is consistent with the theoretically predicted molecular weight of 6006.3.

### Step 4: Enzymatic digestion and purification of fermentation product

Method of enzymatic digestion and purification is as described in Example 1. After optimization of the enzymatic reaction system, digested products prepared by reverse-phase were as follows:
Human insulin B(1-27)-K-E, A(1-21):
   FVNQHLCGSHLVEALYLVCGERGFFYTKE SEQ ID NO. 7
   GIVEQCCTSICSLYQLENYCN SEQ ID NO. 1.

The molecular weight of the obtained product detected by LC-MS is 5738.54, which is consistent with the theoretically predicted molecular weight of 5738.8.

### Step 5: Analysis of disulfide bond structure in cleaved products

Structure of disulfide bond in cleaved products was analyzed as described in Example 1.

Results from analysis of Disulfide bond structure Human insulin B(1-27)-K-E, A(1-21) were as follows:

| Fragment NO | Peptide sequence | LC-MS Mol. wt. | Theoretical Mol. wt. |
|---|---|---|---|
| I | GIVE | 417 | 416.47 |
| II | QCCTSICSLYQLE | 2968.8 | 2969.39 |
| | FVNQHLCGSHLVE | | |
| III | NYCN | 1377.7 | 1377.55 |
| | ALYLVCGE | | |
| IV | RGFFYTKE | 1047.6 | 1047.18 |

These results confirmed that the configurations of the disulfide bonds of recombinant human insulin B(1-27)-K-E, A(1-21) are as follows: one is formed between A20 and B19; the other two are formed by any two selected from A6 Cys, A7 Cys, A11 Cys and B7 Cys.

### Example 5. Preparation of recombinant human insulin B(1-27)-K-P-E, A(1-21)

### Step 1: Cloning and expression of recombinant human insulin B(1-27)-K-P-E, A(1-21) precursor

### 1. Construction of the expression vector comprising recombinant human insulin B(1-27)-K-P-E, A(1-21) precursor:

B(1-27)-K-P-E, A(1-21) was mutated via polymerase chain reaction (PCR). Two single-stranded DNA fragments were synthesized by Invitrogen and used as primers for site-directed mutagenesis, the sequences of the primers are as following:
Primer 1: 5' GGTTTCTTTTACACCAAGCCTGAAAAAAGAGGTATCGTTG 3' SEQ ID NO. 56
Primer 2: 5' CAACGATACCTCTTTTTTCAGGCTTGGTGTAAAAGAAACC 3' SEQ ID NO. 57.

The recombinant vector finally obtained in Example 1 was used as template in site-directed mutagenesis procedure. KOD kit (TOYOBO, Cat KOD-201.) 25µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows, 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis:

The linked recombinant plasmid was analyzed by Invitrogen. Sequence of the obtained DNA encoding the human insulin B(1-27)-K-P-E, A(1-21) precursor was as follows:

TTCGTCAACCAGCACTTGTGTGGTTCCCATTTGGTTGAGGCTCTGTACTTGGT
CTGTGGAGAAAGAGGTTTCTTTTACACCAAGCCTGAAAAAAGAGGTATCGTT
GAGCAATGTTGCACCTCTATTTGTTCCCTGTATCAGTTGGAAAACTACTGCAA
CTAA SEQ ID NO. 36.

### 3. Transformation of expression vector comprising recombinant human insulin B(1-27)-K-P-E, A(1-21) precursor via Electroporation:

Expression vector comprising recombinant human insulin precursor was transformed as Example 1.

### 4. Screening clones of recombinant human insulin B(1-27)-K-P-E, A(1-21) precursor by using G418:

Recombinant human insulin precursor clone was screened as Example 1.

### 5. Inserted fragments verification for recombinant human insulin precursor clone by PCR:

Inserted fragments of recombinant human insulin precursor clone were verified as Example 1. Clone 061-5 was selected for further use.

### 6. Expression and characterization of recombinant human insulin B(1-27)-K-P-E, A(1-21) precursor:

Recombinant human insulin precursor was expressed and identified as in Example 1. The results showed that clone No. 061-5 expressed the interest protein, and was selected for following fermentation.

### Step 2: Fermentation of recombinant human insulin B(1-27)-K-P-E, A(1-21) precursor

No. 061-5 clone was selected for fermentation. The fermentation method was as described in Example 1.

### Step 3: Isolation and purification of fermentation product

The fermentation product was isolated and purificated as described in Example 1. The resulted expression product precursor was as follows:

FVNQHLCGSHLVEALYLVCGERGFFYTKPEKRGIVEQCCTSICSLYQLENYC
N SEQ ID NO. 26.

The molecular weight of the obtained product detected by LC-MS is 6102, which is consistent with the theoretically predicted molecular weight of 6102.

### Step 4: Enzymatic digestion and purification of fermentation product

Method of Enzymatic digestion and purification is the same as described in Example 1. After optimization of the enzymatic reaction system, digested products prepared by reverse-phase were as follows:
Human insulin B(1-27)-K-P-E, A(1-21):
   FVNQHLCGSHLVEALYLVCGERGFFYTKPE, SEQ ID NO. 8
   GIVEQCCTSICSLYQLENYCN SEQ ID NO. 1.

The molecular weight of the obtained product detected by LC-MS is 5835, which is consistent with the theoretically predicted molecular weight of 5835.7.

### Step 5: Analysis of disulfide bond structure in cleaved products

Structure of disulfide bond in cleaved products was analyzed as described in Example 1.

Results obtained from analysis of disulfide bond structure in Human insulin B(1-27)-K-P-E, A(1-21) were as follows:

| Fragment NO | Peptide sequence | LC-MS Mol. wt. | Theoretical Mol. wt. |
|---|---|---|---|
| I | GIVE | 416 | 416.47 |
| II | QCCTSICSLYQLE | 2969 | 2969.39 |
| | FVNQHLCGSHLVE | | |
| III | NYCN | 1376.8 | 1377.55 |
| | ALYLVCGE | | |
| IV | RGFFYTKPE | 1143.6 | 1143.28 |

These results confirmed that the configurations of the disulfide bonds of recombinant human insulin B(1-27)-K-P-E, A(1-21) are as follows: one is formed between A20 and B19; the other two are formed by any two selected from A6 Cys, A7 Cys, A11 Cys and B7 Cys.

### Example 6. Preparation of recombinant human insulin B(1-2)-K-B(4-28)-E-E, A(1-21)

### Step 1: Coning and expression of recombinant human insulin B(1-2)-K-B(4-28)-E-E, A(1-21) precursor

### 1. Construction of the expression vector comprising recombinant human insulin precursor:

B(1-2)-K-B(4-28)-E-E, A(1-21) was mutated via site-directed mutagenesis by polymerase chain reaction (PCR). Single-stranded DNA fragments were synthesized by Invitrogen and used as primers for site-directed mutagenesis, the sequences of the primers are as following:
Primer 1: 5' GAGAAAAGATTCGTCAAGCAGCACTTGTGTGG 3' SEQ ID NO. 58
Primer 2: 5' CCACACAAGTGCTGCTTGACGAATCTTTTCTC 3' SEQ ID NO. 59
Primer 3: 5' GTTTCTTTTACACCCCTGAAGAAAAAAGAGGTATCGTTG 3' SEQ ID NO. 60
Primer 4: 5' CAACGATACCTCTTTTTTCTTCAGGGGTGTAAAAGAAAC 3' SEQ ID NO. 61.

The recombinant vector finally obtained in Example 1 was used as template in site-directed mutagenesis procedure. KOD kit (TOYOBO, Cat KOD-201.) 25µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmid was analyzed by Invitrogen. Sequence of the obtained DNA encoding the human insulin B(1-2)-K-B(4-28)-E-E, A(1-21) precursor was as follows:

TTCGTCAAGCAGCACTTGTGTGGTTCCCATTTGGTTGAGGCTCTGTACTTGGT
CTGTGGAGAAAGAGGTTTCTTTTACACCCCTGAAGAAAAAGAGGTATCGTT
GAGCAATGTTGCACCTCTATTTGTTCCCTGTATCAGTTGGAAAACTACTGCAA
CTAA SEQ ID NO.37.

### 3. Transformation for expression vector comprising recombinant human insulin B(1-2)-K-B(4-28)-E-E, A(1-21) precursor via Electroporation:

Expression vector comprising recombinant human insulin precursor was transformed as Example 1.

### 4. Screening clones of recombinant human insulin B(1-2)-K-B(4-28)-E-E, A(1-21) precursor by using G418:

Recombinant human insulin precursor clone was screened as Example 1.

### 5. Identification of clones with inserted recombinant human insulin precursor fragment by PCR:

Inserted fragments of recombinant human insulin precursor were verified as Example 1. Clone 070-4 was selected for further use.

### 6. Expression and characterization of recombinant human insulin B(1-2)-K-B(4-28)-E-E, A(1-21) precursor:

Recombinant human insulin precursor was expressed and identified as in Example 1. The results showed that clone No. 070-4 expressed the interest protein, and was selected for following fermentation.

### Step 2: Fermentation of recombinant human insulin B(1-2)-K-B(4-28)-E-E, A(1-21) precursor

No. 070-4 clone was selected for fermentation. The fermentation method was as described in Example 1.

### Step 3: Isolation and purification of fermentation product

The fermentation product was isolated and purificated as described in Example 1. The resulted expression product precursor was as follows:
FVKQHLCGSHLVEALYLVCGERGFFYTPEEKRGIVEQCCTSICSLYQLENYC N SEQ ID NO. 21.

The molecular weight of the obtained product detected by LC-MS is 6117, which is consistent with the theoretically predicted molecular weight of 6117.

### Step 4: Enzymatic digestion and purification of fermentation product

Method of Enzymatic digestion and purification was as described in Example 1. After optimization of the enzymatic reaction system, digested products prepared by reverse-phase were as follows:
Human insulin B(1-2)-K-B(4-28)-E-E, A(1-21):
   FVKQHLCGSHLVEALYLVCGERGFFYTPEE, SEQ ID NO.9
   GIVEQCCTSICSLYQLENYCN SEQ ID NO. 1.

The molecular weight of the obtained product detected by LC-MS is 5850, which is consistent with the theoretically predicted molecular weight of 5850.7.

### Step 5: Analysis of disulfide bond structure in cleaved products

Structure of disulfide bond in cleaved products was analyzed as described in Example 1.

Results obtained from analysis of disulfide bond structure in Human insulin B(1-2)-K-B(4-28)-E-E, A(1-21) were as follows:

| Fragment NO | Peptide sequence | LC-MS Mol. wt. | Theoretical Mol. wt. |
|---|---|---|---|
| I | GIVE | 417 | 416.47 |
| II | QCCTSICSLYQLE | 2982.8 | 2983.46 |
| | FVKQHLCGSHLVE | | |
| III | NYCN | 1376.5 | 1377.55 |
| | ALYLVCGE | | |
| IV | RGFFYTPEE | 1145.5 | 1144.3 |

These results confirmed the configurations of the disulfide bonds of recombinant human insulin B(1-2)-K-B(4-28)-E-E, A(1-21) are as follows: one is formed between A20 and B19; the other two are formed by any two selected from A6 Cys, A7 Cys, A11 Cys and B7 Cys.

### Example 7. Preparation of recombinant human insulin B(1-27)-D-K-E, A(1-21) and B(1-27)-D-K-E, A(1-20)-G

### Part I: Cloning and expression of recombinant human insulin B(1-27)-D-K-E, A(1-21)

### Step 1. Construction of the expression vector comprising recombinant human insulin precursor:

B(1-27)-D-K-E, A(1-21) was mutated via site-directed mutation by using polymerase chain reaction (PCR). Single-stranded DNA fragments were synthesized by Invitrogen and used as primers for site-directed mutagenesis, the sequences of the primers are as following:
Primer 1: 5' GGTTTCTTTTACACCGATAAGGAAAAAAGAGGTATCGTTG 3' SEQ ID NO.62
Primer 2: 5' CAACGATACCTCTTTTTTCCTTATCGGTGTAAAAGAAACC 3' SEQ ID NO.63.

The recombinant vector finally obtained in Example 1 was used as template in site-directed mutagenesis procedure. KOD kit (TOYOBO, Cat KOD-201.) 25 µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmid was analyzed by Invitrogen. Sequence of the obtained DNA encoding the human insulin B(1-27)-D-K-E, A(1-21) precursor was as follows:

TTCGTCAACCAGCACTTGTGTGGTTCCCATTTGGTTGAGGCTCTGTACTTGGT
CTGTGGAGAAAGAGGTTTCTTTTACACCGATAAGGAAAAAGAGGTATCGTT
GAGCAATGTTGCACCTCTATTTGTTCCCTGTATCAGTTGGAAAACTACTGCAA
CTAA SEQ ID NO.38.

### 3. Transformation for expression vector comprising recombinant human insulin B(1-27)-D-K-E, A(1-21) precursor via electroporation:

Expression vector comprising recombinant human insulin precursor B(1-27)-D-K-E, A(1-21) was transformed as Example 1.

### 4. Screening clones of recombinant human insulin B(1-27)-D-K-E, A(1-21) precursor by using G418:

Recombinant human insulin precursor clone was screened as Example 1.

### 5. Identification of clones with inserted recombinant human insulin precursor by PCR:

Inserted fragments of recombinant human insulin precursor clone were verified as Example 1. Clone 068-4 was selected for further use.

### 6. Expression and characterization of recombinant human insulin B(1-27)-D-K-E, A(1-21) precursor:

Recombinant human insulin precursor was expressed and identified as in Example 1. The results showed that clone No. 068-4 expressed the interest protein, and was selected for following fermentation.

### Step 2: Fermentation of recombinant human insulin B(1-27)-D-K-E, A(1-21) precursor

No. 068-4 clone was selected for fermentation. The fermentation method was as described in Example 1.

### Step 3: Isolation and purification of fermentation product

The fermentation product was isolated and purificated as described in Example 1. The resulted expression product precursor was as follows:

FVNQHLCGSHLVEALYLVCGERGFFYTDKEKRGIVEQCCTSICSLYQLENY
CN SEQ ID NO.19.

The molecular weight of the obtained product detected by LC-MS is 6119.99, which is consistent with the theoretically predicted molecular weight of 6119.5.

### Step 4: Enzymatic digestion and purification of fermentation product

Methods of Enzymatic digestion and purification are as described in Example 1. After optimization of the enzymatic reaction system, digested products prepared by reverse-phase were as follows:
Human insulin B(1-27)-D-K-E, A(1-21):
   FVNQHLCGSHLVEALYLVCGERGFFYTDKE, SEQ ID NO.10
   GIVEQCCTSICSLYQLENYCN SEQ ID NO. 1.

The molecular weight of the obtained product detected by LC-MS is 5853.6. which is consistent with the theoretically predicted molecular weight of 5853.

### Step 5: Analysis of disulfide bond structure in cleaved products

### Structure of disulfide bond in cleaved products was analyzed as described in Example 1.

Results obtained from analysis of disulfide bond structure in Human insulin B(1-27)-D-K-E, A(1-21) were as follows:

| Fragment NO | Peptide sequence | LC-MS Mol. wt. | Theoretical Mol. wt. |
|---|---|---|---|
| I | GIVE | 417.2 | 416.47 |
| II | QCCTSICSLYQLE | 2968.8 | 2969.39 |
| | FVNQHLCGSHLVE | | |
| III | NYCN | 1378.3 | 1377.55 |
| | ALYLVCGE | | |
| IV | RGFFYTDKE | 1162.6 | 1162.27 |

These results confirmed that the configurations of the disulfide bonds of recombinant human insulin B(1-27)-D-K-E, A(1-21) are as follows: one is formed between A20 and B19; the other two are formed by any two selected from A6 Cys, A7 Cys, A11 Cys and B7 Cys.

### Part II: Preparation of recombinant human insulin B (1-27)-D-K-E, A (1-20)-G

Repeated the steps 1 to 3 of the preparation of recombinant human insulin B (1-27)-D-K-E, A (1-21), except that the primer and the template used are as follows:
1. The primers for site-directed mutagenesis in Step 1 have the sequences as follows:
   Primer 1: 5' GTTGGAAAACTACTGCGGTTAAGAATTCCCTAGG 3' SEQ ID NO.66
   Primer 2: 5' CCTAGGGAATTCTTAACCGCAGTAGTTTTCCAAC 3' SEQ ID NO.67.

   Recombinant vector comprising B(1-27)-D-K-E, A (1-21) (SEQ ID NO.38) was as the template in site-directed mutagenesis procedure.
**2.** No.115-1 clone was selected for fermentation. The fermentation method was as described in Example 1.
**3.** The fermentation product was isolated and purificated as described in Example 1. The resulted expression product precursor was as follows: 
FVNQHLCGSHLVEALYLVCGERGFFYTDKEKRGIVEQCCTSICSLYQLENY
CG SEQ ID NO.77.
**4.** Methods of Enzymatic digestion and purification are as described in Example 1. After optimization of the enzymatic reaction system, digested products prepared by reverse-phase were as follows:
   Human insulin B(1-27)-D-K-E, A(1-20)-G:
      FVNQHLCGSHLVEALYLVCGERGFFYTDKE, SEQ ID NO.10
      GIVEQCCTSICSLYQLENYCG SEQ ID NO.2.
**5.** Structure of disulfide bond in cleaved products was analyzed as described in Example 1. The result confirmed that the product obtained in the example has correct configuration.

### Example 8. Preparation of recombinant human insulin B(1-2)-D-B(4-30)-R-R, A(1-20)-G and B(1-2)-D-B(4-30)-R, R-A(1-20)-G

### Step 1: Cloning and expression of recombinant human insulin B(1-2)-D-B(4-30)-R-R, A(1-20)-G and B(1-2)-D-B(4-30)-R, R-A(1-20)-G precursors

### 1. Construction of the expression vector comprising recombinant human insulin precursors:

B(1-2)-D-B(4-30)-R-R, A(1-20)-G and B(1-2)-D-B(4-30)-R, R-A(1-20)-G precursors were mutated via site-directed mutagenesis by using polymerase chain reaction (PCR). Four single-stranded DNA fragments were synthesized by Invitrogen and used as primers for site-directed mutagenesis, the sequences of the primers are as following:
Primer 1: 5' GGTTTCTTTTACACCCCTAAGACTAGAAGAGGTATCGTTGAG 3' SEQ ID NO.64
Primer 2: 5' CTCAACGATACCTCTTCTAGTCTTAGGGGTGTAAAAGAAACC 3' SEQ ID NO.65
Primer 3: 5' GTTGGAAAACTACTGCGGTTAAGAATTCCCTAGG 3' SEQ ID NO.66
Primer 4: 5' CCTAGGGAATTCTTAACCGCAGTAGTTTTCCAAC 3' SEQ ID NO.67.

KOD kit (TOYOBO, Cat KOD-201.) 25µL system was applied in site-directed mutagenesis procedure: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmids were analyzed by Invitrogen. The sequences of the obtained DNA fragments encoding the human insulin B(1-2)-D-B(4-30)-R-R, A(1-20)-G and B(1-2)-D-B(4-30)-R, R-A(1-20)-G precursors are as follows:

TTCGTCGATCAGCACTTGTGTGGTTCCCATTTGGTTGAGGCTCTGTACTTGGT
CTGTGGAGAAAGAGGTTTCTTTTACACCCCTAAGACTAGAAGAGGTATCGTT
GAGCAATGTTGCACCTCTATTTGTTCCCTGTATCAGTTGGAAAACTACTGCGG
TTAA SEQ ID NO.39.

### 3. Transformation of expression vector comprising recombinant human insulin B(1-2)-D-B(4-30)-R-R, A(1-20)-G and B(1-2)-D-B(4-30)-R, R-A(1-20)-G precursors via electroporation:

Expression vector comprising recombinant human insulin precursor was transformed as Example 1.

### 4. Screening clones of recombinant human insulin B(1-2)-D-B(4-30)-R-R, A(1-20)-G and B(1-2)-D-B(4-30)-R, R-A(1-20)-G precursors by using G418:

Clones of recombinant human insulin precursors were screened as Example 1.

### 5. Identification of clones with inserted fragments of recombinant human insulin precursor by PCR:

Inserted fragments of recombinant human insulin precursor were verified as Example 1. Clone 073-16 was selected for further use.

### 6. Expression and characterization of recombinant human insulin B(1-2)-D-B(4-30)-R-R, A(1-20)-G and B(1-2)-D-B(4-30)-R, R-A(1-20)-G precursors:

Recombinant human insulin precursor was expressed and identified as in Example 1. The results showed that clone No. 073-16 expressed the interest protein, and was selected for following fermentation.

### Step 2: Fermentation of recombinant human insulin precursor

No. 073-16 clone was selected for fermentation. The fermentation method was as described in Example 1.

### Step 3: Isolation and purification of fermentation product

The fermentation product was isolated and purificated as described in Example 1. The resulted product was as follows:
FVDQHLCGSHLVEALYLVCGERGFFYTPKTRRGIVEQCCTSICSLYQLENYCG SEQ ID NO.27.

The molecular weight of the obtained product detected by LC-MS is 6046.3, which is consistent with the theoretically predicted molecular weight of 6045.96.

### Step 4: Enzymatic digestion and purification of fermentation product

Methods of Enzymatic digestion and purification are as described in Example 3. After optimization of the enzymatic reaction system, digested products prepared by reverse-phase were as follows:
Product 1: human insulin B(1-2)-D-B(4-30)-R-R, A(1-20)-G:
   FVDQHLCGSHLVEALYLVCGERGFFYTPKTRR, SEQ ID NO.11
   GIVEQCCTSICSLYQLENYCG SEQ ID NO.2.

The molecular weight of the obtained product detected by LC-MS is 6064.8, which is consistent with the theoretically predicted molecular weight of 6063.96.
Product 2: human insulin B(1-2)-D-B(4-30)-R, R-A(1-20)-G:
   FVDQHLCGSHLVEALYLVCGERGFFYTPKTR, SEQ ID NO.12
   RGIVEQCCTSICSLYQLENYCG SEQ ID NO.3.

The molecular weight of the obtained product detected by LC-MS is 6064.8, which is consistent with the theoretically predicted molecular weight of 6063.96.

### Step 5: Analysis of disulfide bond structure in cleaved products

### Structure of disulfide bond in cleaved products was analyzed as described in Example 1.

Results obtained from analysis of disulfide bond structure in Human insulin B(1-2)-D-B(4-30)-R-R, A(1-20)-G:

| Fragment NO | Peptide sequence | LC-MS Mol. wt. | Theoretical Mol. wt. |
|---|---|---|---|
| I | GIVE | 417.2 | 416.47 |
| II | QCCTSICSLYQLE | 2970.3 | 2969.39 |
| | FVNQHLCGSHL VE | | |
| III | NYCG | 1377 | 1377.55 |
| | ALYLVCGE | | |
| IV | RGFFYTPKTRR | 1428.0 | 1428.6 |

These results confirmed that the configurations of the disulfide bonds of recombinant human insulin B(1-2)-D-B(4-30)-R-R, A(1-20)-G are as follows: one is formed between A20 and B19; the other two are formed by any two selected from A6 Cys, A7 Cys, A11 Cys and B7 Cys.

Results from analysis of Human insulin B (1-2)-D-B (4-30)-R, R-A (1-20)-G disulfide Structure:

| Fragment NO | Peptide sequence | LC-MS Mol. wt. | Theoretical Mol. wt. |
|---|---|---|---|
| I | RGIVE | 573.4 | 572.66 |
| II | QCCTSICSLYQLE | 2970.3 | 2969.39 |
| | FVNQHLCGSHL VE | | |
| III | NYCG | 1320.6 | 1320.5 |
| | ALYLVCGE | | |
| IV | RGFFYTPKTR | 1272.6 | 1272.47 |

These results confirmed that the configurations of the disulfide bonds in recombinant human insulin B(1-2)-D-B(4-30)-R, R-A(1-20)-G are as follows: one is formed between A20 and B19; the other two are formed by any two selected from A6 Cys, A7 Cys, A11 Cys and B7 Cys.

### Example 9. Preparation of recombinant human insulin B(1-2)-K-B(4-28)-E-R-R, A(1-21)

### Step 1: Cloning and expression of recombinant human insulin B(1-2)-K-B(4-28)-E-R-R, A(1-21) precursor

### 1. Construction of the expression vector comprising recombinant human insulin precursor:

B(1-2)-K-B(4-28)-E-R-R, A(1-21) were mutated via site-directed mutagenesis by using polymerase chain reaction (PCR). Two single-stranded DNA fragments were synthesized by Invitrogen and used as primers for site-directed mutagenesis, the sequences of the primers are as following:
Primer 1: 5' CTTTTACACCCCTGAAAGAAGAGGTATCGTTGAG 3' SEQ ID NO.68
Primer 2: 5' CTCAACGATACCTCTTCTTTCAGGGGTGTAAAAG 3' SEQ ID NO.69.

The recombinant vector finally obtained in Example 6 was used as template in site-directed mutagenesis procedure. KOD kit (TOYOBO, Cat KOD-201.) 25 µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmid was analyzed by Invitrogen. The DNA sequence encoding the human insulin B(1-2)-K-B(4-28)-E-R-R, A(1-21) precursor was as follows:

TTCGTCAAGCAGCACTTGTGTGGTTCCCATTTGGTTGAGGCTCTGTACTTGGT
CTGTGGAGAAAGAGGTTTCTTTTACACCCCTGAAAGAAGAGGTATCGTTGAG
CAATGTTGCACCTCTATTTGTTCCCTGTATCAGTTGGAAAACTACTGCAACTA
A SEQ ID NO.40.

### 3. Transformation of expression vector comprising recombinant human insulin B(1-2)-K-B(4-28)-E-R-R, A(1-21) precursor via electroporation:

Expression vector comprising recombinant human insulin precursor was transformed as Example 1.

### 4. Screening clones of recombinant human insulin B(1-2)-K-B(4-28)-E-R-R, A(1-21) precursor by using G418:

Recombinant human insulin precursor clones was screened as Example 1.

### 5. Identification of clones with inserted fragments of recombinant human insulin precursor by PCR:

Clones with inserted fragments of recombinant human insulin precursor were verified as Example 1. Clone 072-16 was selected for further use.

### 6. Expression and characterization of recombinant human insulin B(1-2)-K-B(4-28)-E-R-R, A(1-21) precursor:

Recombinant human insulin precursor was expressed and identified as in Example 1. The results showed that clone No. 072-16 expressed the interest protein, and was selected for following fermentation.

### Step 2: Fermentation of recombinant human insulin B(1-2)-K-B(4-28)-E-R-R, A(1-21) precursor

Clone No. 072-16 was selected for fermentation. The fermentation method was as described in Example 1.

### Step 3: Isolation and purification of fermentation product

The fermentation product was isolated and purificated as described in Example 1. The resulted expression product was as follows:
FVKQHLCGSHLVEALYLVCGERGFFYTPERRGIVEQCCTSICSLYQLENYCN SEQ ID NO.28.

The molecular weight of the obtained product detected by LC-MS is 6015.1, which is consistent with the theoretically predicted molecular weight of 6015.93.

### Step 4: Enzymatic digestion and purification of fermentation product

Methods of Enzymatic digestion and purification are as described in Example 3. After optimization of the enzymatic reaction system, digested products prepared by reverse-phase were as follows:
Human insulin B(1-2)-K-B(4-28)-E-R-R, A(1-21):
   FVKQHLCGSHLVEALYLVCGERGFFYTPERR, SEQ ID NO. 13
   GIVEQCCTSICSLYQLENYCN SEQ ID NO. 1.

The molecular weight of the obtained product detected by LC-MS is 6064.8, which is consistent with the theoretically predicted molecular weight of 6063.96.

### Step 5: Analysis of disulfide bond structure in cleaved products

Structure of disulfide bond in cleaved products was analyzed as described in Example 1.

Results obtained from analysis of disulfide bond structure in Human insulin B(1-2)-K-B(4-28)-E-R-R, A(1-21) were as follows:

| Fragment NO | Peptide sequence | LC-MS Mol. wt. | Theoretical Mol. wt. |
|---|---|---|---|
| I | GIVE | 417.2 | 416.47 |
| II | QCCTSICSLYQLE | 2983.41 | 2983.46 |
| | FVKQHLCGSHLVE | | |
| III | NYCN | 1378.3 | 1377.55 |
| | ALYLVCGE | | |
| IV | RGFFYTPE | 1016.1 | 1016.6 |

These results confirmed that the configurations of the disulfide bonds of recombinant human insulin B(1-2)-K-B(4-28)-E-R-R, A(1-21) are as follows: one is formed between A20 and B19; the other two are formed by any two selected from A6 Cys, A7 Cys, A11 Cys and B7 Cys.

### Example 10. Preparation of recombinant human insulin B(1-2)-D-B(4-29), A(1-21) and B(1-2)-D-B(4-29), R-A(1-21)

### Step 1: Cloning and expression of recombinant human insulin B(1-2)-D-B(4-29), A(1-21) and B(1-2)-D-B(4-29), R-A(1-21) precursors

### 1. Construction of the expression vector comprising recombinant human insulin precursors:

B(1-2)-D-B(4-29), A(1-21) and B(1-2)-D-B(4-29), R-A(1-21) were mutated via site-directed mutation by using polymerase chain reaction (PCR). Two single-stranded DNA fragments were synthesized by Invitrogen and used as primers for site-directed mutagenesis, the sequences of the primers are as following:
Primer 1: 5' CTTTTACACCCCTAAGAGAGGTATCGTTGAGCAATG 3' SEQ ID NO.70
Primer 2: 5' CATTGCTCAACGATACCTCTCTTAGGGGTGTAAAAG 3' SEQ ID NO.71.

KOD kit (TOYOBO, Cat KOD-201.) 25µL system was applied in site-directed mutagenesis procedure: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmids were analyzed by Invitrogen. Sequences of the obtained DNA encoding the human insulin B(1-2)-D-B(4-29), A(1-21) and B(1-2)-D-B(4-29), R-A(1-21) precursors were as follows:

TTCGTCGATCAGCACTTGTGTGGTTCCCATTTGGTTGAGGCTCTGTACTTGGT
CTGTGGAGAAAGAGGTTTCTTTTACACCCCTAAGAGAGGTATCGTTGAGCAA
TGTTGCACCTCTATTTGTTCCCTGTATCAGTTGGAAAACTACTGCAACTAA
SEQ ID NO.41.

### 3. Transformation of expression vector comprising recombinant human insulin B(1-2)-D-B(4-29), A(1-21) and B(1-2)-D-B(4-29), R-A(1-21) precursor via electroporation:

Expression vector comprising recombinant human insulin precursor was transformed as Example 1.

### 4. Screening clones of recombinant human insulin B(1-2)-D-B(4-29), A(1-21) and B(1-2)-D-B(4-29), R-A(1-21) precursor by using G418:

Recombinant human insulin precursor clone was screened as Example 1.

### 5. Identification of clones with inserted fragments of recombinant human insulin precursor by PCR:

Inserted fragments of recombinant human insulin precursor clone were verified as Example 1. Clone 087-1 was selected for further use.

### 6. Expression and characterization of recombinant human insulin B(1-2)-D-B(4-29), A(1-21) and B(1-2)-D-B(4-29), R-A(1-21) precursor:

Recombinant human insulin precursor was expressed and identified as in Example 1. The results showed that clone No. 087-1 expressed the interest protein, and was selected for following fermentation.

### Step 2: Fermentation of recombinant human insulin B(1-2)-D-B(4-29), A(1-21) and B(1-2)-D-B(4-29), R-A(1-21) precursor

Clone No. 087-1 was selected for fermentation. The fermentation method was as described in Example 1.

### Step 3: Isolation and purification of fermentation product

The fermentation product was isolated and purificated as described in Example 1. The resulted expression product was as follows:
FVDQHLCGSHLVEALYLVCGERGFFYTPKRGIVEQCCTSICSLYQLENYCN SEQ ID NO.29.

The molecular weight of the obtained product detected by LC-MS is 5846.2, which is consistent with the theoretically predicted molecular weight of 5845.74.

### Step 4: Enzymatic digestion and purification of fermentation product

Methods of Enzymatic digestion and purification are as described in Example 3. After optimization of the enzymatic reaction system, digested products prepared by reverse-phase were as follows:
Human insulin B(1-2)-D-B(4-29), A(1-21):
   FVDQHLCGSHLVEALYLVCGERGFFYTPK, SEQ ID NO.14
   GIVEQCCTSICSLYQLENYCN SEQ ID NO. 1.

The molecular weight of the obtained product detected by LC-MS is 5707.53, which is consistent with the theoretically predicted molecular weight of 5708.

The product from Step 3 was digested with Lys-C (Sigma, P3428). Digested product 2 obtained by reverse-phase was as follows:
Human insulin B (1-2)-D-B (4-29), R-A (1-21):
   FVDQHLCGSHLVEALYLVCGERGFFYTPK, SEQ ID NO.14
   RGIVEQCCTSICSLYQLENYCN SEQ ID NO.4.

The molecular weight of the obtained product detected by LC-MS is 5864.5, which is consistent with the theoretically predicted molecular weight of 5863.72.

### Step 5: Analysis of disulfide bond structure in cleaved products

Structure of disulfide bond in cleaved products was analyzed as described in Example 1.

Results obtained from analysis of disulfide bond structure in Human insulin B(1-2)-D-B(4-29), A(1-21) were as follows:

| Fragment NO | Peptide sequence | LC-MS Mol. wt. | Theoretical Mol. wt. |
|---|---|---|---|
| I | GIVE | 417.2 | 416.47 |
| II | QCCTSICSLYQLE | 2970.8 | 2970.37 |
| | FVDQHLCGSHLVE | | |
| III | NYCN | 1377.8 | 1377.55 |
| | ALYLVCGE | | |
| IV | RGFFYTPK | 1015.6 | 1015.18 |

These results confirmed that the configurations of the disulfide bonds of recombinant human insulin B(1-2)-D-B(4-29), A(1-21) are as follows: one is formed between A20 and B19; the other two are formed by any two selected from A6 Cys, A7 Cys, A11 Cys and B7 Cys.

Results from analysis of Human insulin B (1-2)-D-B (4-29), R-A (1-21) disulfide bond structure:

| Fragment NO | Peptide sequence | LC-MS Mol. wt. | Theoretical Mol. wt. |
|---|---|---|---|
| I | RGIVE | 573.3 | 572.66 |
| II | QCCTSICSLYQLE | 2970.3 | 2970.37 |
| | FVDQHLCGSHLVE | | |
| III | NYCN | 1377.8 | 1377.55 |
| | ALYLVCGE | | |
| IV | RGFFYTPK | 1015.4 | 1015.18 |

These results confirmed that the configurations of the disulfide bonds of recombinant human insulin B(1-2)-D-B(4-29), R-A(1-21) are as follows: one is formed between A20 and B19; the other two are formed by any two selected from A6 Cys, A7 Cys, A11 Cys and B7 Cys.

### Example 11. Preparation of recombinant human insulin B(1-27)-K-E, A(1-20)-G

### Step 1: Cloning and expression of recombinant human insulin B(1-27)-K-E, A(1-20)-G precursor

### 1. Construction of the expression vector comprising recombinant human insulin precursor:

B(1-27)-K-E, A(1-20)-G was mutated via site-directed mutation by using polymerase chain reaction (PCR). Two single-stranded DNA fragments were synthesized by Invitrogen and used as primers for site-directed mutagenesis, the sequences of the primers are as following:
Primer 1: 5' GTTGGAAAACTACTGCGGTTAAGAATTCCCTAGG 3' SEQ ID NO.72
Primer 2: 5' CCTAGGGAATTCTTAACCGCAGTAGTTTTCCAAC 3' SEQ ID NO.73.

The recombinant vector finally obtained in Example 4 was used as template in site-directed mutagenesis procedure. KOD kit (TOYOBO, Cat KOD-201.) 25 µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmid was analyzed by Invitrogen. Sequence of the obtained DNA encoding the human insulin B(1-27)-K-E, A(1-20)-G precursor was as follows:

TTCGTCAACCAGCACTTGTGTGGTTCCCATTTGGTTGAGGCTCTGTACTTGG
TCTGTGGAGAAAGAGGTTTCTTTTACACCAAGGAAAAAAGAGGTATCGTTGA
GCAATGTTGCACCTCTATTTGTTCCCTGTATCAGTTGGAAAACTACTGCGGTT
AAGAATTCCCTAG SEQ ID NO.42.

### 3. Transformation for expression vector of recombinant human insulin B(1-27)-K-E, A(1-20)-G precursor via electroporation:

Expression vector of recombinant human insulin B(1-27)-K-E, A(1-20)-G precursor was transformed as Example 1.

### 4. Screening clones of recombinant human insulin B(1-27)-K-E, A(1-20)-G precursor by using G418:

Recombinant human insulin precursor clone was screened as Example 1.

### 5. Identification of clones with inserted fragments of recombinant human insulin B(1-27)-K-E, A(1-20)-G precursor by PCR:

Clones inserted with fragments of recombinant human insulin precursor B(1-27)-K-E, A(1-20)-G were verified as Example 1. Clone 094-4 was selected for further use.

### 6. Expression and characterization of recombinant human insulin B(1-27)-K-E, A(1-20)-G precursor:

Recombinant human insulin precursor was expressed and identified as in Example 1. The results showed that clone No. 094-4 expressed the interest protein, and was selected for following fermentation.

### Step 2: Fermentation of recombinant human insulin precursor

No. 094-4 clone was selected for fermentation. The fermentation method was as described in Example 1.

### Step 3: Isolation and purification of fermentation product

The fermentation product was isolated and purificated as described in Example 1. The resulted B(1-27)-K-E, A(1-20)-G precursor was as follows:
FVNQHLCGSHLVEALYLVCGERGFFYTKEKRGIVEQCCTSICSLYQLENYCG SEQ ID NO.30.

The molecular weight of the obtained product detected by LC-MS is 5948, which is consistent with the theoretically predicted molecular weight of 5947.85.

### Step 4: Enzymatic digestion and purification of fermentation product

Methods of Enzymatic digestion and purification are as described in Example 1. After optimization of the enzymatic reaction system, digested products prepared by reverse-phase were as follows:
Human insulin B(1-27)-K-E, A(1-20)-G:
   FVNQHLCGSHLVEALYLVCGERGFFYTKE, SEQ ID NO.15
   GIVEQCCTSICSLYQLENYCG SEQ ID NO.2.

The molecular weight of the obtained product detected by LC-MS is 5681, which is consistent with the theoretically predicted molecular weight of 5681.49.

### Step 5: Analysis of disulfide bond structure in cleaved products

Structure of disulfide bond in cleaved products was analyzed as described in Example 1.

Results obtained from analysis of disulfide bond structure in Human insulin B(1-27)-K-E, A(1-20)-G were as follows:

| Fragment NO | Peptide sequence | LC-MS Mol. wt. | Theoretical Mol. wt. |
|---|---|---|---|
| I | GIVE | 416 | 416.47 |
| II | QCCTSICSLYQLE | 2969 | 2969.39 |
| | FVNQHLCGSHLVE | | |
| III | NYCG | 1320 | 1320.5 |
| | ALYLVCGE | | |
| IV | RGFFYTDKE | 1047 | 1047.18 |

These results confirmed that the configurations of the disulfide bonds of recombinant human insulin B(1-27)-K-E, A(1-20)-G are as follows: one is formed between A20 and B19; the other two are formed by any two selected from A6 Cys, A7 Cys, A11 Cys and B7 Cys.

### Example 12. Preparation of recombinant human insulin B(1-2)-K-B(4-28)-E-E, A(1-20)-G

### Step 1: Cloning and expression of recombinant human insulin B(1-2)-K-B(4-28)-E-E, A(1-20)-G precursor

### 1. Construction of the expression vector comprising recombinant human insulin B(1-2)-K-B(4-28)-E-E, A(1-20)-G precursor:

B(1-2)-K-B(4-28)-E-E, A(1-20)-G precursor was mutated via site-directed mutagenesis by using polymerase chain reaction (PCR). Two single-stranded DNA fragments were synthesized by Invitrogen and used as primers for site-directed mutagenesis, the sequences of the primers are as following:
Primer 1: 5' GTTGGAAAACTACTGCGGTTAAGAATTCCCTAGG 3' SEQ ID NO.74
Primer 2: 5' CCTAGGGAATTCTTAACCGCAGTAGTTTTCCAAC 3' SEQ ID NO.75.

The recombinant vector finally obtained in Example 6 was used as template in site-directed mutagenesis procedure. KOD kit (TOYOBO, Cat KOD-201) 25µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmid was analyzed by Invitrogen. Sequence of the obtained DNA encoding the human insulin B(1-2)-K-B(4-28)-E-E, A(1-20)-G precursor was as follows:

TTCGTCAAGCAGCACTTGTGTGGTTCCCATTTGGTTGAGGCTCTGTACTTG
GTCTGTGGAGAAAGAGGTTTCTTTACACCCCTGAAGAAAAAGAGGTATCG
TTGAGCAATGTTGCACCTCTATTTGTTCCCTGTATCAGTTGGAAAACTACTGC
GGTTAAGAATTCCCTAGG
SEQ ID NO.43.

### 3. Transformation of expression vector comprising recombinant human insulin B(1-2)-K-B(4-28)-E-E, A(1-20)-G precursor via electroporation:

Expression vector comprising recombinant human insulin B(1-2)-K-B(4-28)-E-E, A(1-20)-G precursor was transformed as Example 1.

### 4. Screening clones of recombinant human insulin B(1-2)-K-B(4-28)-E-E, A(1-20)-G precursor by using G418:

Clones with recombinant human insulin B(1-2)-K-B(4-28)-E-E, A(1-20)-G precursor were screened as Example 1.

### 5. Identification of clones with inserted fragments of recombinant human insulin B(1-2)-K-B(4-28)-E-E, A(1-20)-G precursor by PCR:

Clones inserted with fragments of recombinant human insulin precursor were verified as Example 1. Clone 093-15 was selected for further use.

### 6. Expression and characterization of recombinant human insulin B(1-2)-K-B(4-28)-E-E, A(1-20)-G precursor:

Recombinant human insulin B(1-2)-K-B(4-28)-E-E, A(1-20)-G precursor was expressed and identified as in Example 1. The results showed that clone No. 093-15 expressed the interest protein, and was selected for following fermentation.

### Step 2: Fermentation of recombinant human insulin precursor

Clone No. 093-15 was selected for fermentation. The fermentation method was as described in Example 1.

### Step 3: Isolation and purification of fermentation product

The fermentation product was isolated and purificated as described in Example 1. The resulted expression product was as follows:
FVKQHLCGSHLVEALYLVCGERGFFYTPEEKRGIVEQCCTSICSLYQLENYC G SEQ ID NO. 20.

The molecular weight of the obtained product detected by LC-MS is 6060, which is consistent with the theoretically predicted molecular weight of 6060.

### Step 4: Enzymatic digestion and purification of fermentation product

Methods of Enzymatic digestion and purification are as described in Example 1. After optimization of the enzymatic reaction system, digested products prepared by reverse-phase were as follows:
Human insulin B(1-2)-K-B(4-28)-E-E, A(1-20)-G:
   FVKQHLCGSHLVEALYLVCGERGFFYTPEE, SEQ ID NO.16
   GIVEQCCTSICSLYQLENYCG SEQ ID NO.2.

The molecular weight of the obtained product detected by LC-MS is 5793, which is consistent with the theoretically predicted molecular weight of 5793.6.

### Step 5: Analysis of disulfide bond structure in cleaved products

Structure of disulfide bond in cleaved products was analyzed as described in Example 1.

Results obtained from analysis of disulfide bond structure in Human insulin B(1-2)-K-B(4-28)-E-E, A(1-20)-G were as follows:

| Fragment NO | Peptide sequence | LC-MS Mol. wt. | Theoretical Mol. wt. |
|---|---|---|---|
| I | GIVE | 416 | 416.47 |
| II | QCCTSICSLYQLE | 2982.6 | 2983.46 |
| | FVKQHLCGSHLVE | | |
| III | NYCG | 1319.9 | 1320.5 |
| | ALYLVCGE | | |
| IV | RGFFYTPEK | 1144.8 | 1345.24 |

These results confirmed that the configurations of the disulfide bonds of recombinant human insulin B(1-2)-K-B(4-28)-E-E, A(1-20)-G are as follows: one is formed between A20 and B19; the other two are formed by any two selected from A6 Cys, A7 Cys, A11 Cys and B7 Cys.

### Example 13. Preparation of recombinant human insulin B(1-2)-D-B(4-29)-R, A(1-20)-G, B(1-2)-D-B(4-29), A(1-20)-G and B(1-2)-D-B(4-29), R-A(1-20)-G

### Step 1: Cloning and expression of recombinant human insulin B(1-2)-D-B(4-29)-R, A(1-20)-G, B(1-2)-D-B(4-29), A(1-20)-G and B(1-2)-D-B(4-29), R-A(1-20)-G precursors

### 1. Construction of the expression vector comprising recombinant human insulin precursors:

B(1-2)-D-B(4-29)-R, A(1-20)-G, B(1-2)-D-B(4-29), A(1-20)-G and B(1-2)-D-B(4-29), R-A(1-20)-G were mutated via site-directed mutagenesis by using polymerase chain reaction (PCR). Two single-stranded DNA fragments were synthesized by Invitrogen and used as primers for site-directed mutagenesis, the sequences of the primers are as following:
Primer 1: 5' GTTGGAAAACTACTGCGGTTAAGAATTCCCTAGG 3' SEQ ID NO.76
Primer 2: 5' CCTAGGGAATTCTTAACCGCAGTAGTTTTCCAAC 3' SEQ ID NO.67.

The recombinant vector finally obtained in Example 10 was used as template in site-directed mutagenesis procedure. KOD kit (TOYOBO, Cat KOD-201.) 25 µL system was applied: 2.5µL 10 × KOD buffer, 2.5µL 2mM dNTPs, 1µL primer 1 (10µM), 1µL primer 2 (10µM), 0.5µL KOD plus, 1µL 25mM MgSO₄, 16µL ddH₂O. The amplification program was as follows: 94°C 2min, 94°C 30sec, 55°C 30sec, 68°C 11min, for 25 amplification cycles, followed by incubation for 11 min at 68°C. PCR product was digested for 1hr by adding 1µL of DpnI (NEB, Cat. R0176L) directly, then was transformed into TOP10 competent cells to obtain interest plasmid. The resulted recombinant expression vector was delivered to Invitrogen for sequence analysis.

### 2. Result of sequence analysis.

The linked recombinant plasmids were analyzed by Invitrogen. Sequences of the obtained DNA encoding the human insulin B(1-2)-D-B(4-29)-R, A(1-20)-G, B(1-2)-D-B(4-29), A(1-20)-G and B(1-2)-D-B(4-29), R-A(1-20)-G precursors were as follows:

TTCGTCGATCAGCACTTGTGTGGTTCCCATTTGGTTGAGGCTCTGTACTTGG
TCTGTGGAGAAAGAGGTTTCTTTTACACCCCTAAGAGAGGTATCGTTGAGCA
ATGTTGCACCTCTATTTGTTCCCTGTATCAGTTGGAAAACTACTGCGGTTAAG
AATTCCCTAGG SEQ ID NO.44.

### 3. Transformation for expression vector of recombinant human insulin B(1-2)-D-B(4-29)-R, A(1-20)-G, B(1-2)-D-B(4-29), A(1-20)-G and B(1-2)-D-B(4-29), R-A(1-20)-G precursors via electroporation:

Expression vector of recombinant human insulin precursor was transformed as Example 1.

### 4. Screening clones of recombinant human insulin B(1-2)-D-B(4-29)-R, A(1-20)-G, B(1-2)-D-B(4-29), A(1-20)-G and B(1-2)-D-B(4-29), R-A(1-20)-G precursors by using G418:

Clone with recombinant human insulin precursor was screened as Example 1.

### 5. Identification of clones with inserted fragments of recombinant human insulin B(1-2)-D-B(4-29)-R, A(1-20)-G, B(1-2)-D-B(4-29), A(1-20)-G or B(1-2)-D-B(4-29), R-A(1-20)-G precursors by PCR:

Clones with inserted fragments of recombinant human insulin precursor were verified as Example 1. Clone 096-4 was selected for further use.

### 6. Expression and characterization of recombinant human insulin B(1-2)-D-B(4-29)-R, A(1-20)-G, B(1-2)-D-B(4-29), A(1-20)-G and B(1-2)-D-B(4-29), R-A(1-20)-G precursor:

Recombinant human insulin precursor was expressed and identified as in Example 1. The results showed that clone No. 096-4 expressed the interest protein, and was selected for following fermentation.

### Step 2: Fermentation of recombinant human insulin B(1-2)-D-B(4-29)-R, A(1-20)-G, B(1-2)-D-B(4-29), A(1-20)-G and B(1-2)-D-B(4-29), R-A(1-20)-G precursor

Clone No. 096-4 was selected for fermentation. The fermentation method was as described in Example 1.

### Step 3: Isolation and purification of fermentation product

The fermentation product was isolated and purificated as described in Example 1. The resulted expression product precursor was as follows:
FVDQHLCGSHLVEALYLVCGERGFFYTP KRGIVEQCCTSICSLYQLENYCG SEQ ID NO.31.

The molecular weight of the obtained product detected by LC-MS is 5789, which is consistent with the theoretically predicted molecular weight of 5788.67.

### Step 4: Enzymatic digestion and purification of fermentation product

Methods of Enzymatic digestion and purification are as described in Example 3. After optimization of the enzymatic reaction system, digested products prepared by reverse-phase were as follows:
Human insulin B(1-2)-D-B(4-29)-R, A(1-20)-G:
   FVDQHLCGSHLVEALYLVCGERGFFYTPKR, SEQ ID NO.17
   GIVEQCCTSICSLYQLENYCG SEQ ID NO.2.

The molecular weight of the obtained product detected by LC-MS is 5807, which is consistent with the theoretically predicted molecular weight of 5806.67.

Enzymatic digestion and purification methods as in Example 1. After optimization of enzymatic reaction system, the digested product 2 obtained by reverse-phase preparative were as follows:
Human insulin B(1-2)-D-B(4-29), A(1-20)-G:
   FVDQHLCGSHLVEALYLVCGERGFFYTPK, SEQ ID NO.18
   GIVEQCCTSICSLYQLENYCG SEQ ID NO.2.

The molecular weight of the obtained product detected by LC-MS is 5651, which is consistent with the theoretically predicted molecular weight of 5650.48.

The product from Step 3 (096-4 fermentation) was digested by using the same enzymatic method as described in Example 10. The Digested product prepared by inverse-phase was as follows:
Human insulin B(1-2)-D-B(4-29), R-A(1-20)-G:
   FVDQHLCGSHLVEALYLVCGERGFFYTPK, SEQ ID NO.18
   RGIVEQCCTSICSLYQLENYCG SEQ ID NO.3.

The molecular weight of the obtained product detected by LC-MS is 5807, which is consistent with the theoretically predicted molecular weight of 5806.7.

### Step 5: Analysis of disulfide bond structure in cleaved products

Structure of disulfide bond in cleaved products was analyzed as described in Example 1.

Results obtained from analysis of disulfide bond structure in Human insulin B(1-2)-D-B(4-29)-R, A(1-20)-G were as follows:

| Fragment NO | Peptide sequence | LC-MS Mol. wt. | Theoretical Mol. wt. |
|---|---|---|---|
| I | GIVE | 416 | 416.47 |
| II | QCCTSICSLYQLE | 2970 | 2970.3 |
| | FVDQHLCGSHLVE | | |
| III | NYCG | 1320 | 1320.5 |
| | ALYLVCGE | | |
| IV | RGFFYTPKR | 1171 | 1171.37 |

These results confirmed that the configurations of the disulfide bonds of recombinant human insulin B(1-2)-D-B(4-29)-R, A(1-20)-G are as follows: one is formed between A20 and B19; the other two are formed by any two selected from A6 Cys, A7 Cys, A11 Cys and B7 Cys.

Results from Analysis of Human insulin B (1-2)-D-B (4-29), A (1-20)-G disulfide Structure:

| Fragment NO | Peptide sequence | LC-MS Mol. wt. | Theoretical Mol. wt. |
|---|---|---|---|
| I | GIVE | 416 | 416.47 |
| II | QCCTSICSLYQLE | 2970 | 2970.3 |
| | FVDQHLCGSHLVE | | |
| III | NYCG | 1320 | 1320.5 |
| | ALYLVCGE | | |
| IV | RGFFYTPK | 1015 | 1015.18 |

These results confirmed that the configurations of the disulfide bonds of recombinant human insulin B(1-2)-D-B(4-29)-R, A(1-20)-G are as follows: one is formed between A20 and B19; the other two are formed by any two selected from A6 Cys, A7 Cys, A11 Cys and B7 Cys.

Results from Analysis of Human insulin B (1-2)-D-B(4-29), R-A (1-20)-G disulfide Structure :

| Fragment NO | Peptide sequence | LC-MS Mol. wt. | Theoretical Mol. wt. |
|---|---|---|---|
| I | RGIVE | 573 | 572.66 |
| II | QCCTSICSLYQLE | 2970 | 2970.3 |
| | FVDQHLCGSHLVE | | |
| III | NYCG | 1320 | 1320.5 |
| | ALYLVCGE | | |
| IV | RGFFYTPK | 1015 | 1015.18 |

These results confirmed that the configurations of the disulfide bonds of recombinant human insulin B(1-2)-D-B(4-29)-R, A(1-20)-G are as follows: one is formed between A20 and B19; the other two are formed by any two selected from A6 Cys, A7 Cys, A11 Cys and B7 Cys.

### Example 14. Preparation of B28D-N^{εB29}-(N^{α}-(HOOC(CH₂)₁₄CO)-γ-Glu)-B30E human insulin (HS061)

### 1. Preparation of methyl hexadecandioyl-Glu (OSu)-OMe

Hexadecanedioic acid (5.0g, 17.48mmol) was dissolved in anhydrous methanol (50mL), cooled with liquid nitrogen to -10°C, then thionyl chloride was dropwise added (3.2mL, 43.7mmol), at -10°C for 20min, and reaction solution was slowly warmed to reflux for 4hrs. Reaction was terminated, and reaction solution was cooled to room temperature and concentrated to dryness, 5.15g (94%) of hexadecanedioic acid dimethyl.

Hexadecanedioic acid dimethyl (5.0g, 15.92mmol) was dissolved in anhydrous methanol (50mL). Ba (OH)₂ • 8H₂O (5.0g, 15.8mmol) dissolved in methanol was dropwise added into Hexadecanedioic acid dimethyl, and reaction solution was slowly warmed to 30°C and the mixture was stirred for 24hr. After methanol was removed via vacuum concentration, the residue was dissolved in ethyl acetate and washed three times with 0.1M HCl, the organic phase was collected and dried over anhydrous sodium sulfate, filtered, and concentrated under vacuum and dried in vacuum, 4.55g (yield 95%) hexadecanedioic acid monomethyl was obtained.

Hexadecanedioic acid monomethyl (2.0g, 6.67mmol) was dissolved in DCM (50mL), followed by adding HOSu (0.92g, 8.0mmol), then DCC (2.06g, 10mmol) was added, stirred for 24hr at room temperature. Reaction solution was filtered, the organic phase was washed three times with 200mL 0.1M HCl and then dried over anhydrous sodium sulfate, filtered and concentrated under vacuum, 2.45g (92%) methyl cetane diacid succinimide ester was obtain.

H-Glu-OMe (0.81g, 5.04mmol) was dissolved in 5mL of purified water, the obtained solution was added to THF (50mL) solution dissolved with Methyl cetane diacid succinimide ester (1.0g, 2.52mmol). DIEA (0.65g, 5.04mmol) was added to the obtained mixture and stirred at room temperature for 24hrs. The reaction solution was filtered and concentrated under vacuum. The residue was dissolved in ethyl acetate, and the organic phase was washed three times with 200mL 0.1M HCl, then the organic phase was collected and dried over anhydrous sodium sulfate, filtered, concentrated under vacuum and purified by reverse phase high performance liquid chromatography, 0.7g (63%) methyl hexadecandioyl-Glu-OMe, ESI-MS: 444.3 ([M + H] +) was obtained after concentration.

Methyl hexadecandioyl-Glu-OMe (0.3g, 0.677mmol) was dissolved in DCM (20mL), then HOSu (85.7mg, 0.745mmol) and DCC (0.167g, 0.812mmol) was added, stirred at room temperature for 24hrs, filtered, washed with 0.1M HCl and the organic phase was collected, dried over anhydrous sodium sulfate, filtered and concentrated under vacuum, dried in vacuum to obtain 0.3g (82%) methyl hexadecandioyl-Glu (OSu)-OMe, which has structural formula as follows:

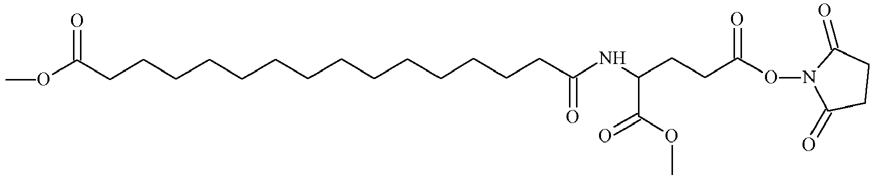

### 2. Preparation of B28D-N^{εB29}-(N^{α}-(HOOC(CH₂)₁₄CO)-γ-Glu)-B30E human insulin precursor

Recombinant human insulin B(1-27)-D-K-E, A(1-21) (50mg, 8.5mmol) (Example 7) was dissolved in 10mL of 10mM HCl. pH value of the solution was adjusted to about 10.5 by 0.2M NaOH. Methyl hexadecandioyl-Glu (OSu)-OMe (13.9mg, 25.6mmol) was dissolved in 10mL of acetonitrile and then added to the above B(1-27)-D-K-E, A(1-21) human insulin solution to start reaction for 40min. The reaction process was controlled by RP-HPLC. 40 min later, pH value of the solurion was adjusted to about 2.2 with 10% HCl, reaction wad terminated, and crude precursor solution was obtained.

### 3. Purification of B28D-N^{εB29}-(N^{α}-(HOOC(CH₂)₁₄CO)-γ-Glu)-B30E human insulin precursor

The above crude precursor solution was diluted with water to make the content of organic phase about 30% (v: v), after being filtered through a 0.45µm membrane, the solution was purified with RP-HPLC, wherein the reverse phase column was Luna C18, 250 × 25mm, 5µm, 100Å, mobile phase A was 0.1% TFA/H₂O, mobile phase B was 0.1% TFA/ACN. The desired product was eluted and collected with a gradient phase B from 38% to 68% at a flow rate of 20mL/min within 60min, ESI-MS: 1570.8 ([M +4 H] 4 +), the purified precursor solution was obtained.

### 4. Saponification of B28D-N^{εB29}-(N^{α}-(HOOC(CH₂)₁₄CO)-γ-Glu)-B30E human insulin precursor

Acetonitrile was removed from the purified precursor solution via vacuum concentration. Then the pH value of the solution was adjusted to 8.0 with 10% NaOH solution and saponified by adding an equal volume of 0.2M ice-cooled NaOH. The saponified product was analyzed with RP-HPLC. The reaction was terminated by adding 10% HCl.

### 5. Purification and lyophilization of saponified human insulin B28D-N^{εB29}-(N^{α}-(HOOC(CH₂)₁₄CO)-γ-Glu)-B30E precursor solution

The above saponified precursor solution was diluted with acetonitrile to make the content of organic phase about 30% (v: v), after being filtered through a 0.45µm membrane, the solution was purified with RP-HPLC, wherein the reverse phase column was Luna C18, 250 × 25mm, 5µm, 100Å, mobile phase A was 0.1% TFA/H₂O, mobile phase B was 0.1% TFA/ACN. The desired product was eluted and collected with a gradient phase B from 35% to 65% at a flow rate of 20mL/min within 60min, lyophilized and 3.6mg of product (purity 97.9%) was obtained. The resulted product has structure as follows:

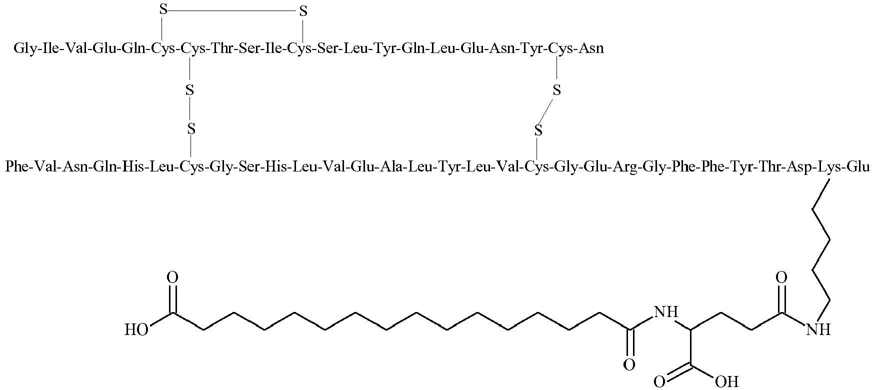

### 6. Confirmation of the structure of human insulin B28D-N^{εB29}-(N^{α}-(HOOC(CH₂)₁₄CO)-γ-Glu)-B30E

The molecular weight of human insulin B28D-N^{εB29}-(N^{α}-(HOOC(CH₂)₁₄CO)-γ-Glu)-B30E detected by ESI-MS assay is 6251.65. which is consistent with the theoretically predicted molecular weight of 6251.16.

Human insulin B28D-N^{εB29}-(N^{α}-(HOOC(CH₂)₁₄CO)-γ-Glu)-B30E was digested with trypsin. The digested products were analyzed by LC-MS. The result showed that the molecular weights of the two fragments F1 and F2 were 4865 and 1403, respectively, consistent with the theoretical molecular weight of the digested products. Fragment F2 corresponds to the modified B-chain fragment. Modified sites were proven the same as be expected.

### Example 15. Preparation of human insulin B28D-N^{εB29}-(N^{α}-(HO(CH₂)₁₅CO)-γ-Glu)-B30E (HS062)

### 1. 16-hydroxy hexadecanoyl-Glu (OSu)-OMe

16-hydroxy hexadecanoic acid (3.0g, 11.03mmol) was dissolved in DCM (50mL), then O-(N-succinimidyl)-1,1,3,3 - tetramethyluronium tetrafluoroborate ester and N, N-diisopropylethylamine (1.71g, 13.2mmol) was added. The reaction solution was stirred at room temperature for 24hrs. After filteration, the organic phase was washed three times with 0.1M HCl and dried over anhydrous sodium sulfate. The organic phase was concentrated under vacuum, 3.5g (70%) 16-hydroxy hexadecane succinimide ester was obtained.

H-Glu-OMe (3.4g, 21.64mmol) dissolved in 5mL of purified water was added to THF solution (50mL) dissolved with 16- hydroxyl hexadecane succinimide ester. DIEA (2.8g, 21.64mmol) was added and stirred at room temperature for 24hrs. The reaction solution was filtered and concentrated under vacuum. The residue was dissolved in ethyl acetate, and the organic phase was washed three times with 200mL 0.1M HCl. The organic phase was collected and dried over anhydrous sodium sulfate, filtered, concentrated under vacuum and purified by reverse phase high performance liquid chromatography, after final concentration, 2.5g(45%) 16-hydroxy hexadecanoyl-Glu-OMe, ESI-MS: 417 ([M + H] +) was obtained.

16-hydroxy hexadecanoyl-Glu-OMe (1.0g, 2.4mmol) was dissolved in DCM (20mL), then HOSu (0.305g, 2.65mmol) and EDC·HCl (0.69g, 3.61mmol) was added, stirred at room temperature for 24hrs, filtered, washed with 0.1M HCl and the organic phase was collected, dried over anhydrous sodium sulfate, filtered and concentrated under vacuum, dried in vacuum, 0.9g of 16-hydroxy hexadecanoyl-Glu (OSu)-OMe (purity was 73%) was obtained, which has the following structural formula:

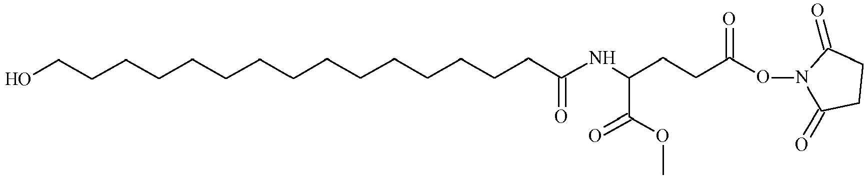

### 2. Preparation of human insulin B28D-N^{εB29}-(N^{α}-(HO(CH₂)₁₅CO)-γ-Glu)-B30E precursor

Recombinant human insulin B(1-27)-D-K-E, A(1-21) (50mg, 8.5mmol) was dissolved in 10mL of 10mM HCl. pH value of the solution was adjusted to about 10.5 with 0.2M NaOH. 16-hydroxy hexadecanoyl-Glu (OSu)-OMe (13.2mg, 25.5mmol) was dissolved in 10mL of acetonitrile and then added to the above B(1-27)-D-K-E, A(1-21) human insulin solution to start reaction for 40 min. The reaction process was controlled by RP-HPLC. 40 min later, the pH value of the solution was adjusted to about 2.2 with 10% HCl, then the reaction was terminated, and crude precursor solution was obtained.

### 3. Purification of human insulin B28D-N^{εB29}-(N^{α}-(HO(CH₂)₁₅CO)-γ-Glu)-B30E precursor

The above crude precursor solution was diluted with water to make the content of organic phase about 30% (v: v), after being filtered through a 0.45µm membrane, the solution was purified with RP-HPLC, wherein the reverse phase column was Luna C18, 250 × 25mm, 5µm, 100Å, mobile phase A was 0.1% TFA/H₂O, mobile phase B was 0.1% TFA/ACN. The desired product was eluted and collected with a gradient phase B from 35% to 65% a at a flow rate of 20mL/min within 60min, ESI-MS: 1563.4 ([M +4 H] 4 +), the purified precursor solution was obtained.

### 4. Saponification of human insulin B28D-N^{εB29}-(N^{α}-(HO(CH₂)₁₅CO)-γ-Glu)-B30E precursor

Acetonitrile was removed from the purified precursor solution via vacuum concentration. Then the pH value of the solution was adjusted to 8.0 with 10% NaOH solution and saponified for 50 min by adding an equal volume of 0.2M ice-cooled NaOH. The saponified product was analyzed with RP-HPLC. After 50 min, the reaction was terminated by adding 10% HCl.

### 5. Purification and lyophilization of human insulin B28D-N^{εB29}-(N^{α}-(HO(CH₂)₁₅CO)-γ-Glu)-B30E precursor

The above saponified precursor solution was diluted with acetonitrile to make the content of organic phase about 30% (v: v), after being filtered through a 0.45µm membrane, the solution was purified with RP-HPLC, wherein the reverse phase column was Luna C18, 250 × 25mm, 5µm, 100Å, mobile phase A was 0.1% TFA/H₂O, mobile phase B was 0.1% TFA/ ACN. The desired product was eluted and collected with a gradient phase B from 35% to 65% at a flow rate of 20mL/min within 60min, lyophilized, 10mg product was obtained (purity 94.3%). The resulted product has structural formula as follows:

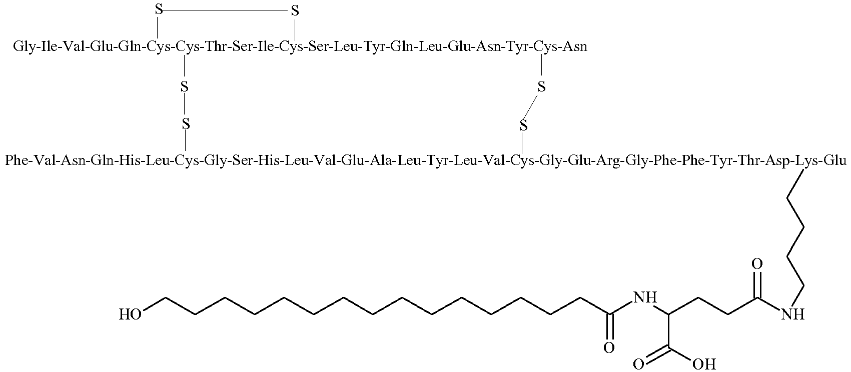

### 6. Confirmation of the structure of human insulin B28D-N^{εB29}-(N^{α}-(HO(CH₂)₁₅CO)-γ-Glu)-B30E

The molecular weight of human insulin B28D-N^{εB29}-(N^{α}-(HO(CH₂)₁₅CO)-γ-Glu)-B30E detected by ESI-MS is 6237.52, which is consistent with the theoretically predicted molecular weight of 6237.17.

Human insulin B28D-N^{εB29}-(N^{α}-(HOC(CH₂)₁₅CO)-γ-Glu)-B30E was digested with trypsin. The digested products were analyzed by LC-MS. The result showed the molecular weights of the two fragments F1 and F2 were 4865 and 1389, respectively, consistent with the theoretical molecular weight of the digested products. Fragment F2 corresponds to modified B-chain fragment. Modified sites were proven as the same as being expected.

### Example 16. Preparation of N^{α}-(HOOC(CH₂)₁₄CO)-N^{ε}-(OCCH₂CH₂CO-(B28D-N^{εB29}-B30E human insulin))-Lys-OH (HS067)

### 1. Preparation of N^{α}-(methyl hexadecandioyl)-Nε-(3-acyl propionic acid-OSu) lysine methyl ester

H-Lys(Fmoc)-OMe (4.33g, 11.29mmol) dissolved in 5mL of purified water was added to THF (50mL) solution dissolved with methyl-hexadecanoic acid succinimidyl ester (3.0g, 7.55mmol), DIEA (1.46g, 11.29mmol) was added and stirred at room temperature for 24hrs. The reaction solution was filtered and concentrated under vacuum. The residue was dissolved in ethyl acetate, and the organic phase was washed three times with 200mL 0.1M HCl, then the organic phase was collected and dried over anhydrous sodium sulfate, after filtration and vacuum concentration, the product was crystallized by using 200mL of acetonitrile : water = 10: 1 (v: v), 5.0g (99.6%) methyl hexadecandioyl-Lys (Fmoc)-OMe was obtained.

Methyl hexadecandioyl-Lys (Fmoc)-OMe (5.0g, 7.52mmol) was dissolved in THF (80mL), and then hexahydro-pyridine (20mL) was added, stirred at room temperature for 20min. The solution was concentrated under vacuum. The resulted solid was dissolved in ethyl acetate (100mL), washed with water for three times, and the organic phase was dried over anhydrous sodium sulfate for 30min. After filtration and concentration under vacuum, the resulted solid was dissolved in chloroform (100mL), into which succinic anhydride (11.34g, 113.4mmol) and N, N-diisopropylethylamine (14.1g, 113.4mmol) were added, and the reaction mixture was stirred at room temperature for 2hrs. 100°C purified water (100mL) was added into the reaction solution three times, for washing. The organic phase was dried over anhydrous sodium sulfate for 30min. After filtration and vacuum concentration, the obtained solid was purified by reverse phase HPLC, after concentration, 3.45g (84.7%) N^{α}-(methyl hexadecandioyl)- N^{ε}- (3-acyl propionic acid) Lys methyl ester, ESI-MS: 543.6 ([M + H] +) was obtained.

N^{α}-(methyl hexadecandioyl)-N^{ε}-(3-acyl propionic acid) lysine methyl ester (0.5g, 0.9mmol) was dissolved in DCM (50mL), HOSu (0.116g, 2.0mmol) and EDC • HCl (0.265g, 2.0mmol) was added, then stirred at room temperature for 48hrs, filtered, then washed with 0.1M HCl, the organic phase was collected. The organic phase was dried over anhydrous sodium sulfate for 30min, after filtration and vacuum concentration, 0.36g (63%) N^{α}-(methyl hexadecandioyl)-N^{ε}-(3-acyl propionic acid-OSu)lysine methyl ester, which has structural formula as shown below.

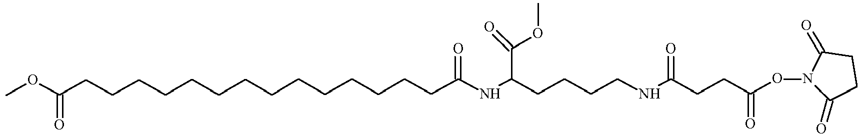

### 2. Preparation of N^{α}-(HOOC(CH₂)₁₄CO)-N^{ε}-(OCCH₂CH₂CO-(B28D-N^{εB29}-B30E human insulin))-Lys-OH precursor

Recombinant human insulin B(1-27)-D-K-E, A(1-21) (50mg, 8.5mmol) was dissolved in 10mL of 10mM HCl, then pH value was adjusted to about 10.5 with 0.2M NaOH. N^{α}-(methyl hexadecandioyl)-N^{ε}-(3-acyl propionic acid-OSu) lysine methyl ester (16.4mg, 25.6mmol) dissolved in 10mL of acetonitrile was added into the above B(1-27)-D-K-E, A(1-21) human insulin solution to start the reaction. The reaction process was controlled by RP-HPLC. pH value of the solution was adjusted to about 2.2 with 10% HCl, after 40 min, the reaction was terminated and crude precursor solution was obtained.

### 3. Purification of N^{α}-(HOOC(CH₂)₁₄CO)-N^{ε}-(OCCH₂CH₂CO-(B28D-N^{εB29}-B30E human insulin))-Lys-OH precursor

The above crude precursor solution was diluted with water to make the content of organic phase about 30% (v: v), after being filtered through a 0.45µm membrane, the solution was purified with RP-HPLC, wherein the reverse phase column was Luna C18, 250 × 25mm, 5µm, 100Å, mobile phase A was 0.1% TFA/H₂O, mobile phase B was 0.1% TFA/ACN. The desired product was eluted and collected with a gradient phase B from 38% to 68% at a flow rate of 20mL/min within 60min, ESI-MS: 1595 ([M +4 H] 4 +), a purified precursor solution was obtained.

### 4. Saponification of N^{α}-(HOOC(CH₂)₁₄CO)-N^{ε}-(OCCH₂CH₂CO-(B28D-N^{εB29}-B30E human insulin))-Lys-OH precursor

Acetonitrile was removed from the purified precursor solution via vacuum concentration. Then pH value of the solution was adjusted to 8.0 with 10% NaOH solution and an equal volume of 0.2M ice-cooled NaOH was added to start saponification. The saponified product was analyzed with RP-HPLC. After 50 min, the reaction was terminated by adding 10% HCl.

### 5. Purification and lyophilization of saponified N^{α}-(HOOC(CH₂)₁₄CO)-N^{ε}-(OCCH₂CH₂CO-(B28D-N^{εB29}-B30E human insulin))-Lys-OH precursor solution

The above saponified precursor solution was diluted with acetonitrile to make the content of organic phase about 30% (v: v), after being filtered through a 0.45µm membrane, the solution was purified with RP-HPLC, wherein the reverse phase column was Luna C18, 250 × 25mm, 5µm, 100Å, mobile phase A was 0.1% TFA/H₂O, mobile phase B was 0.1% TFA/ACN. The desired product was eluted and collected with a gradient phase B from 35% to 65% at a flow rate of 20mL/min within 60min, 4.0mg of product (purity 94.5%) was obtained. The obtained product has following structure:

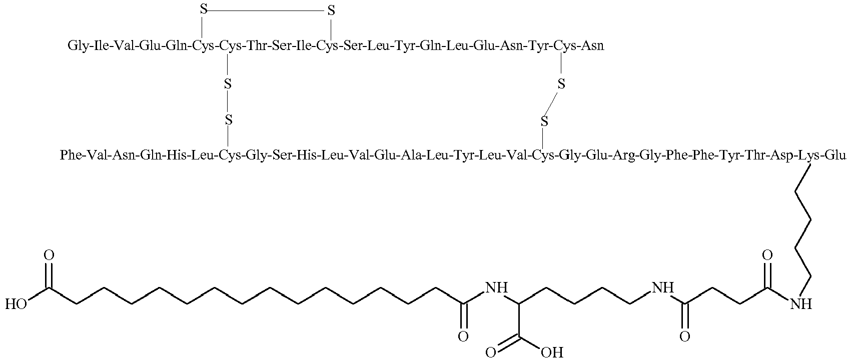

### 6. Confirmation of the structure of N^{α}-(HOOC(CH₂)₁₄CO)-N^{ε}-(OCCH₂CH₂CO-(B28D-N^{εB29}-B30E human insulin))-Lys-OH

The molecular weight of N^{α}-HOOC(CH₂)₁₄CO)-N^{ε}-(OCCH₂CH₂CO-(B28D-N^{εB29}-B30E human insulin))-Lys-OH detected by ESI-MS is 6350.68, which is consistent with the theoretically predicted molecular weight of 6350.28.

N^{α}-(HOOC(CH₂)₁₄CO)-N^{ε}-(OCCH₂CH₂CO-(B28D-N^{εB29}-B30E human insulin))-Lys-OH was digested with trypsin. The digested products were analyzed by LC-MS. The result showed the molecular weight of the two fragments F1 and F2 were 4865 and 1502, respectively, consistent with the theoretical molecular weight of the digested products. Fragment F2 corresponds to the modified B-chain fragment. Modifed sites were proven to be the same as being expected.

### Example 17. Preparation of human insulin N^{εB29}-(N^{α}-(HOOC(CH₂)₁₄CO)-γ-Glu)-Des(B30) (HS060)

The preparation procedure of the present example was the same as that for preparation of human insulin N^{εB3}-(N^{α}-(HOOC(CH₂)₁₄CO)-γ-Glu) -B29E-B30E, as described in example 14, except that human insulin B(1-27)-D-K-E, A(1-21) (Sequence 6) used in Example 14 was replaced with recombinant human insulin Des (B30) (50mg, 8.76mmol) (Example 2).

The molecular weight of N^{εB29}-(N^{α}-(HOOC(CH₂)₁₄CO)-γ-Glu)-Des(B30) human insulin detected by ESI-MS is 6103.9, which is consistent with the theoretically predicted molecular weight of 6103.5.

Human insulin N^{εB29}-(N^{α}-(HOOC(CH₂)₁₄CO)-γ-Glu)-Des(B30) was digested with trypsin. The digested products were analyzed by LC-MS. The result showed the molecular weights of the two fragments F1 and F2 were 4865 and 1256, respectively, consistent with the theoretical molecular weight of the digested products. Fragment F2 corresponds to modified B-chain fragment. Modified sites were proven to be the same as being expected. The resulted product has structure as follows:

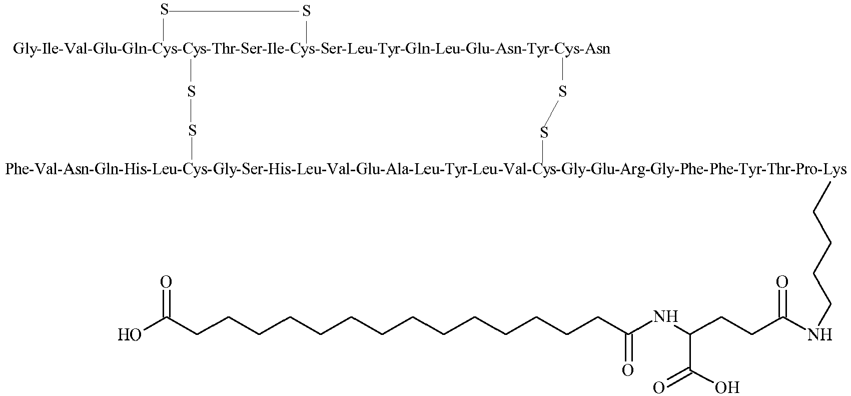

### Example 18. Preparation of human insulin N^{εB3}-(N^{α}-(HOOC(CH₂)₁₄CO)-γ-Glu)-B29E-B30E (HS065)

The preparation procedure of the present example was the same as that for the preparation of N^{εB3}-(N^{α}-(HOOC(CH₂)₁₄CO)-γ-Glu)-B29E-B30E, as described in example 14, except that human insulin B(1-27)-D-K-E, A(1-21) used in Example 14 was replaced with recombinant human insulin B(1-2)-K-B(4-28)-E-E, A(1-21) (27.2mg, 4.65mmol) (Example 6).

The molecular weight of human insulin N^{εB29}-(N^{α}-(HOOC(CH₂)₁₄CO)-γ-Glu)-B29E-B30E detected by ESI-MS is 6249, which is consistent with the theoretically predicted molecular weight of 6248.23.

Human insulin N^{εB29}-(N^{α}-(HOOC(CH₂)₁₄CO)-γ-Glu)-B29E-B30E was digested with trypsin. The digested products were analyzed by LC-MS. The result showed the molecular weights of the two fragments F1 and F2 were 5277.2 and 989, respectively, consistent with the theoretical molecular weight of the digested products. Fragment F1 corresponds to modified B-chain fragment. Modified sites were proven the same as being expected. The resulted product has structure as follows:

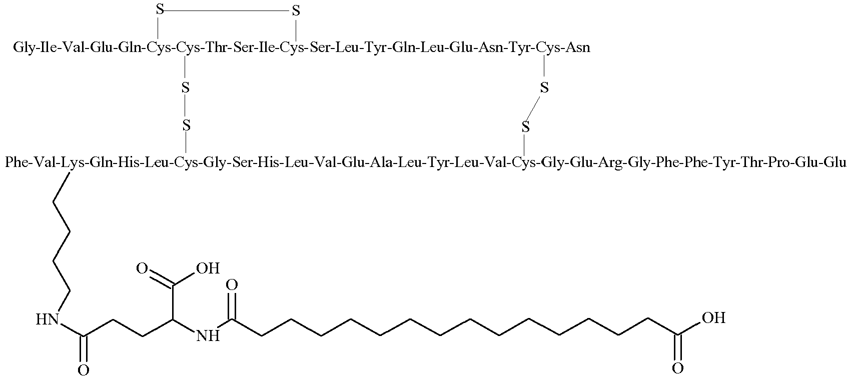

### Example 19. Preparation of N^{α}-(HOOC(CH₂)₁₄CO)-N^{ε}-(OCCH₂CH₂CO-(N^{εB3}-B29E-B30E human insulin))-Lys-OH (HS606)

The preparation procedure of the present example was the same as that for the preparation of N^{α}-(HOOC(CH₂)₁₄CO)-N^{ε}-(OCCH₂CH₂CO-(N^{εB3}-B29E-B30E human insulin))-Lys-OH, as described in example 16, except that human insulin B(1-27)-D-K-E, A(1-21) used in Example 16 was replaced with recombinant human insulin B(1-2)-K-B(4-28)-E-E, A(1-21) (25mg, 4.27mmol) (Example 6).

The molecular weight of N^{α}-HOOC(CH₂)₁₄CO)-N^{ε}-(OCCH₂CH₂CO-(N^{εB3}-B29E-B30E human insulin))-Lys-OH detected by ESI-MS is 6248, which is consistent with the theoretically predicted molecular weight of 6247.4.

N^{α}-(HOOC(CH₂)₁₄CO)-N^{ε}-(OCCH₂CH₂CO-(N^{εB3}-B29E-B30E human insulin))-Lys-OH was digested with trypsin. The digested products were analyzed by LC-MS. The result showed the molecular weights of the two fragments F1 and F2 were 5376.3 and 989, respectively, consistent with the theoretical molecular weight of the digested products. Fragment F1 corresponds to modified B-chain fragment. Modified sites were proven the same as being expected. The resulted product has structure as follows:

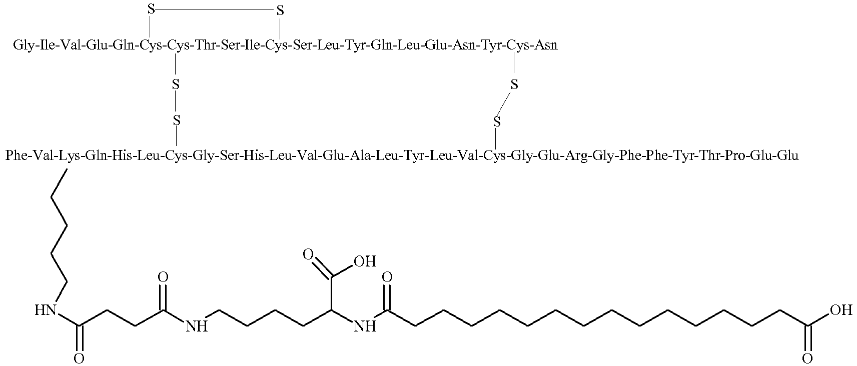

### Example 20 Preparation of human insulin N^{εB3}-(N^{α}-(HOC(CH₂)₁₅CO)-γ-Glu) -B29E-B30E (HS607)

The preparation procedure of the present example was the same as that for preparation of human insulin N^{εB3}-(N^{α}-(HOC(CH₂)₁₅CO)-γ-Glu)-B29E-B30E, as described in example 15, except that human insulin B(1-27)-D-K-E, A(1-21) used in Example 15 was replaced with recombinant human insulin B(1-2)-K-B(4-28)-E-E, A(1-21) (27.2mg, 4.65mmol) (Example 6).

The molecular weight of human insulin N^{εB3}-(N^{α}-(HOC(CH₂)₁₅CO)-γ-Glu)-B29E-B30E detected by ESI-MS is 6236, which is consistent with the theoretically predicted molecular weight of 6234.3.

Human insulin N^{εB3}-(N^{α}-(HOC(CH₂)₁₅CO)-γ-Glu)-B29E-B30E was digested with trypsin. The digested products were analyzed by LC-MS. The result showed the molecular weights of the two fragments F1 and F2 were 5263.2 and 989, respectively, consistent with the theoretical molecular weight of the digested products. Fragment F1 corresponds to modified B-chain fragment. Modified sites were proven the same as being expected. The resulted product has structure as follows:

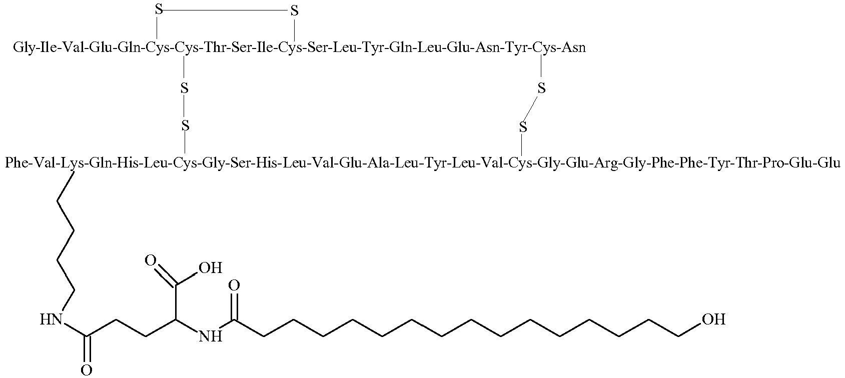

### Example 21. Preparation of human insulin N^{εB3}-(N^{α}-(HOOC(CH₂)₁₄CO) -γ-Glu)-B29E-B30E, A21G (HS608)

The preparation procedure of the present example was the same as that for preparation of N^{εB3}-(N^{α}-(HOOC(CH₂)₁₄CO)-γ-Glu)-B29E-B30E, A21G, as described in example 14, except that human insulin B(1-27)-D-K-E, A(1-21) used in Example 14 was replaced with recombinant human insulin B(1-2)-K-B(4-28)-E-E, A(1-20)-G (24.3mg, 4.19mmol) (Example 12).

The molecular weight of human insulin N^{εB3}-(N^{α}-(HOOC(CH₂)₁₄CO)-γ-Glu) -B29E-B30E, A21G detected by ESI-MS is 6192.5, which is consistent with the theoretically predicted molecular weight of 6190.5.

Human insulin N^{εB3}-(N^{α}-(HOOC(CH₂)₁₄CO)-γ-Glu)-B29E-B30E, A21G was digested with trypsin. The digested products were analyzed by LC-MS. The result showed the molecular weights of the two fragments F1 and F2 were 5219.5 and 989, respectively, consistent with the theoretical molecular weight of the digested products. Fragment F1 was corresponding to modified B-chain fragment. Modified sites were proven the same as being expected. The resulted product has structure as follows:

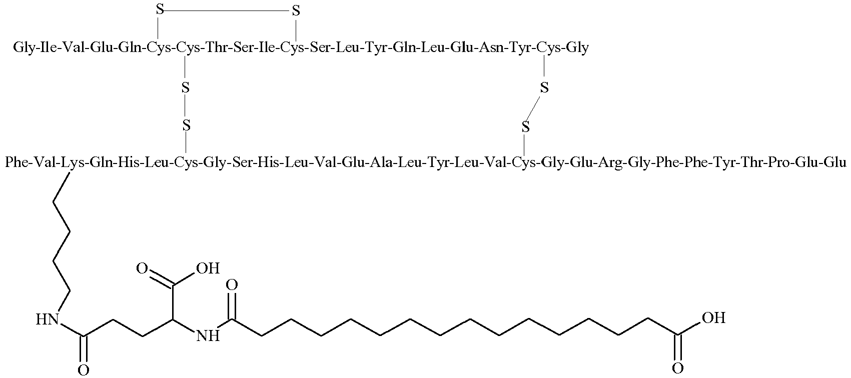

### Example 22. Preparation of N^{α}-(HOOC(CH₂)₁₄CO)-N^{ε}-(OCCH₂CH₂CO-(N^{εB3}-B29E-B30E, A21G human insulin))-Lys-OH (HS609)

The preparation procedure of the present example was the same as that for preparation of N^{α}-(HOOC(CH₂)₁₄CO)-N^{ε}-(OCCH₂CH₂CO-(N^{εB3}-B29E-B30E, A21G human insulin))-Lys-OH as described in example 16, except that human insulin B(1-27)-D-K-E, A(1-21) used in Example 16 was replaced with human insulin B(1-2)-K-B(4-28)-E-E, A(1-20)-G (24.1mg, 4.16mmol) (Example 12).

The molecular weight of N^{α}HOOC(CH₂)₁₄CO)-N^{ε}-(OCCH₂CH₂CO-(N^{εB3}-B29E-B30E, A21G human insulin))-Lys-OH detected by ESI-MS is 6289, which is consistent with the theoretically predicted molecular weight of 6289.6.

N^{α}-(HOOC(CH₂)₁₄CO)-N^{ε}-(OCCH₂CH₂CO-(N^{εB3}-B29E-B30E, A21G human insulin))-Lys-OH was digested with trypsin. The digested products were analyzed by LC-MS. The result showed the molecular weights of the two fragments F1 and F2 were 5318.5 and 989, respectively, consistent with the theoretical molecular weight of the digested products. Fragment F1 corresponds to modified B-chain fragment. Modified sites were proven the same as being expected. The resulted product has structure as follows:

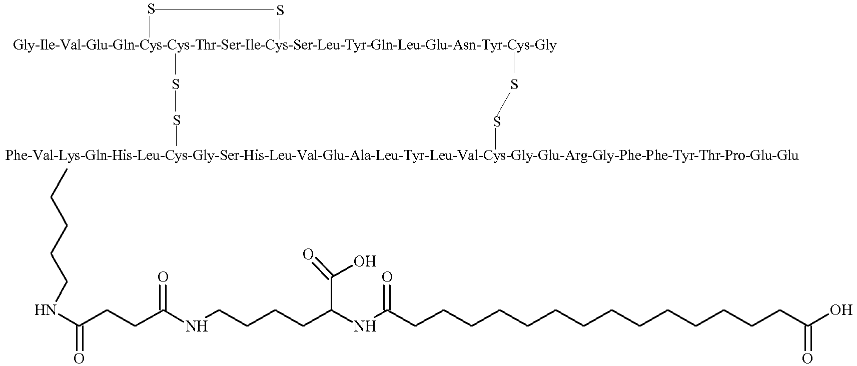

### Example 23.Preparation of human insulin N^{εB3}-(N^{α}-(HOC(CH₂)₁₅CO)-γ-Glu) -B29E-B30E, A21G (HS610)

The preparation procedure of the present example was of the same as that for preparation of human insulin N^{εB3}-(N^{α}-(HOC(CH₂)₁₅CO)-γ-Glu)-B29E-B30E, A21G as described in example 15, except that human insulin B(1-27)-D-K-E, A(1-21) used in Example 15 was replaced with recombinant human insulin B(1-2)-K-B(4-28)-E-E, A(1-20)-G (24.8mg, 4.28mmol) (Example 12).

The molecular weight of human insulin N^{εB3}-(N^{α}-(HOC(CH₂)₁₅CO)-γ-Glu) -B29E-B30E, A21G detected by ESI-MS is 6177, which is consistent with the theoretically predicted molecular weight of 6176.5.

Human insulin N^{εB3}-(N^{α}-(HOC(CH₂)₁₅CO)-γ-Glu)-B29E-B30E, A21G was digested with trypsin. The digested products were analyzed by LC-MS. The result showed the molecular weights of the two fragments F1 and F2 were 5205.4 and 989, respectively, consistent with the theoretical molecular weight of the digested products. Fragment F1 corresponds to modified B-chain fragment. Modified sites were proven the same as being expected. The resulted product has structure as follows:

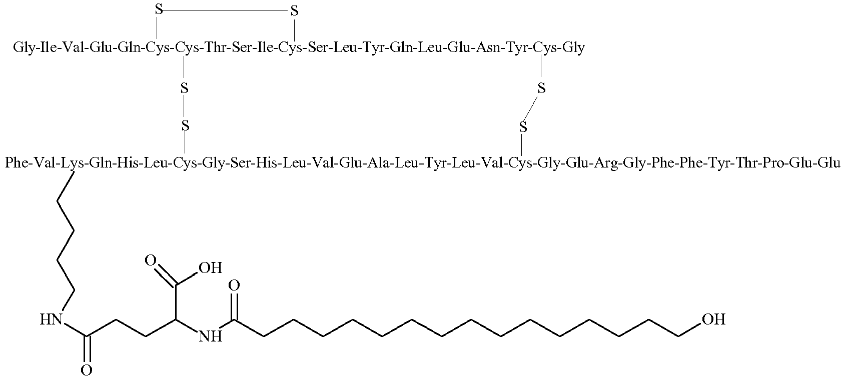

### Example 24. Preparation of PEGylated (20KD)-insulin

### 1. Pegylation (20KD) of insulin B(1-27)-D-K-E, A(1-21)

50mg of insulin B(1-27)-D-K-E, A(1-21) was dissolved in 25ml of water, pH value was adjusted to 11.40 with 1.0M Na₂CO₃, then 500mg of m-PEG-OSu solution was slowly added (m-PEG-OSu was dissolved in mixed solvent having 10ml acetonitrile and 6ml THF), the reaction solution was stirred at room temperature for 2hrs. Then pH value of the reaction system was adjusted to 6.0 with 0.1M HCl to quench the reaction, the organic solvent was removed under reduced pressure. The reaction was terminated, and purification was performed by using GE SP-Sepharose cation exchange gel. The final reaction product was diluted 10-fold with solution A (solution A, 20mM Gly pH3.0; solution B, 20mM Gly pH3.0 1M NaCl) and loaded, the column was equilibrated with solution A in 5 volumes, and eluted in linear gradient from 0 to 100% B/20 column volumes. Eluted peaks were collected, then desalted and concentrated in a 10KD entrapped ultrafiltration tube, the desired product was obtained, referred as PEG (20KD) -156.

### 2. Identification of the PEG (20KD) modified position of insulin B(1-27)-D-K-E, A(1-21)

Before and after Pegylation, the insulin B(1-27)-D-K-E, A(1-21) and PEGylated B(1-27)-D-K-E, A(1-21) were digested with GLu-C, respectively, and subjected to LC-MS analysis. The digested products are as follows:

| F-I | F-II | F-III | F-IV |
|---|---|---|---|
| | QCCTSICSLYQLE | NYCN | |
| GIVE | FVNQHLCGSHLVE | ALYLVCGE | RGFFYTDKE |

The enzymatically digested products were compared via HPLC analysis. It was found that enzymatically digested fragments F-I, F-II and F-III did not alter before and after enzymatic digestion, whereas the hydrophobicity of F-IV fragment, which was obtained from enzymatically digested PEgylated insulin B(1-27)-D-K-E, A(1-21) was dramatically increased. Such data demonstrated that the pegylated position was on B29K, the structure of which as follows:

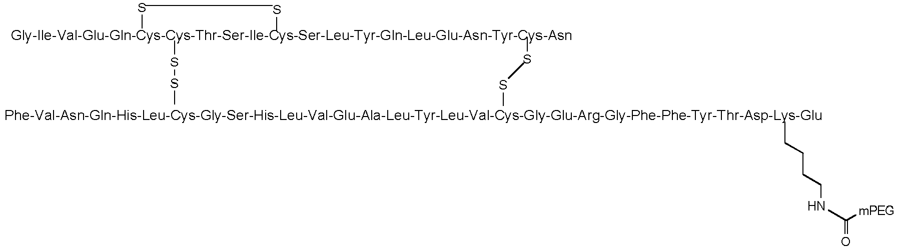

### 3. Pegylated insulin B(1-2)-K-B(4-28)-E-E, A(1-21) was prepared as described in the above Step 1, the target product was obtained, referred as PEG (20KD)-107. The resulted product was identified using the method shown in Step 2, DTT (10mM) was used for reduction of the obtained product. No change was detected in A-chain. Hydrophobicity of B-chain was greatly increased. The data demonstrated that the modified position is on position B3K. Molecular structure of modified products obtained was as follows:

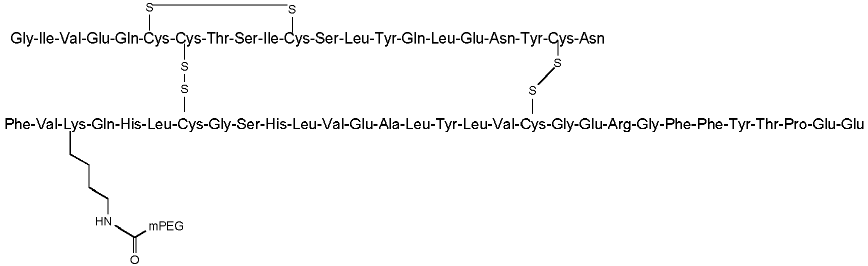

### Example 25. Preparation of PEGylated (30KD)-insulin

### 1. Pegylation (30KD) of insulin B(1-27)-D-K-E, A(1-21)

50mg of insulin B(1-27)-D-K-E, A(1-21) was dissolved in 25ml of water, pH value was adjusted to 11.40 with 1.0M Na₂CO₃, then 750mg of m-PEG-OSu(30K) solution (m-PEG-OSu was dissolved in mixed solvent of 10ml acetonitrile and 6ml THF) was added slowly, the reaction solution was stirred at room temperature for 2hrs. Then pH value of the reaction system was adjusted to 6.0 with 0.1M HCl to quench the reaction, the organic solvent was removed under reduced pressure. The reaction was terminated and purification was performed by GE SP-Sepharose cation exchange gel. The final reaction product was diluted 10-fold with solution A (solution A, 20mM Gly pH3.0; solution B, 20mM Gly pH3.0 1M NaCl) and loaded, the column was equilibrated with solution A in 5 volumes, and eluted in linear gradient from 0 to 100% B/20 column volumes. Eluted peaks were collected, then desalted and concentrated in a 10KD entrapped ultrafiltration tube, the desired product was obtained, referred as PEG (30KD) -156.

### 2. Identification of the PEG (30KD) modified position of insulin B(1-27)-D-K-E, A(1-21)

Before and after Pegylation, the insulin B(1-27)-D-K-E, A(1-21) and PEGylated B(1-27)-D-K-E, A(1-21) were digested with GLu-C, respectively. The digested products are as follows:

| F-I | F-II | F-III | F-IV |
|---|---|---|---|
| | QCCTSICSLYQLE | NYCN | |
| GIVE | FVNQHLCGSHLVE | ALYLVCGE | RGFFYTDKE |

Each digested products was analyzed by HPLC. The results showed that no change was detected in fragments FI, F-II and F-III before and after pegylation. However, hydrophobicity of digested product fragment F-IV significantly increased. The data demonstrated that the pegylation position is on B29K, the structure of which is as follows::

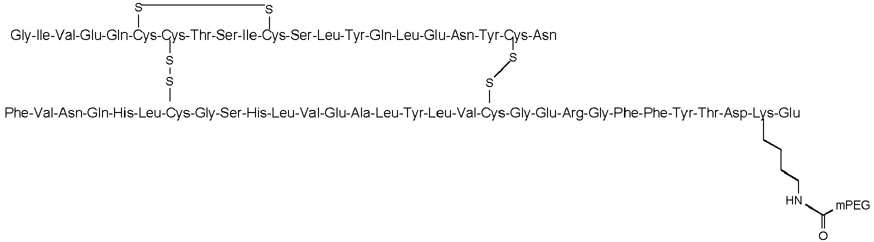

### 3. Pegylated (30KD) insulin B(1-2)-K-B(4-28)-E-E, A(1-21) was prepared as described in above Step 1 , the target product was obtained , referred as PEG (30KD) -107. The resulted product was identified using the method shown in Step 2, DTT (10mM) was used for reduction of the obtained product. No change was detected in A-chain. Hydrophobicity of B-chain was greatly increased. It demonstrated that the modified position is on position B3K. Molecular structure of modified products obtained was as follows:

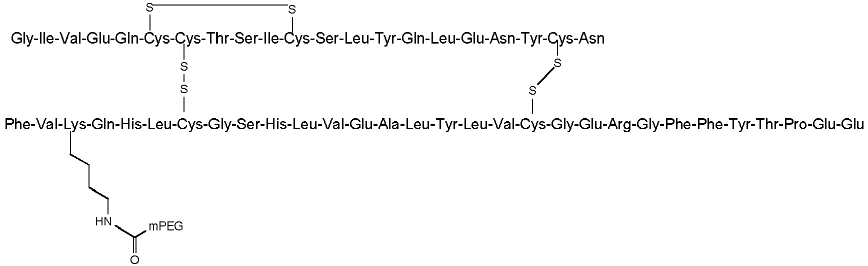

### Example 26. Preparation of PEGylated (branched 40KD) insulin

### 1. Pegylation (40KD) of insulin B(1-27)-D-K-E, A(1-21))

50mg of B(1-27)-D-K-E, A(1-21) insulin was dissolved in 25ml of water, pH value of the solution was adjusted to 11.40 with 1.0M Na₂CO₃, then 1000mg of m-PEG-OSu(40K) solution (m-PEG-OSu was dissolved in mixed solvent of 10ml acetonitrile and 6ml THF) was added slowly, the reaction was stirred at room temperature for 2hrs. Then the pH value of the reaction system was adjusted to 6.0 with 0.1M HCl to quench the reaction, the organic solvent was removed under reduced pressure. The reaction was terminated and purification was performed by GE SP-Sepharose cation exchange gel. The final reaction product was diluted 10-fold with solution A (solution A, 20mM Gly pH3.0; solution B, 20mM Gly pH3.0 1M NaCl), and loaded, the column was equilibrated with solution A in 5 volumes, and eluted in linear gradient from 0 to 100% B/20 column volumes. Eluted peaks were collected, then desalted and concentrated in a 10KD entrapped ultrafiltration tube, the desired product was obtained, referred as PEG (40KD) -156.

### 2. Identification of the PEG (40KD) modified position of insulin B(1-27)-D-K-E, A(1-21)

Before and after Pegylation, the insulin B(1-27)-D-K-E, A(1-21) and PEGylated B(1-27)-D-K-E, A(1-21) were digested with GLu-C, respectively. The digested products are as follows:

| F-I | F-II | F-III | F-IV |
|---|---|---|---|
| | QCCTSICSLYQLE | NYCN | |
| GIVE | FVNQHLCGSHLVE | ALYLVCGE | RGFFYTDKE |

Digested products were analyzed by HPLC. The results showed that no change in fragments FI, F-II and F-III was detected before and after pegylation. However, hydrophobicity of digested product fragment F-IV significantly increased. The data demonstrated that the pegylated site is on B29K, the structure of which as follows::

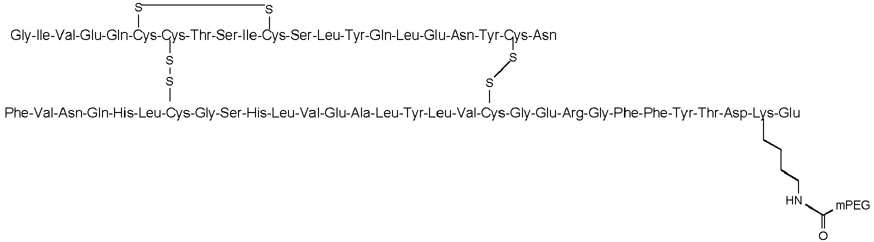

### 3. Pegylated (40KD) insulin B(1-2)-K-B(4-28)-E-E, A(1-21) was prepared as described in above Step 1, the target product was obtained, referred as PEG (40KD) -107. The resulted producted was identified using the method shown in Step 2, DTT (10mM) was used for reduction of the product. No change was detected in A-chain. Hydrophobicity of B-chain was greatly increased. It confirmed that the modified position is on B3K. Molecular structure of modified products obtained was as follows:

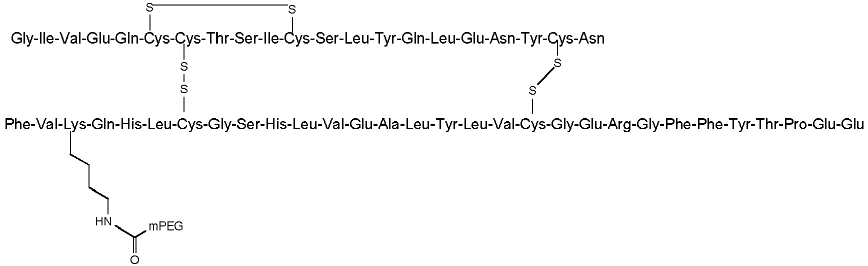

### BIOLOGICAL ASSAYS

### Test Example 1: binding assay of human insulin analogue to the insulin receptor (IR) or insulin-like growth factor receptor 1 (IGF1-R)

The relative binding intensity of human insulin analogue according to the present invention to the insulin receptor or insulin-like growth factor receptor 1 was detected by competitive receptor binding assay.

Membranes expressing insulin receptor and insulin-like growth factor-1 receptor were extracted from IM-9 and H19-7 cells (ATCC). 5 × 10⁸ cells were collected, washed with PBS and resuspended in lysis buffer (50mM Tris-HCl, pH7.8, 150mM NaCl, protease inhibitors (Roche)) in a cell density of 6 × 10⁷ cells / mL, samples were placed on ice. Samples were homogenized by electric homogenizer at 25000rpm 10sec, twice, then centrifuged at low speed (2000g, 15min), the supernatant was collected. Precipitated fraction was resuspended in an appropriate amount of the lysis buffer. The above step was repeated for three times and the supernatant from each time was pooled. The pooled supernatant was centrifuged at high speed (4°C, 35000rpm, 60min), then the resulted supernatant was discarded, the precipitated fraction was resuspended in an appropriate amount of the cell lysis buffer, experimental membrane proteins was obtained. Membrane protein extracted from the IM-9 cell was insulin receptor, referred as IM-9 cell membrane protein. Membrane protein extracted from H19-7 cell was insulin-like growth factor-1 receptor, referred as H19-7 cell membrane proteins. The protein concentration was quantified by Bradford method.

In the insulin receptor binding experiment, each well of a 96-well plate was added with 40µL of 125pM [¹²⁵I] isotope insulin (Perkin Elmer, Cat. No 420010UC), 3µg of IM-9 cell membrane protein (50µL), and 10µL gradient dilutions of human insulin analogues to be tested. All of the above solution were prepared in a reaction solution (50mM Tris-HCl, pH7.8, 150mM NaCl, 0.1% BSA). The above mixture was gently stirred at room temperature for 1hr. Membrane proteins incubated were collected by FilterMate Havester (Perkin Elmer, Cat No S / N: DA12073369) to the membrane of MicroBeta Filtermat B (Perkin Elmer, Cat No 1450-521.), which was pre-wetted with 0.3% PEI, then washed with the reaction solution for three times and dried in an oven at 60°C for 1hr. The dried filter membrane was put into a good seal membrane, added with 15mL of scintillation fluid, and sealed. Finally the result was read by Microbeta Plate Counter (Perkin Elmer, Cat. No.1450).

In insulin-like growth factor-1 receptor binding experiments, each well of a 96 well-plate was added with 40µL of 125pM [¹²⁵I] isotope IGF-1, 9µg of H19-7 cell membrane protein (50µL), and 10µL gradient dilutions of human insulin analogues to be tested. The remaining steps were the same as those in insulin receptor binding experiments.

Data obtained in above experiments were nonlinear fitting using Graphpad Prism. IC₅₀ values (nM) were obtained in binding competitive experiments of human insulin analogues to insulin receptor or insulin-like growth factor-1 receptor. The results were shown in the table below.

**Table 1: Binding assay for insulin and analogues thereof to the insulin receptor (IR) or insulin-like growth factor receptor 1 (IGF1-R)**

| Sequence NO | Example | Human insulin abbreviation | IR | IGF1-R |
|---|---|---|---|---|
| hI | 1 | B(1-30), A(1-21) | 0.46 | 316.5 |
| Des(B30)-hI | 2 | B(1-29), A(1-21) | 0.92 | 134.5 |
| 5 | 6 | B(1-2)-K-B(4-28)-E-E, A(1-21) | 0.45 | 1058 |
| 6 | 7 | B(1-27)-D-K-E, A(1-21) | 0.19 | 120.9 |
| 13 | 12 | B(1-2)-K-B(4-28)-E-E, A(1-20)-G | 0.24 | >2000 |

| | | | | |
|---|---|---|---|---|
| Note: hI, human insulin; Des (B30)-hI, human insulin lacking B30, both of which were used as positive control. | | | | |

The data demonstrated that insulin analogues of the present invention have comparative binding affinity as positive controls, wherein the binding affinity of B(1-2)-K-B(4-28)-E-E, A(1-20)-G with IR is 1.9-fold as potent as that of positive control (hI), whereas its affinity to IGF1-R is decreased to one-tenth lower than that of hI.

### Test Example 2: In vivo activity evaluation of human insulin analogues

1. ICR male mice used in this experiment were purchased from SINO-BRITSH SIPPR/BK LAB. ANIMAL LTD., CO, 18-20g. Mice were fed three days in laboratory environment, temperature 20-25°C; humidity of 40-60%.
2. Drug used in this experiment, Humulin R (Lilly Egypt, HRE046. 100IU/mL), was formulated to 1 IU / mL with 0.05N HCl (0.05 mg / kg), then diluted with 0.9% NaCl to solutions in concentrations of 0, 0.01, 0.02, 0.04, 0.08, 0.16, 0.32, 0.64 IU / mL.
3. 40 male mice were fasted for 16hrs before this experiment. In the beginning, these mice were weighted and the basic blood glucose levels were measured (cut off the tails and the blood glucose levels were detected by using Roche Glucometer and the corresponding test strips, similarly hereafter). On the basis of blood glucose values, mice were randomly divided into eight groups, five mice per group. The administration dosages to each group were as follows: 0, 0.1, 0.2, 0.4, 0.8, 1.6, 3.2 IU / kg (administration volume was 100 µL per 20 g body weight).
4. Blood glucose level (mmol/L) of each mouse was determined at 1hr after administration.
5 Data analysis: The measured blood glucose level was expressed as a percentage of the blood glucose level before administration. Drug reactions (hypoglycemic) curve were plotted with Graphpad Prism 5, ED₅₀ value (IU / kg or mg / kg) was obtained. ED₅₀ value means the dosage of drug for subcutaneous injection required for reaching 50% of maximum hypoglycemic effect.
6. ED₅₀ values of insulin analogues were showed in the table below:

**Table 2:In vivo activity assay of human insulin analogues**

| Sequence NO | Corresponding example | Human insulin abbreviation | relative activity |
|---|---|---|---|
| hI | 1 | B(1-30),A(1-21) | 1.11 |
| Des(B30)-hI | 2 | B(1-29),A(1-21) | 1.00 |
| 1 | 1 | B(1-29), R-A(1-21) | 0.96 |
| 2 | 3 | B(1-27)-K-E-R, A(1-21) | 1.29 |
| 3 | 4 | B(1-27)-K-E, A(1-21) | 0.81 |
| 4 | 5 | B(1-27)-K-P-E, A(1-21) | 0.81 |
| 5 | 6 | B(1-2)-K-B(4-28)-E-E, A(1-21) | 1.53 |
| 6 | 7 | B(1-27)-D-K-E, A(1-21) | 1.61 |
| 9 | 9 | B(1-2)-K-B(4-28)-E-R-R, A(1-21) | 1.47 |
| 11 | 10 | B(1-2)-D-B(4-29), R-A(1-21) | 1.46 |
| 12 | 11 | B(1-27)-K-E, A(1-20)-G | 0.90 |
| 13 | 12 | B(1-2)-K-B(4-28)-E-E, A(1-20)-G | 1.64 |
| 14 | 13 | B(1-2)-D-B(4-29)-R, A(1-20)-G | 1.09 |
| 15 | 13 | B(1-2)-D-B(4-29), A(1-20)-G | 1.48 |
| 16 | 13 | B(1-2)-D-B(4-29), R-A(1-20)-G | 0.91 |
| 17 | 7 | B(1-27)-D-K-E, A(1-20)-G | 1.34 |

| | | | |
|---|---|---|---|
| Note: The relative activity refers to ratio of ED₅₀ value of human insulin analogue versus ED₅₀ value of Des (B30)-hI. Des(B30)-hI was used as control for each experiment, and the ED₅₀ value of Des(B30)-hI was set to 1. | | | |

These above results indicated that the *in vivo* activity of human insulin analogues according to the present invention was comparative to that of the positive control. *In vivo* activity of some human insulin analogues, such as B(1-2)-K-B(4-28)-E-E, A(1-20)-G, was increased more than 50% compared with hI (human insulin) or Des (B30)-hI (human insulin lacking B30), and was significantly superior to the positive control.

### Test Example 3: in vivo long-acting activity assay of insulin derivatives

SD rats, male, used in this experiment were purchased from SINO-BRITSH SIPPR/BK LAB. ANIMAL LTD., CO, License number: SCXK (Shanghai) 2008-0016,18-20g. The rats were fed in environment of SPF level. ICR male mice were fed three days in laboratory environment, at temperature 20-25°C, humidity of 40-60%. The mice were fasted for 8hrs before the experiment. 100 mg/kg STZ was intraperitoneally injected to establish rat models sufferring from diabetes. Two days later, fasting blood glucose values were measured. Rats, whose blood glucose values were greater than 11.1mmol / 1, were considered as rats sufferring from diabetes. At the beginning of the experiment, rats were weighted, and the blood glucose levels were measured (cut off tails and the blood glucose levels were detected by using Roche Glucometer and the corresponding test strips, similarly hereafter). On the basis of blood glucose values, rats were randomly divided into groups, 43 rats per group. The drug to be used was prepared in required concentrations. After subcutaneous injection of drugs, and blood glucose levels were measured at multiple time points. IP (Index of Prolongation) was calculated according to blood glucose level.

### Positive control:

1. Humulin R: a Recombinant human insulin injection purchased from Lilly.
2, Detemir: a detemir insulin injection purchased from Novo Nordisk.
3, HS060 is Degludec, a molecule in phase III clinical trial of Novo Nordisk, human insulin N^{εB29}-(N^{α}-(HOOC(CH₂)₁₄CO)-γ-Glu)-Des(B30), see Example 17.

Compounds to be tested were insulin derivatives of the acylated human insulin analogues, indicated as follows:
1. HS061, B28D -N^{εB29}-(N^{α}-(HOOC(CH₂)₁₄CO)-γ-Glu)-B30E, see Example 14.
2. HS062, B28D-N^{εB29}-(N^{α}-(HO(CH₂)₁₅CO)-γ-Glu)-B30E, see Example 15.
3. HS067, N^{α}-(HOOC(CH₂)₁₄CO)-N^{ε}-(OCCH₂CH₂CO-(B28D-N^{εB29}-B30E human insulin))-Lys-OH, see Example 16.

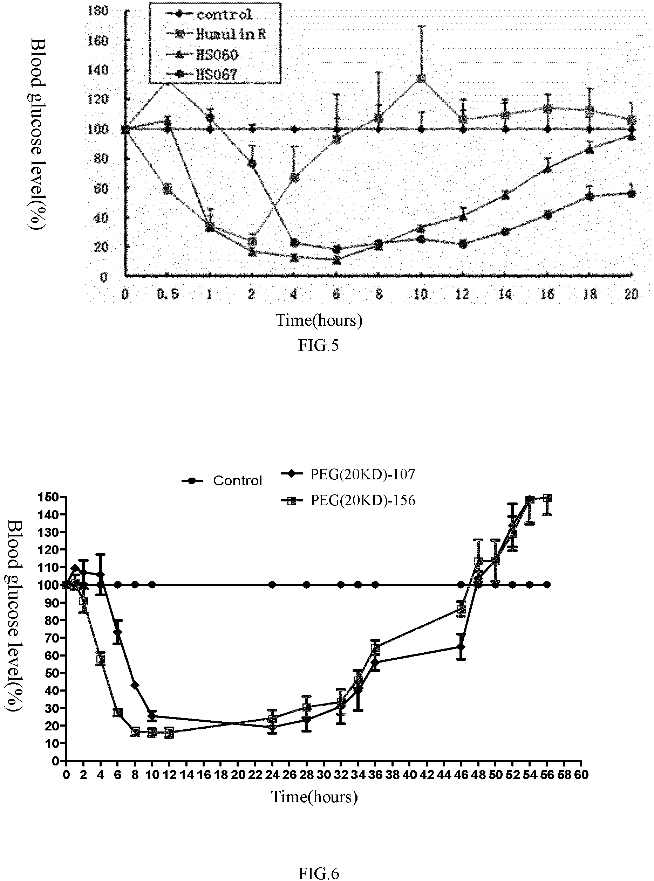

The results shown in Table 3 demonstrated that the long-action activities of HS061 (IP: 117.9), HS062 (IP: 112.3), HS067 (IP: 132.9) are all better than that ofDetemir (IP: 100) or HS060 (IP: 105.9) (both are controls). Specifically, HS067 maintained basic blood glucose level up to 12hrs in this model (results are shown in Figure 5).

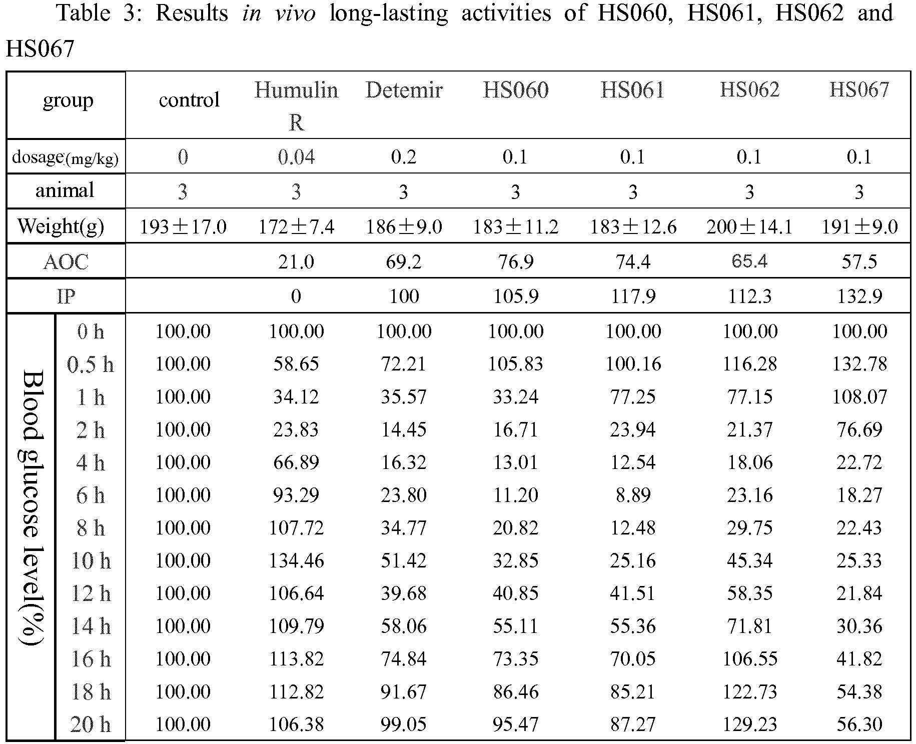

### Test Example 4: binding assay of PEGylated insulin to insulin receptor (IR)

1. The test method was the same as described in test example 1.
2. Compounds to be tested:
   PEGylated (20KD) insulin: PEG (20KD)-107 represents the PEGylated insulin B(1-2)-K-B(4-28)-E-E, A(1-21), wherein the molecular weight of PEG is 20KD; PEG (20KD)-156 represents PEGylated insulin B(1-27)-D-K-E, A(1-21), wherein the molecular weight of PEG is 20KD). Particularly see Example 24.
3. In competitive binding assay, IC₅₀ values (nM) of PEGylated (20KD) insulin binding to insulin receptor were shown in Table 4. It showed that the preferred sequence according to this invention can still bind to insulin receptor (IR) after pegylation.

**Table 4: Binding assay of PEGylated insulin to insulin receptor (IR)**

| Sequence NO | Corresponding example | Human insulin abbreviation | IR(nM) |
|---|---|---|---|
| 6 | 24 | PEG (20KD) -107 | 18.8 |
| 5 | 24 | PEG (20KOD) -156 | 71.8 |

### Test Example 5: in vivo long-acting activity assay of PEGylated insulin

### 1. Test methods were as same as described in Test Example 3.

### 2. Compounds to be tested:

1). PEGylated (20KD) insulin: PEG (20KD)-107 represents the pegylated insulin B(1-2)-K-B(4-28)-E-E, A(1-21), wherein molecular weight of PEG is 20KD; PEG (20KD)-156 represents pegylated (20KD)insulin B(1-27)-D-K-E, A(1-21). Particularly see Example 24.
2). PEGylated (30KD) insulin: PEG (30KD)-107 represents the Pegylated (30KD) insulin B(1-2)-K-B(4-28)-E-E, A(1-21); PEG(30KD)-156 represents pegylated insulin (30KD) B(1-27)-D-K-E, A(1-21). Particularly see Example 25.
3). PEG (40KD) insulin: PEG (40KD) -107 represents the Pegylated (branched 40KD) insulin B(1-2)-K-B(4-28)-E-E, A(1-21); PEG(40KD)-156 represents pegylated (branched 40KD) insulin B(1-27)-D-K-E, A(1-21). Particularly see Example 26.

### 3. Test Results

### 1). Results of in vivo evaluation for long-acting activity of PEGylated (20KD) insulin were shown in the following Table 5 and Figure 6.

**Table 5: Results of in vivo evaluation for long-acting activity of PEGylated (20KD) insulin B(1-27)-D-K-E, A(1-21) and B(1-2)-K-B(4-28)-E-E, A(1-21)**

| group | | control | Humulin R | Detemir | PEG(20KD)-107 | PEG(20KD)-156 |
|---|---|---|---|---|---|---|
| Dosage(mg/kg) | | 0 | 0.04 | 0.2 | 0.1 | 0.1 |
| Numbers of Animals | | 4 | 4 | 4 | 4 | 4 |
| Weight (g) | | 136 | 139 | 130 | 137 | 139 |
| AOC% | | | 5.25 | 7.39 | 51.27 | 51.71 |
| IP | | | 0 | 100 | 207.36 | 204.26 |
| Blood glucose value (%) | 0h | 100 | 100 | 100 | 100 | 100 |
| | 1h | 100 | 45.94 | 96.63 | 109.43 | 101.39 |
| | 2h | 100 | 25.26 | 57.24 | 106.92 | 91.03 |
| | 4h | 100 | 58.85 | 32.49 | 105.73 | 58.14 |
| | 6h | 100 | 86.49 | 40.98 | 73.21 | 27.32 |
| | 8h | 100 | 138.71 | 86.94 | 43 | 16.52 |
| | 10h | 100 | 152.95 | 112.66 | 25.48 | 16.14 |
| | 12h | 100 | 151.89 | 127.52 | 23.81 | 16.06 |
| | 24h | 100 | | | 19.09 | 24.21 |
| | 28h | 100 | | | 23.19 | 30.5 |
| | 32h | 100 | | | 30.82 | 33.49 |
| | 34h | 100 | | | 39.89 | 46.34 |
| | 36h | 100 | | | 55.94 | 64.5 |
| | 46h | 100 | | | 64.89 | 87.17 |
| | 48h | 100 | | | 103.59 | 113.56 |
| | 50h | 100 | | | 113.66 | 113.64 |
| | 52h | 100 | | | 133.77 | 129.07 |
| | 54h | 100 | | | 148.74 | 144.42 |
| | 56h | 100 | | | 153.91 | 149.59 |

| | | | | | | |
|---|---|---|---|---|---|---|
| Conclusion: The results in Table 5 showed that the PEG (20KD)-107 and PEG (20KD)-156 maintained stable blood glucose level up to 32- 46hrs, see Figure 6. IP (Index of Prolongation) of the two were approximate, were 207.36 and 204.26, respectively (see Table 5), which were significantly better than that of positive control. | | | | | | |

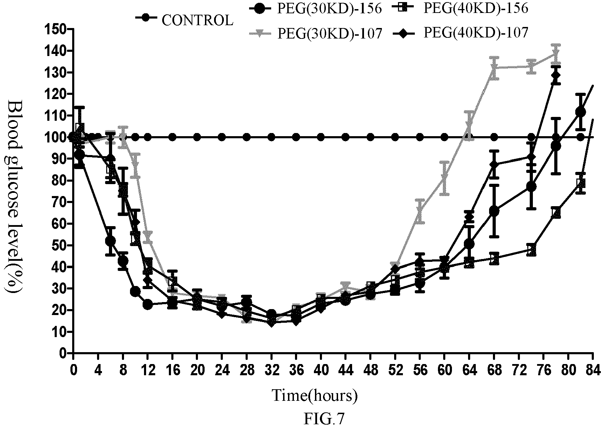

### 2. Results of in vivo Evaluations of the long-acting activity of PEGylated (30KD) insulin and PEGylated (40KD) insulin were shown in the following Table 6 and Figure 7.

**Table 6: Results of in vivo long-acting activity of PEG (30KD)-107, PEG (40KD)-107, PEG (30KD)-156 and PEG (40KD)-156**

| Group | | control | Humulin R | Detemir | PEG(30KD) -107 | PEG(40KD) -107 | PEG(30KD) -156 | PEG(40KD) -156 |
|---|---|---|---|---|---|---|---|---|
| Dosage(mg/kg) | | 0 | 0.04 | 0.2 | 0.1 | 0.1 | 0.1 | 0.1 |
| Numbers of Animals | | 4 | 4 | 4 | 4 | 4 | 4 | 4 |
| Weight (g) | | 130 | 126 | 130 | 125 | 142 | 133 | 133 |
| AOC% | | | 4.55 | 5.92 | 44.43 | 49.40 | 56.44 | 56.76 |
| IP | | | 0 | 100 | 282.5 | 285.0 | 289.8 | 297.4 |
| Blood glucose value (%) | 0 | 100 | 100.00 | 100.00 | 100.00 | 100.00 | 100.00 | 100.00 |
| | 1 | 100 | 42.42 | 104.15 | 96.80 | 91.48 | 91.83 | 104.61 |
| | 2 | 100 | 20.48 | 74.83 | 81.25 | 80.58 | 78.64 | 83.09 |
| | 3 | 100 | 28.59 | 46.46 | 86.28 | 91.16 | 85.00 | 95.43 |
| | 4 | 100 | 37.50 | 23.36 | 85.71 | 83.59 | 68.84 | 83.55 |
| | 6 | 100 | 90.10 | 17.11 | 99.95 | 90.46 | 51.95 | 85.54 |
| | 8 | 100 | 119.53 | 32.52 | 99.93 | 75.16 | 42.96 | 75.72 |
| | 10 | 100 | 115.95 | 94.89 | 86.79 | 60.73 | 28.67 | 53.28 |
| | 12 | 100 | 111.03 | 110.45 | 53.75 | 33.90 | 22.28 | 40.84 |
| | 16 | 100 | | | 27.91 | 24.32 | 23.52 | 33.61 |
| | 20 | 100 | | | 26.69 | 22.07 | 25.64 | 25.00 |
| | 24 | 100 | | | 25.59 | 18.25 | 21.91 | 23.78 |
| | 28 | 100 | | | 16.79 | 16.32 | 23.12 | 19.47 |
| | 32 | 100 | | | 13.82 | 14.38 | 18.00 | 16.22 |
| | 36 | 100 | | | 21.29 | 15.02 | 17.74 | 20.28 |
| | 40 | 100 | | | 24.64 | 20.74 | 23.01 | 25.44 |
| | 44 | 100 | | | 30.52 | 26.78 | 25.04 | 25.79 |
| | 48 | 100 | | | 28.49 | 28.51 | 27.41 | 31.25 |
| | 52 | 100 | | | 39.14 | 39.00 | 29.26 | 34.18 |
| | 56 | 100 | | | 65.78 | 42.57 | 32.61 | 37.64 |
| | 60 | 100 | | | 81.03 | 43.12 | 39.00 | 39.85 |
| | 64 | 100 | | | 105.43 | 63.25 | 50.70 | 42.13 |
| | 68 | 100 | | | 132.02 | 87.42 | 66.91 | 43.95 |
| | 74 | 100 | | | 132.73 | 90.82 | 77.16 | 48.04 |
| | 78 | 100 | | | 138.56 | 128.71 | 96.09 | 65.20 |
| | 82 | 100 | | | | | 111.74 | 78.79 |
| | 86 | 100 | | | | | 135.91 | 137.17 |

| | | | | | | | | |
|---|---|---|---|---|---|---|---|---|
| Conclusion: The results in table 6 and Figure 7 showed that PEG (30KD) -107 (IP: 282.5) maintained blood glucose level stable up to 48hrs in mice; PEG (40KD) -107 (IP: 285.0) maintained blood glucose level stable up to 60hrs in mice; PEG (30KD) -156 (IP: 289.8) maintained blood glucose level stable up to 56hrs in mice; PEG (40KD) -156 (IP: 297.4) maintained blood glucose level stable up to 76hrs in mice, which were significantly better than that of positive control. | | | | | | | | |

SEQUENCE LISTING

<110¡µShanghai hengrui pharmaceutical co.,LTD Jiangsu hengrui medicine CO.,LTD

<120¡µHUMAN INSULIN ANALOGUE AND ACYLATED DERIVATIVE THEREOF

<130> 810308CG-EF

<160¡µ78

<170> PatentIn version 3.1

<210> 1
<211> 21
<212> PRT
<213> HUMAN

¡´220¡µ
¡´223¡µHUMAN INSULIN ANALOGUE A CHAIN

<400> 1
Gly Ile Val Glu Gln Cys Cys Thr Ser Ile Cys Ser Leu Tyr Gln
                5                   10                  15
Leu Glu Asn Tyr Cys Asn
                20

¡´210¡µ2
¡´211¡µ21
¡´212¡µPRT
¡´213¡µARTIFICIAL

¡´220¡µ
¡´223¡µHUMAN INSULIN ANALOGUE A CHAIN

¡´400¡µ2
Gly Ile Val Glu Gln Cys Cys Thr Ser Ile Cys Ser Leu Tyr Gln
                5                   10                  15
Leu Glu Asn Tyr Cys Gly
                20
¡´210¡µ3
¡´211¡µ22
¡´212¡µPRT
¡´213¡µARTIFICIAL

¡´220¡µ
¡´223¡µHUMAN INSULIN ANALOGUE A CHAIN

¡´400¡µ3
Arg Gly Ile Val Glu Gln Cys Cys Thr Ser Ile Cys Ser Leu Tyr
                5                   10                  15
Gln Leu Glu Asn Tyr Cys Gly
                20

¡´210¡µ4
¡´211¡µ22
¡´212¡µPRT
¡´213¡µARTIFICIAL

¡´220¡µ
¡´223¡µHUMAN INSULIN ANALOGUE A CHAIN

¡´400¡µ4

Arg Gly Ile Val Glu Gln Cys Cys Thr Ser Ile Cys Ser Leu Tyr
                5                   10                  15
Gln Leu Glu Asn Tyr Cys Asn
                20

¡´210¡µ5
¡´211¡µ29
¡´212¡µPRT
¡´213¡µARTIFICIAL

¡´220¡µ
¡´223¡µHUMAN INSULIN ANALOGUE B CHAIN

¡´400¡µ5
Phe Val Asn Gln His Leu Cys Gly Ser His Leu Val Glu Ala Leu
                5                   10                  15
Tyr Leu Val Cys Gly Glu Arg Gly Phe Phe Tyr Thr Pro Lys
                20                  25

¡´210¡µ6
¡´211¡µ30
¡´212¡µPRT
¡´213¡µARTIFICIAL

¡´220¡µ
¡´223¡µHUMAN INSULIN ANALOGUE B CHAIN

¡´400¡µ6
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Glu Arg
                20                  25                  30

¡´210¡µ7
¡´211¡µ29
¡´212¡µPRT
¡´213¡µARTIFICIAL

¡´220¡µ
¡´223¡µHUMAN INSULIN ANALOGUE B CHAIN

¡´400¡µ7
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Glu
                20                  25

¡´210¡µ8
¡´211¡µ30
¡´212¡µPRT
¡´213¡µARTIFICIAL

¡´220¡µ
¡´223¡µHUMAN INSULIN ANALOGUE B CHAIN

¡´400¡µ8
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Pro Glu
                20                  25                  30

¡´210¡µ9
¡´211¡µ30

¡´212¡µPRT
¡´213¡µARTIFICIAL

¡´220¡µ
¡´223¡µHUMAN INSULIN ANALOGUE B CHAIN

¡´400¡µ9
Phe Val Lys Gln His Leu Cys Gly Ser His Leu Val Glu Ala Leu
                5                   10                  15
Tyr Leu Val Cys Gly Glu Arg Gly Phe Phe Tyr Thr Pro Glu Glu
                20                  25                  30

¡´210¡µ10
¡´211¡µ53
¡´212¡µPRT
¡´213¡µARTIFICIAL

¡´220¡µ
¡´223¡µHUMAN INSULIN ANALOGUE B CHAIN

¡´400¡µ10
Phe Val Asn Gln His Leu Cys Gly Ser His Leu Val Glu Ala Leu
                5                   10                  15
Tyr Leu Val Cys Gly Glu Arg Gly Phe Phe Tyr Thr Asp Lys Glu
                20                  25                  30

¡´210¡µ11
¡´211¡µ32
¡´212¡µPRT
¡´213¡µARTIFICIAL

¡´220¡µ
¡´223¡µHUMAN INSULIN ANALOGUE B CHAIN

¡´400¡µ11
Phe Val Asp Gln His Leu Cys Gly Ser His Leu Val Glu Ala Leu
                5                   10                  15
Tyr Leu Val Cys Gly Glu Arg Gly Phe Phe Tyr Thr Pro Lys Thr
                20                  25                  30
Arg Arg

¡´210¡µ12
¡´211¡µ31
¡´212¡µPRT
¡´213¡µARTIFICIAL

¡´220¡µ
¡´223¡µHUMAN INSULIN ANALOGUE B CHAIN

¡´400¡µ12
Phe Val Asp Gln His Leu Cys Gly Ser His Leu Val Glu Ala Leu
                5                   10                  15
Tyr Leu Val Cys Gly Glu Arg Gly Phe Phe Tyr Thr Pro Lys Thr Arg
                20                  25                  30

¡´210¡µ13
¡´211¡µ31
¡´212¡µPRT
¡´213¡µARTIFICIAL

¡´220¡µ
¡´223¡µHUMAN INSULIN ANALOGUE B CHAIN

¡´400¡µ13
Phe Val Lys Gln His Leu Cys Gly Ser His Leu Val Glu Ala Leu
                5                   10                  15
Tyr Leu Val Cys Gly Glu Arg Gly Phe Phe Tyr Thr Pro Glu Arg Arg
                20                  25                  30

¡´210¡µ14
¡´211¡µ29
¡´212¡µPRT
¡´213¡µARTIFICIAL

¡´220¡µ
¡´223¡µHUMAN INSULIN ANALOGUE B CHAIN

¡´400¡µ14
Phe Val Asp Gln His Leu Cys Gly Ser His Leu Val Glu Ala Leu
                5                   10                  15
Tyr Leu Val Cys Gly Glu Arg Gly Phe Phe Tyr Thr Pro Lys
                20                  25

¡´210¡µ15
¡´211¡µ29
¡´212¡µPRT
¡´213¡µARTIFICIAL

¡´220¡µ
¡´223¡µHUMAN INSULIN ANALOGUE B CHAIN

¡´400¡µ15
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Glu
                20                  25

¡´210¡µ16
¡´211¡µ30
¡´212¡µPRT
¡´213¡µARTIFICIAL

¡´220¡µ
¡´223¡µHUMAN INSULIN ANALOGUE B CHAIN

¡´400¡µ16
Phe Val Lys Gln His Leu Cys Gly Ser His Leu Val Glu Ala Leu
                5                   10                  15
Tyr Leu Val Cys Gly Glu Arg Gly Phe Phe Tyr Thr Pro Glu Glu
                20                  25                  30

¡´210¡µ17
¡´211¡µ30
¡´212¡µPRT
¡´213¡µARTIFICIAL

¡´220¡µ
¡´223¡µHUMAN INSULIN ANALOGUE B CHAIN

¡´400¡µ17
Phe Val Asp Gln His Leu Cys Gly Ser His Leu Val Glu Ala Leu
                5                   10                  15
Tyr Leu Val Cys Gly Glu Arg Gly Phe Phe Tyr Thr Pro Lys Arg
                20                  25                  30

¡´210¡µ18

¡´211¡µ29
¡´212¡µPRT
¡´213¡µARTIFICIAL

¡´220¡µ
¡´223¡µHUMAN INSULIN ANALOGUE B CHAIN

¡´400¡µ18
Phe Val Asp Gln His Leu Cys Gly Ser His Leu Val Glu Ala Leu
                5                   10                  15
Tyr Leu Val Cys Gly Glu Arg Gly Phe Phe Tyr Thr Pro Lys
                20                  25

¡´210¡µ19
¡´211¡µ53
¡´212¡µPRT
¡´213¡µARTIFICIAL

¡´220¡µ
¡´223¡µHUMAN INSULIN ANALOGUE PRECURSOR

¡´400¡µ19
Phe Val Asn Gln His Leu Cys Gly Ser His Leu Val Glu Ala Leu
                5                   10                  15
Tyr Leu Val Cys Gly Glu Arg Gly Phe Phe Tyr Thr Asp Lys Glu
                20                  25                  30
Lys Arg Gly Ile Val Glu Gln Cys Cys Thr Ser Ile Cys Ser Leu
                35                  40                  45
Tyr Gln Leu Glu Asn Tyr Cys Asn
                50

¡´210¡µ20
¡´211¡µ53
¡´212¡µPRT
¡´213¡µARTIFICIAL

¡´220¡µ
¡´223¡µHUMAN INSULIN ANALOGUE PRECURSOR

¡´400¡µ20
Phe Val Asn Gln His Leu Cys Gly Ser His Leu Val Glu Ala Leu
                5                   10                  15
Tyr Leu Val Cys Gly Glu Arg Gly Phe Phe Tyr Thr Pro Glu Glu
                20                  25                  30
Lys Arg Gly Ile Val Glu Gln Cys Cys Thr Ser Ile Cys Ser Leu
                35                  40                  45
Tyr Gln Leu Glu Asn Tyr Cys Gly
                50

¡´210¡µ21
¡´211¡µ53
¡´212¡µPRT
¡´213¡µARTIFICIAL

¡´220¡µ
¡´223¡µHUMAN INSULIN ANALOGUE PRECURSOR

¡´400¡µ21
Phe Val Asn Gln His Leu Cys Gly Ser His Leu Val Glu Ala Leu
                5                   10                  15
Tyr Leu Val Cys Gly Glu Arg Gly Phe Phe Tyr Thr Pro Glu Glu

20                  25                  30
Lys Arg Gly Ile Val Glu Gln Cys Cys Thr Ser Ile Cys Ser Leu
                35                  40                  45
Tyr Gln Leu Glu Asn Tyr Cys Asn
                50

¡´210¡µ22
¡´211¡µ53
¡´212¡µPRT
¡´213¡µARTIFICIAL

¡´220¡µ
¡´223¡µHUMAN INSULIN ANALOGUE PRECURSOR

¡´400¡µ22
Phe Val Asn Gln His Leu Cys Gly Ser His Leu Val Glu Ala Leu
                5                   10                  15
Tyr Leu Val Cys Gly Glu Arg Gly Phe Phe Tyr Thr Pro Lys Thr
                20                  25                  30
Lys Arg Gly Ile Val Glu Gln Cys Cys Thr Ser Ile Cys Ser Leu
                35                  40                  45
Tyr Gln Leu Glu Asn Tyr Cys Asn
                50

¡´210¡µ23
¡´211¡µ51
¡´212¡µPRT
¡´213¡µARTIFICIAL

¡´220¡µ
¡´223¡µHUMAN INSULIN ANALOGUE PRECURSOR

¡´400¡µ23
Phe Val Asn Gln His Leu Cys Gly Ser His Leu Val Glu Ala Leu
                5                   10                  15
Tyr Leu Val Cys Gly Glu Arg Gly Phe Phe Tyr Thr Pro Lys Arg
                20                  25                  30
Gly Ile Val Glu Gln Cys Cys Thr Ser Ile Cys Ser Leu Tyr Gln
                35                  40                  45
Leu Glu Asn Tyr Cys Asn
                50

¡´210¡µ24
¡´211¡µ54
¡´212¡µPRT
¡´213¡µARTIFICIAL

¡´220¡µ
¡´223¡µHUMAN INSULIN ANALOGUE PRECURSOR

¡´400¡µ24
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Glu Arg
                20                  25                  30
Ala Ala Lys Gly Ile Val Glu Gln Cys Cys Thr Ser Ile Cys Ser
                35                  40                  45
Leu Tyr Gln Leu Glu Asn Tyr Cys Asn
                50

¡´210¡µ25
¡´211¡µ52

¡´212¡µPRT
¡´213¡µARTIFICIAL

¡´220¡µ
¡´223¡µHUMAN INSULIN ANALOGUE PRECURSOR

¡´400¡µ25
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Glu Lys
                20                  25                  30
Arg Gly Ile Val Glu Gln Cys Cys Thr Ser Ile Cys Ser Leu Tyr
                35                  40                  45
Gln Leu Glu Asn Tyr Cys Asn
                50

¡´210¡µ26
¡´211¡µ53
¡´212¡µPRT
¡´213¡µARTIFICIAL

¡´220¡µ
¡´223¡µHUMAN INSULIN ANALOGUE PRECURSOR

¡´400¡µ26
Phe Val Asn Gln His Leu Cys Gly Ser His Leu Val Glu Ala Leu
                5                   10                  15
Tyr Leu Val Cys Gly Glu Arg Gly Phe Phe Tyr Thr Lys Pro Glu
                20                  25                  30
Lys Arg Gly Ile Val Glu Gln Cys Cys Thr Ser Ile Cys Ser Leu
                35                  40                  45
Tyr Gln Leu Glu Asn Tyr Cys Asn
                50

¡´210¡µ27
¡´211¡µ53
¡´212¡µPRT
¡´213¡µARTIFICIAL

¡´220¡µ
¡´223¡µHUMAN INSULIN ANALOGUE PRECURSOR

¡´400¡µ27
Phe Val Asp Gln His Leu Cys Gly Ser His Leu Val Glu Ala Leu
                5                   10                  15
Tyr Leu Val Cys Gly Glu Arg Gly Phe Phe Tyr Thr Pro Lys Thr
                20                  25                  30
Arg Arg Gly Ile Val Glu Gln Cys Cys Thr Ser Ile Cys Ser Leu
                35                  40                  45
Tyr Gln Leu Glu Asn Tyr Cys Gly
                50

¡´210¡µ28
¡´211¡µ52
¡´212¡µPRT
¡´213¡µARTIFICIAL

¡´220¡µ
¡´223¡µHUMAN INSULIN ANALOGUE PRECURSOR

¡´400¡µ28
Phe Val Lys Gln His Leu Cys Gly Ser His Leu Val Glu Ala Leu
                5                   10                  15

Tyr Leu Val Cys Gly Glu Arg Gly Phe Phe Tyr Thr Pro Glu Arg
                20                  25                  30
Arg Gly Ile Val Glu Gln Cys Cys Thr Ser Ile Cys Ser Leu Tyr
                35                  40                  45
Gln Leu Glu Asn Tyr Cys Asn
                50

¡´210¡µ29
¡´211¡µ51
¡´212¡µPRT
¡´213¡µARTIFICIAL

¡´220¡µ
¡´223¡µHUMAN INSULIN ANALOGUE PRECURSOR

¡´400¡µ29
Phe Val Asp Gln His Leu Cys Gly Ser His Leu Val Glu Ala Leu
                 5                  10                  15
Tyr Leu Val Cys Gly Glu Arg Gly Phe Phe Tyr Thr Pro Lys Arg
                20                  25                  30
Gly Ile Val Glu Gln Cys Cys Thr Ser Ile Cys Ser Leu Tyr Gln
                35                  40                  45
Leu Glu Asn Tyr Cys Asn
                50

¡´210¡µ30
¡´211¡µ52
¡´212¡µPRT
¡´213¡µARTIFICIAL

¡´220¡µ
¡´223¡µHUMAN INSULIN ANALOGUE PRECURSOR

¡´400¡µ30
Phe Val Asn Gln His Leu Cys Gly Ser His Leu Val Glu Ala Leu
                 5                  10                  15
Tyr Leu Val Cys Gly Glu Arg Gly Phe Phe Tyr Thr Lys Glu Lys
                20                  25                  30
Arg Gly Ile Val Glu Gln Cys Cys Thr Ser Ile Cys Ser Leu Tyr
                35                  40                  45
Gln Leu Glu Asn Tyr Cys Gly
                50

¡´210¡µ31
¡´211¡µ51
¡´212¡µPRT
¡´213¡µARTIFICIAL

¡´220¡µ
¡´223¡µHUMAN INSULIN ANALOGUE PRECURSOR

¡´400¡µ31
Phe Val Asp Gln His Leu Cys Gly Ser His Leu Val Glu Ala Leu
                 5                  10                  15
Tyr Leu Val Cys Gly Glu Arg Gly Phe Phe Tyr Thr Pro Lys Arg
                20                  25                  30
Gly Ile Val Glu Gln Cys Cys Thr Ser Ile Cys Ser Leu Tyr Gln
                35                  40                  45
Leu Glu Asn Tyr Cys Gly
                50

¡´210¡µ32
¡´211¡µ162

¡´212¡µDNA
¡´213¡µARTIFICIAL

¡´220¡µ
¡´223¡µDNA ENCODING HUMAN INSULIN ANALOGUE PRECURSOR

¡´400¡µ32
tttgtgaacc aacacctgtg cggctcacac ctggtggaag ctctctacct agtgtgcggg
60
gaacgaggct tcttctacac acccaagacc aagcgtggca ttgtggaaca atgctgtacc
120
agcatctgct ccctctacca gctggagaac tactgcaact ag
162

¡´210¡µ33
¡´211¡µ156
¡´212¡µDNA
¡´213¡µARTIFICIAL

¡´220¡µ
¡´223¡µDNA ENCODING HUMAN INSULIN ANALOGUE PRECURSOR

¡´400¡µ33
tttgtgaacca acacctgtgc ggctcacacc tggtggaagc tctctaccta gtgtgcggg
60
gaacgaggctt cttctacaca cccaagcgtg gcattgtgga acaatgctgt accagcatc
120
tgctccctcta ccagctggag aactactgca actag
156

¡´210¡µ34
¡´211¡µ165
¡´212¡µDNA
¡´213¡µARTIFICIAL

¡´220¡µ
¡´223¡µDNA ENCODING HUMAN INSULIN ANALOGUE PRECURSOR

¡´400¡µ34
ttcgtcaacc agcacttgtg tggttcccat ttggttgagg ctctgtactt ggtctgtgga
60
gaaagaggtt tcttttacac caaggaaaga gctgctaaag gtatcgttga gcaatgttgc
120
acctctattt gttccctgta tcagttggaa aactactgca actaa
165

¡´210¡µ35
¡´211¡µ159
¡´212¡µDNA
¡´213¡µARTIFICIAL

¡´220¡µ
¡´223¡µDNA ENCODING HUMAN INSULIN ANALOGUE PRECURSOR

¡´400¡µ35
ttcgtcaacc agcacttgtg tggttcccat ttggttgagg ctctgtactt ggtctgtgga
60
gaaagaggtt tcttttacac caaggaaaaa agaggtatcg ttgagcaatg ttgcacctct
120
atttgttccc tgtatcagtt ggaaaactac tgcaactaa
159
35

¡´210¡µ36
¡´211¡µ162
¡´212¡µDNA
¡´213¡µARTIFICIAL

¡´220¡µ
¡´223¡µDNA ENCODING HUMAN INSULIN ANALOGUE PRECURSOR

¡´400¡µ36
ttcgtcaacc agcacttgtg tggttcccat ttggttgagg ctctgtactt ggtctgtgga
60
gaaagaggtt tcttttacac caagcctgaa aaaagaggta tcgttgagca atgttgcacc
120
tctatttgtt ccctgtatca gttggaaaac tactgcaact aa
162

¡´210¡µ37
¡´211¡µ162
¡´212¡µDNA
¡´213¡µARTIFICIAL

¡´220¡µ
¡´223¡µDNA ENCODING HUMAN INSULIN ANALOGUE PRECURSOR

¡´400¡µ37
ttcgtcaagc agcacttgtg tggttcccat ttggttgagg ctctgtactt ggtctgtgga
60
gaaagaggtt tcttttacac ccctgaagaa aaaagaggta tcgttgagca atgttgcacc
120
tctatttgtt ccctgtatca gttggaaaac tactgcaact aa
162

¡´210¡µ38
¡´211¡µ162
¡´212¡µDNA
¡´213¡µARTIFICIAL

¡´220¡µ
¡´223¡µDNA ENCODING HUMAN INSULIN ANALOGUE PRECURSOR

¡´400¡µ38
ttcgtcaacc agcacttgtg tggttcccat ttggttgagg ctctgtactt ggtctgtgga
60
gaaagaggtt tcttttacac cgataaggaa aaaagaggta tcgttgagca atgttgcacc
120
tctatttgtt ccctgtatca gttggaaaac tactgcaact aa
162

¡´210¡µ39
¡´211¡µ162
¡´212¡µDNA
¡´213¡µARTIFICIAL

¡´220¡µ
¡´223¡µDNA ENCODING HUMAN INSULIN ANALOGUE PRECURSOR

¡´400¡µ39
ttcgtcgatc agcacttgtg tggttcccat ttggttgagg ctctgtactt ggtctgtgga
60
gaaagaggtt tcttttacac ccctaagact agaagaggta tcgttgagca atgttgcacc
120
tctatttgtt ccctgtatca gttggaaaac tactgcggtt aa
162

¡´210¡µ40
¡´211¡µ159
¡´212¡µDNA
¡´213¡µARTIFICIAL

¡´220¡µ
¡´223¡µDNA ENCODING HUMAN INSULIN ANALOGUE PRECURSOR

¡´400¡µ40
ttcgtcaagc agcacttgtg tggttcccat ttggttgagg ctctgtactt ggtctgtgga
60
gaaagaggtt tcttttacac ccctgaaaga agaggtatcg ttgagcaatg ttgcacctct
120
atttgttccc tgtatcagtt ggaaaactac tgcaactaa
159

¡´210¡µ41
¡´211¡µ156
¡´212¡µDNA
¡´213¡µARTIFICIAL

¡´220¡µ
¡´223¡µDNA ENCODING HUMAN INSULIN ANALOGUE PRECURSOR

¡´400¡µ41
ttcgtcgatc agcacttgtg tggttcccat ttggttgagg ctctgtactt ggtctgtgga
60
gaaagaggtt tcttttacac ccctaagaga ggtatcgttg agcaatgttg cacctctatt
120
tgttccctgt atcagttgga aaactactgc aactaa
156

¡´210¡µ42
¡´211¡µ170
¡´212¡µDNA
¡´213¡µARTIFICIAL

¡´220¡µ
¡´223¡µDNA ENCODING HUMAN INSULIN ANALOGUE PRECURSOR

¡´400¡µ42
ttcgtcaacc agcacttgtg tggttcccat ttggttgagg ctctgtactt ggtctgtgga
60
gaaagaggtt tcttttacac caaggaaaaa agaggtatcg ttgagcaatg ttgcacctct
120
atttgttccc tgtatcagtt ggaaaactac tgcggttaag aattccctag
170

¡´210¡µ43
¡´211¡µ174
¡´212¡µDNA
¡´213¡µARTIFICIAL

¡´220¡µ
¡´223¡µDNA ENCODING HUMAN INSULIN ANALOGUE PRECURSOR

¡´400¡µ43
ttcgtcaagc agcacttgtg tggttcccat ttggttgagg ctctgtactt ggtctgtgga
60
gaaagaggtt tcttttacac ccctgaagaa aaaagaggta tcgttgagca atgttgcacc
120
tctatttgtt ccctgtatca gttggaaaac tactgcggtt aagaattccc tagg

174

¡´210¡µ44
¡´211¡µ168
¡´212¡µDNA
¡´213¡µARTIFICIAL

¡´220¡µ
¡´223¡µDNA ENCODING HUMAN INSULIN ANALOGUE PRECURSOR

¡´400¡µ44
ttcgtcgatc agcacttgtg tggttcccat ttggttgagg ctctgtactt ggtctgtgga
60
gaaagaggtt tcttttacac ccctaagaga ggtatcgttg agcaatgttg cacctctatt
120
tgttccctgt atcagttgga aaactactgc ggttaagaat tccctagg
168

¡´210¡µ45
¡´211¡µ50
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ45
gtgcggggaa cgaggcttct tctacacacc caagaccaag cgtggcattg 50

¡´210¡µ46
¡´211¡µ50
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ46
gtagagggag cagatgctgg tacagcattg ttccacaatg ccacgcttgg 50

¡´210¡µ47
¡´211¡µ50
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ47
tgcggctcac acctggtgga agctctctac ctagtgtgcg gggaacgagg 50

¡´210¡µ48
¡´211¡µ50
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ48
ctgactgaat tctagttgca gtagttctcc agctggtaga gggagcagat          50

¡´210¡µ49
¡´211¡µ50
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ49
acttgctcga gaaaagattt gtgaaccaac acctgtgcgg ctcacacctg          50

¡´210¡µ50
¡´211¡µ32
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ50
cttctacaca cccaagcgtg gcattgtgga ac          32

¡´210¡µ51
¡´211¡µ32
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ51
gttccacaat gccacgcttg ggtgtgtaga ag          32

¡´210¡µ52
¡´211¡µ46
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ52
ggtttctttt acaccaagga aagagctgct aaaggtatcg ttgagc          46

¡´210¡µ53
¡´211¡µ46
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ53
gctcaacgat acctttagca gctctttcct tggtgtaaaa gaaacc          46

¡´210¡µ54
¡´211¡µ37
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ54
ggtttctttt acaccaagga aaaaagaggt atcgttg                37

¡´210¡µ55
¡´211¡µ37
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ55
caacgatacc tcttttttcc ttggtgtaaa agaaacc                37

¡´210¡µ56
¡´211¡µ40
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ56
ggtttctttt acaccaagcc tgaaaaaaga ggtatcgttg                40

¡´210¡µ57
¡´211¡µ40
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ57
caacgatacc tcttttttca ggcttggtgt aaaagaaacc                40

¡´210¡µ58
¡´211¡µ32
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ58
gagaaaagat tcgtcaagca gcacttgtgt gg                32

¡´210¡µ59
¡´211¡µ32
¡´212¡µDNA

¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ59
ccacacaagt gctgcttgac gaatcttttc tc          32

¡´210¡µ60
¡´211¡µ39
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ60
gtttctttta cacccctgaa gaaaaaagag gtatcgttg          39

¡´210¡µ61
¡´211¡µ39
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ61
caacgatacc tcttttttct tcaggggtgt aaaagaaac          39

¡´210¡µ62
¡´211¡µ40
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ62
ggtttctttt acaccgataa ggaaaaaaga ggtatcgttg          40

¡´210¡µ63
¡´211¡µ40
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ63
caacgatacc tcttttttcc ttatcggtgt aaaagaaacc          40

¡´210¡µ64
¡´211¡µ42
¡´212¡µDNA
¡´213¡µARTIFICIAL

¡´220¡µ

¡´223¡µPRIMER FOR CONSTRUCTION OF EXPRESSION VECTOR COMPRISING HUMAN INSULIN
ANALOGUE PRECURSOR

¡´400¡µ64
ggtttctttt acacccctaa gactagaaga ggtatcgttg ag                42

¡´210¡µ65
¡´211¡µ42
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ65
ctcaacgata cctcttctag tcttaggggt gtaaaagaaa cc                42

¡´210¡µ66
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ66
gttggaaaac tactgcggtt aagaattccc tagg                34

¡´210¡µ67
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ67
cctagggaat tcttaaccgc agtagttttc caac                34

¡´210¡µ68
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ68
cttttacacc cctgaaagaa gaggtatcgt tgag                34

¡´210¡µ69
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ69
ctcaacgata cctcttcttt caggggtgta aaag                34

¡´210¡µ70
¡´211¡µ36
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ70
cttttacacc cctaagagag gtatcgttga gcaatg                36

¡´210¡µ71
¡´211¡µ36
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ71
cattgctcaa cgatacctct cttaggggtg taaaag                36

¡´210¡µ72
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ72
gttggaaaac tactgcggtt aagaattccc tagg                34

¡´210¡µ73
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ73
cctagggaat tcttaaccgc agtagttttc caac                34

¡´210¡µ74
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ74
gttggaaaac tactgcggtt aagaattccc tagg                34

¡´210¡µ75
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ75
cctagggaat tcttaaccgc agtagttttc caac                34

¡´210¡µ76
¡´211¡µ34
¡´212¡µDNA
¡´213¡µARTIFICIAL

¡´220¡µ
¡´223¡µPRIMER FOR CONSTRUCTION OF EXPRESSION VECTOR COMPRISING HUMAN INSULIN
ANALOGUE PRECURSOR

¡´400¡µ76
gttggaaaac tactgcggtt aagaattccc tagg                34

¡´210¡µ77
¡´211¡µ53
¡´212¡µPRT
¡´213¡µARTIFICIAL

¡´220¡µ
¡´223¡µHUMAN INSULIN ANALOGUE PRECURSOR

¡´400¡µ77
Phe Val Asn Gln His Leu Cys Gly Ser His Leu Val Glu Ala Leu
                5                   10                  15
Tyr Leu Val Cys Gly Glu Arg Gly Phe Phe Tyr Thr Asp Lys Glu
                20                  25                  30
Lys Arg Gly Ile Val Glu Gln Cys Cys Thr Ser Ile Cys Ser Leu
                35                  40                  45
Tyr Gln Leu Glu Asn Tyr Cys Gly
                50

¡´210¡µ78
¡´211¡µ30
¡´212¡µPRT
¡´213¡µARTIFICIAL

¡´220¡µ
¡´223¡µHUMAN INSULIN ANALOGUE B CHAIN

¡´400¡µ78
Phe Val Asn Gln His Leu Cys Gly Ser His Leu Val Glu Ala Leu
                5                   10                  15
Tyr Leu Val Cys Gly Glu Arg Gly Phe Phe Tyr Thr Pro Lys Thr
                20                  25                  30

## Claims

1. A human insulin analogue and a physiologically tolerable salt thereof having A chain and B chain as follows: 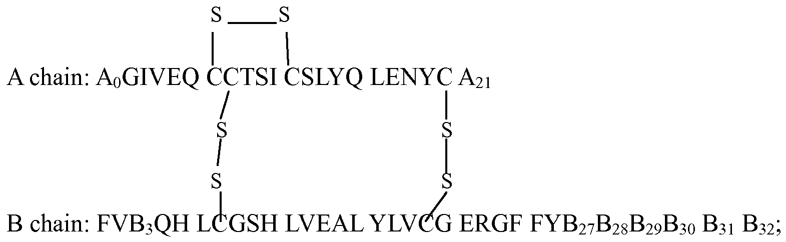 wherein:
A₀ can be R or missing;
A₂₁ can be N or G;
B₃ can be K, D or N;
B₂₇ can be E, T, D or K;
B₂₈ can be E, D, P or K;
B₂₉ can be E, K or P;
B₃₀ can be K, R, E, T or missing;
B₃₁,B₃₂ can be R, optionally single missing or both missing;
provided that:
when the said A₀ is missing, and both A₂₁ and B₃ are N:
B₃₁B₃₂ is missing, B₂₇B₂₈B₂₉B₃₀ is not TPKT, TKPT or TDKT;
or B₃₀ B₃₁B₃₂ is missing, B₂₇B₂₈B₂₉ is not TPK;
when the said A₀ is missing, A₂₁ is N, B₃ is K, and B₃₁B₃₂ is missing, B₂₇B₂₈B₂₉B₃₀ is not TEKT;
when the said A₀ is missing, A₂₁ is G, and B₃ is N,
B₂₇B₂₈B₂₉B₃₀ B₃₁B₃₂ is not TPKTRR;
when B3 and B27-B30 are lysine residues,
the ε-amino group of the lysine residues can be acylated;
optionally, α-amino at N'-terminal of A chain and B chain can also be acylated.

2. The human insulin analogue and a physiologically tolerable salt thereof according to claim 1, wherein only one of the said B3 and B27-B30 amino acid residues is lysine residue.

3. The human insulin analogue and a physiologically tolerable salt thereof according to claim 1, wherein when the said B₃ in B chain is N:
B₂₈ is selected from K, P or D, B₂₉ is selected from E, K or P, B₃₀ is selected from R, E, T or missing, and B₃₁ and B₃₂ are missing.

4. The human insulin analogue and a physiologically tolerable salt thereof according to claim 3, wherein when the said B₃₀ B₃₁B₃₂ is missing, B₂₈B₂₉ is KE.

5. The human insulin analogue and a physiologically tolerable salt thereof according to claim 1, wherein when the said B₃ in B chain is D:
B₂₈ and B₂₉ are P or K;
B₃₀ is R or T or missing;
B₃₁ and B₃₂ are R or missing;
among which B₂₈B₂₉ is preferably PK.

6. The human insulin analogue and a physiologically tolerable salt thereof according to claim 1, wherein when A₀ is missing and B₃ on B chain is K:
B₂₈ and B₂₉ are P or E;
B₃₀ is E or R;
B₃₁ is R or missing;
and B₃₂ is missing;
among which B₂₈B₂₉ is preferably PE.

7. The human insulin analogue and a physiologically tolerable salt thereof according to claim 1, wherein the sequences of the said A chain and B chain are selected from the group consisting of:
| | | |
|---|---|---|
| A chain: | RGIVEQCCTSICSLYQLENYCN | SEQ ID NO.4 |
| B chain: | FVNQHLCGSHLVEALYLVCGERGFFYTPK | SEQ ID NO.5 |
| | | |
| A chain: | GIVEQCCTSICSLYQLENYCN | SEQ ID NO.1 |
| B chain: | FVNQHLCGSHLVEALYLVCGERGFFYTKER | SEQ ID NO.6 |
| | | |
| A chain: | GIVEQCCTSICSLYQLENYCN | SEQ ID NO.1 |
| B chain: | FVNQHLCGSHLVEALYLVCGERGFFYTKE | SEQ ID NO.7 |
| | | |
| A chain: | GIVEQCCTSICSLYQLENYCN | SEQ ID NO.1 |
| B chain: | FVNQHLCGSHLVEALYLVCGERGFFYTKPE | SEQ ID NO.8 |
| | | |
| A chain: | GIVEQCCTSICSLYQLENYCN | SEQ ID NO.1 |
| B chain: | FVKQHLCGSHLVEALYLVCGERGFFYTPEE | SEQ ID NO.9 |
| | | |
| A chain: | GIVEQCCTSICSLYQLENYCN | SEQ ID NO.1 |
| B chain: | FVNQHLCGSHLVEALYLVCGERGFFYTDKE | SEQ ID NO. 10 |
| | | |
| A chain: | GIVEQCCTSICSLYQLENYCG | SEQ ID NO.2 |
| B chain: | FVDQHLCGSHLVEALYLVCGERGFFYTPKTRR | SEQ ID NO.11 |
| A chain: | RGIVEQCCTSICSLYQLENYCG | SEQ ID NO.3 |
| B chain: | FVDQHLCGSHLVEALYLVCGERGFFYTPKTR | SEQ ID NO.12 |
| | | |
| A chain: | GIVEQCCTSICSLYQLENYCN | SEQ ID NO.1 |
| B chain: | FVKQHLCGSHLVEALYLVCGERGFFYTPERR | SEQ ID NO. 13 |
| | | |
| A chain: | GIVEQCCTSICSLYQLENYCN | SEQ ID NO.1 |
| B chain: | FVDQHLCGSHLVEALYLVCGERGFFYTPK | SEQ ID NO.14 |
| | | |
| A chain: | RGIVEQCCTSICSLYQLENYCN | SEQ ID NO.4 |
| B chain: | FVDQHLCGSHLVEALYLVCGERGFFYTPK | SEQ ID NO.14 |
| | | |
| A chain: | GIVEQCCTSICSLYQLENYCG | SEQ ID NO.2 |
| B chain: | FVNQHLCGSHLVEALYLVCGERGFFYTKE | SEQ ID NO. 15 |
| | | |
| A chain: | GIVEQCCTSICSLYQLENYCG | SEQ ID NO.2 |
| B chain: | FVKQHLCGSHLVEALYLVCGERGFFYTPEE | SEQ ID NO.16 |
| | | |
| A chain: | GIVEQCCTSICSLYQLENYCG | SEQ ID NO.2 |
| B chain: | FVDQHLCGSHLVEALYLVCGERGFFYTPKR | SEQ ID NO. 17 |
| | | |
| A chain: | GIVEQCCTSICSLYQLENYCG | SEQ ID NO.2 |
| B chain: | FVDQHLCGSHLVEALYLVCGERGFFYTPK | SEQ ID NO.18 |
| | | |
| A chain: | RGIVEQCCTSICSLYQLENYCG | SEQ ID NO.3 |
| B chain: | FVDQHLCGSHLVEALYLVCGERGFFYTPK | SEQ ID NO.18 |
| | | |
| A chain: | GIVEQCCTSICSLYQLENYCG | SEQ ID NO.2 |
| B chain: | FVNQHLCGSHLVEALYLVCGERGFFYTDKE | SEQ ID NO. 10. |

8. The human insulin analogue and a physiologically tolerable salt thereof according to any one of claims 1 to 7, wherein the said human insulin analogue is PEGylated.

9. The human insulin analogue and a physiologically tolerable salt thereof according to any one of claims 1 to 8, wherein the said human insulin analogue is modified by PEG; the molecular weight of the said PEG molecule is 5-100 KD , preferably 10-80 KD, more preferably 15-45KD, most preferably 20-40KD; and the said PEG molecule is branched-chain or straight-chain type.

10. A method for preparing the human insulin analogue or physiologically tolerable salt thereof according to any one of claims 1 to 9, comprising constructing an expression vector, expressing the vector transformed into the host cells, isolating and purifying the expressed precursor, releasing the said insulin analogue from the expressed precursor via chemical and/or enzymatic methods; optionally the human insulin analogue is PEGylated, the said PEgylation is preferably a chemically acylating modification method, which comprises synthesizing an acylating agent, acylating the amino group located in human insulin analogues, and removing the protecting group from the acylated group.

11. An expressed precursor used for the preparation of human insulin analogue or physiologically tolerable salt thereof according to any one of claims 1 to 9, which has the following formula (I):
A-R1-B (I)
wherein:
R1 is a peptide fragment having 0 to 5 amino acid residues, the said peptide fragment consists of alanine (A), lysine (K) and arginine (R);
A and B correspond to A chain and B chain of the said human insulin analogue, respectively.

12. The expressed precursor according to claim 11, wherein the said expressed precursor is selected from the group consisting of:
SEQ ID NO.19:
FVNQHLCGSHLVEALYLVCGERGFFYTDKEKRGIVEQCCTSICSLYQLENYCN,
SEQ ID NO.20:
FVKQHLCGSHLVEALYLVCGERGFFYTPEEKRGIVEQCCTSICSLYQLENYCG,
SEQ ID NO.21:
FVKQHLCGSHLVEALYLVCGERGFFYTPEEKRGIVEQCCTSICSLYQLENYCN or
SEQ ID NO.77:
FVNQHLCGSHLVEALYLVCGERGFFYTDKEKRGIVEQCCTSICSLYQLENYCG.

13. A DNA encoding the expressed precursor as claimed in claim 11 or 12.

14. An expression vector comprising the DNA as claimed in claim 13.

15. A host cell transformed with the expression vector as claimed in claim 14.

16. The host cell according to claim 15. wherein the said host cell is bacterium, preferably Escherichia coli.

17. The host cell according to claim 15. wherein said the host cell is yeast, preferably Pichia pastoris or Saccharomyces cerevisiae.

18. An insulin derivative, **characterized in that** acylated insulin is formed by connecting an acylated group to the α-amino group at the N-terminal of A chain and B chain, or to the ε-amino group at lysine residue of B3, B27-B30, the acylated insulin has the following formula:
S-W-X-Y-Z
wherein S is insulin or human insulin analogue according to one of claims 1 to 7;
-W-X-Y-Z is an acylated group of human insulin analogue, wherein, W is selected from
• a group having a two-acyl structure of -OC(CH2)mCO-, wherein m is an integer between 2 and 10, an amide bond is formed between one of carboxylic groups on the said structure and α-amino group at the N-terminal amino acid residue of A-chain or B-chain of the parent insulin or analogue thereof or ε-amino group at a Lys residue existing in B-chain;
• an α-amino acid residue with a carboxyl group on the side chain or a peptide having 2 to 4 α-amino acids with a carboxyl group on the side chain, wherein an amide bond is formed between the said residue or the said peptide and α-amino group at the N-terminal of A-chain or B-chain of the parent insulin or analogue thereof or ε-amino group at a Lys residue existing in B-chain;
X is selected from
• -CO-;
• a diamino compound comprising a carboxylic group, wherein an amide bond is formed between one of α-amino groups of the said diamino compound and the carboxylic group of W;
a) when W is an α-amino acid residue or a peptide having 2 to 4 α-amino acids, the said amide bond is formed between the amino group of W and the -CO- of X;
b) when W is a group having a two-acyl structure, the said X group is linked to the two-acyl structure via one of its amino groups;
Y is selected from
• -A(CH₂)ₘ-, wherein m is an integer between 6 and 32, and A is absent or is CO-;
• bivalent hydrocarbon chain comprising an acyl group, which contains 1, 2 or 3 -CH = CH- groups and several -CH2- groups sufficient to obtain totally 10-32 carbon atoms on the chain;
• bivalent hydrocarbon chain having the formula of -B(CH₂)ᵥC₆H₄(CH₂)_{w}-, wherein B is absent or CO-, v and w are integers or one of them is 0, making v and w in the range of 6 to 30;
a) when X is CO-, A or B is absent; or
b) when X is a diamino compound, A or B is CO-;
Z is selected from the group consisting of -OH, -NH₂, -COOH, -SO₃H, -PO₃H.

19. The insulin derivative according to claim 18, wherein the acylated group -W-X-Y-Z is connected to the ε-amino group at lysine residues of B3, B27 to B30 of parent insulin.

20. The insulin derivative according to claim 18, wherein the acylated group -W-X-Y-Z is connected to the α-amino group at the N-terminal of chain A and chain B of parent insulin.

21. The insulin derivative according to claim 18, wherein S is human insulin analogue selected from the group consisting of:
| | | |
|---|---|---|
| A chain: | GIVEQCCTSICSLYQLENYCN | SEQ ID NO.1 |
| B chain: | FVNQHLCGSHLVEALYLVCGERGFFYTDKE | SEQ ID NO.10 |
| | | |
| A chain: | GIVEQCCTSICSLYQLENYCN | SEQ ID NO.1 |
| B chain: | FVKQHLCGSHLVEALYLVCGERGFFYTPEE | SEQ ID NO.9 |
| | | |
| A chain: | GIVEQCCTSICSLYQLENYCG | SEQ ID NO.2 |
| B chain: | FVKQHLCGSHLVEALYLVCGERGFFYTPEE | SEQ ID NO.16. |

22. The insulin derivative according to claim 18, wherein the acylated group -W-X-Y-Z is selected from the group consisting of:
N^{α}-(HOOC(CH₂)₁₄CO)-γ-Glu;
N^{α}-(HO(CH₂)₁₅CO)-γ-Glu;
N^{α}-(HOOC(CH₂)₁₄CO)-N^{ε}-(3-acyl propionic acid *)-Lys, in which * is the connection point for the insulin.

23. The insulin derivative according to claim 18, which is selected from the group consisting of:
B28D-N^{εB29}-(N^{α}-(HOOC(CH₂)₁₄CO)-γ-Glu)-B30E human insulin;
B28D-N^{εB29}-(N^{α}-(HO(CH₂)₁₅CO)-γ-Glu)-B30E human insulin;
N^{α}-(HOOC(CH₂)₁₄CO)-N^{ε}-(OCCH₂CH₂CO-(B28D-N^{εB29}-B30E human insulin;)) -Lys-OH;
N^{εB3}-(N^{α}-(HOOC(CH₂)₁₄CO)-γ-Glu)-B29E-B30E human insulin;
N^{εB3}-(N^{α}-(HOC(CH₂)₁₅CO)-γ-Glu)-B29E-B30E human insulin;
N^{α}-(HOOC(CH₂)₁₄CO)-N^{ε}-(OCCH₂CH₂CO-(N^{εB3}-B29E-B30E human insulin;))-Lys-OH;
N^{εB3}-(N^{α}-(HOOC(CH₂)₁₄CO)-γ-Glu)-B29E-B30E, A21G human insulin;
N^{εB3}-(N^{α}-(HOC(CH₂)₁₅CO)-γ-Glu)-B29E-B30E, A21G human insulin;
N^{α}-(HOOC(CH₂)₁₄CO)-N^{ε}-(OCCH₂CH₂CO-(N^{εB3}-B29E-B30E, A21G human insulin;))-Lys-OH.

24. A pharmaceutical formulation comprising human insulin analogue or physiologically tolerable salt thereof according to any one of claims 1 to 9, or comprising insulin derivatives according to any one of claims 18 to 23.

25. The pharmaceutical formulation according to claim 24, comprising human insulin analogue or a physiologically tolerable salt thereof in dissolved, amorphous and/or crystalline forms.

26. The pharmaceutical formulation according to claim 24, comprising a long-term adjuvant, the said long-term adjuvant is present along with the medicament in a manner of co-crystallization.

27. An injectable solution having insulin activity, wherein the solution comprises the pharmaceutical formulation according to claim 24 present in dissolved form.

28. Use of human insulin analogue and/or a physiologically tolerable salt thereof according to any one of claims 1 to 9, or insulin derivative according to any one of claims 18 to 23, in the preparation of a medicament for the treatment of type II diabetes, hyperglycemia, obesity or insulin resistance syndrome.

29. A use of human insulin analogue and/or a physiologically tolerable salt thereof according to any one of claims 1 to 9, in the preparation of a quick-acting and/or long-acting pharmaceutical formulation having insulin activity.
